# EUROPÄISCHE PATENTSCHRIFT

(11) **EP 2 188 273 B1**
(45) Veröffentlichungstag und Bekanntmachung des Hinweises auf die Patenterteilung: **22.10.2014**
(21) Anmeldenummer: 08827286.9
(22) Anmeldetag: 14.08.2008
(51) Int. Cl.: C07D 307/12, A61P 3/00

(54) **SUBSTITUIERTE TETRAHYDRONAPHTHALINE, VERFAHREN ZU IHRER HERSTELLUNG UND IHRE VERWENDUNG ALS ARZNEIMITTEL**
SUBSTITUTED TETRA HYDRO NAPHTHALINES, METHOD FOR THEIR MANUFACTURE AND THEIR USE AS DRUGS
TETRAHYDRONAPHTHALINE SUBSTITUÉE, SON PROCÉDÉ DE FABRICATION ET SON UTILISATION EN TANT QUE MÉDICAMENT

(30) Priorität: 15.08.2007 EP 07291010
(43) Veröffentlichungstag der Anmeldung: 26.05.2010
(73) Patentinhaber: SANOFI, 75008 Paris (FR)
(72) Erfinder: SCHWINK, Lothar, 65926 Frankfurt am Main (DE); STENGELIN, Siegfried, 65926 Frankfurt am Main (DE); GOSSEL, Matthias, 65926 Frankfurt am Main (DE); HAACK, Torsten, 65926 Frankfurt am Main (DE); LENNIG, Petra, 65926 Frankfurt am Main (DE)
(74) Vertreter: Dey, Michael
(86) Internationale Anmeldenummer: PCT/EP2008/006700
(87) Internationale Veröffentlichungsnummer: WO 2009/021740

(56) Entgegenhaltungen:
- WO-A-03/087046
- WO-A-2005/033063
- MEYERS ET AL: "Aminomethyl tetrahydronaphthalene biphenyl carboxamide MCH-R1 antagonists-Increasing selectivity over hERG" BIOORGANIC & MEDICINAL CHEMISTRY LETTERS, OXFORD, GB, Bd. 17, Nr. 3, 19. Januar 2007 (2007-01-19), Seiten 814-818, XP005835945 ISSN: 0960-894X
- MEYERS ET AL: "Aminomethyl tetrahydronaphthalene ketopiperazine MCH-R1 antagonists-Increasing selectivity over hERG" BIOORGANIC & MEDICINAL CHEMISTRY LETTERS, OXFORD, GB, Bd. 17, Nr. 3, 19. Januar 2007 (2007-01-19), Seiten 819-822, XP005835946 ISSN: 0960-894X
- HERTZOG, D.L., WITTY, D.R.: "Biphenyl Amides and Isosteres as MCH R1 Antagonists" CURRENT TOPICS IN MEDICINAL CHEMISTRY, Bd. 7, 15. August 2007 (2007-08-15), Seiten 1455-1470, XP002463084
- MCBRIAR, M.: "Melanin Concentrating Hormone Receptor Antagonists as Antiobesity Agents: From M2 to MCHR-1" CURRENT TOPICS IN MEDICINAL CHEMISTRY, Bd. 7, 15. August 2007 (2007-08-15), Seiten 1440-1454, XP002463085

## Beschreibung

Die Erfindung betrifft substituierte Tetrahydronaphthaline und deren Derivate, sowie deren physiologisch verträgliche Salze und physiologisch funktionelle Derivate, deren Herstellung, Arzneimittel enthaltend mindestens ein erfindungsgemäßes substituiertes Tetrahydronaphthalin oder dessen Derivat und die Verwendung der erfindungsgemäßen substituierten Tetrahydronaphthaline und deren Derivate als Arzneimittel.

Es sind bereits den in der vorliegenden Anmeldung beschriebenen substituierten Tetrahydronaphthalinen und deren Derivaten in ihrer Gesamtstruktur ähnliche Verbindungen mit pharmakologischer Wirkung im Stand der Technik beschrieben, z. B. in der WO2002/064565 und der WO 2000/051970.
In WO2008/002575, WO2008/001160, WO2006/044293, WO2005/033063, US2005/0075324, US 2006/0247239 sowie in Meyers K. M. et al., Biorganic & Medicinal Chemistry Letters 17, 2007, 814-18; Meyers K. M. et al., Biorganic & Medicinal Chemistry Letters 17, 2007, 819-22; Mendez-Andino J.L. et al., Biorganic & Medicinal Chemistry Letters 15, 2007, 2092-2105 werden Amino-substituierte Tetrahydronaphthalin-Derivate mit MCH R1-antagonistischer Wirkung zur Behandlung von Obesitas offenbart.

Weitere Verbindungen mit MCH-antagonistischer Wirkung zur Behandlung der Obesitas sind im Stand der Technik beschrieben (Beispiele: WO2005047293, WO2004092181, WO2005103039, WO2004024702, WO2001021577, WO2003035624, WO2002089729, WO2002006245, WO2002002744, WO2002057233, WO2003045313, WO2003097047, WO2002010146, WO 2003087044, WO2003/087046, WO2001/021577). Eine Übersicht wird in Rokosz, L. L., Expert Opin. Drug Discov. 2007, 2, 1301-1327 gegeben.

Der Erfindung lag die Aufgabe zugrunde, Verbindungen zur Verfügung zu stellen, die eine Gewichtsreduktion bei Säugetieren bewirken und die zur Prävention und Behandlung von Obesitas und Diabetes sowie deren vielfältigen Folgeerkrankungen geeignet sind.

Überraschenderweise wurde eine Serie von Verbindungen gefunden, die die Aktivität von MCH-Rezeptoren modulieren. Insbesondere zeichnen sich die Verbindungen durch einen Antagonismus des MCH R1 aus.

Die Erfindung betrifft daher Verbindungen der Formel I, 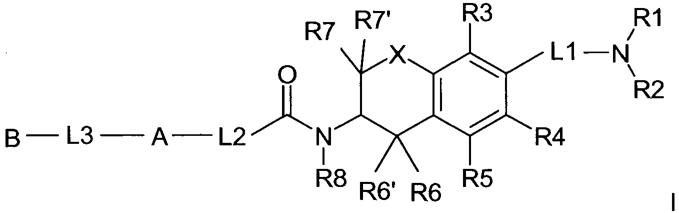 worin bedeuten
R1, R2
   unabhängig voneinander H, (C₁-C₈)-Alkyl, (C₁-C₄)-Alkoxy-(C₁-C₄)-alkyl, (C₃-C₈)-Alkenyl, (C₃-C₈)-Alkinyl, CO(R9), (C(R10)(R11))_{q}-R12, CO(C(R13)(R14))ᵣ-R15, CO-O(C₁-C₈)-Alkyl, CO(C(R13)(R14))ᵣ-N(R16)(R17);
   oder
R1 und R2 bilden zusammen mit dem Stickstoffatom, an das sie gebunden sind, einen 4 bis 10-gliedrigen mono-, bi- oder spirocyclischen Ring welcher außer dem Stickstoffatom 0 bis 3 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, CN, (C₁-C₆)-Alkyl, (C₃-C₈)-Cycloalkyl, O-(C₁-C₈)-Alkyl, (C₁-C₄)-Alkoxy-(C₁-C₄)-alkyl, Hydroxy-(C₁-C₆)-alkyl, Oxo, CO(R18), CON(R19)(R20), Hydroxy, COO(R21), N(R22)CO(C₁-C₆)-Alkyl, N(R23)(R24) oder SO₂(C₁-C₆)-Alkyl;
R10, R11
   unabhängig voneinander H, (C₁-C₆)-Alkyl, Hydroxy-(C₁-C₂)-Alkyl, F, OH;
R9, R13, R14, R16, R17, R18, R19, R20, R21, R22, R23, R24,
   unabhängig voneinander H, (C₁-C₆)-Alkyl;
   oder
R16 und R17, R23 und R24
   bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R12, R15
   unabhängig voneinander H, OH, F, O-(C₁-C₆)-Alkyl, S-(C₁-C₆)-Alkyl, O-phenyl, CN, COO(R25), N(R26)CO(C₁-C₆)-Alkyl, N(R27)(R28), CON(R29)(R30), SO₂(C₁-C₆)-Alkyl, 3-12 gliedriger mono-, bi- oder spirocyclischer Ring, der ein bis vier Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, N(R31)(R32), COO(R33), SO₂(C₁-C₆)-Alkyl und COOH enthalten kann;
R25, R26, R27, R28, R29, R30, R31, R32, R33
   unabhängig voneinander H, (C₁-C₈)-Alkyl;
   oder
R27 und R28, R29 und R30, R31 und R32
   bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
L1 C(R34)(R35), C(R36)(R37)C(R38)(R39), (C₃-C₆)-Cycloalkyl;
   optional kann R1 mit einem der Reste R34, R35, R36, R37, R38 oder R39 verbunden sein, so dass ein 5-6 gliedriger Ring entsteht;
R34, R35, R36, R37, R38, R39
   unabhängig voneinander H, (C₁-C₈)-Alkyl;
R3, R4, R5
   unabhängig voneinander H, F, Cl, Br, I, OH, CF₃, NO₂, CN, OCF₃, O-(C₁-C₆)-Alkyl, S-(C₁-C₆)-Alkyl, O-(C₁-C₄)-Alkoxy-(C₁-C₄)-alkyl, (C₁-C₆)-Alkyl, CON(R40)(R41), CO(R42);
R40, R41, R42
   unabhängig voneinander H, (C₁-C₈)-Alkyl;
   oder
R40 und R41
   bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
X C(R43)(R43');
R6, R6', R7, R7', R43, R43'
   unabhängig voneinander H, F, (C₁-C₈)-Alkyl, OH, O-(C₁-C₆)-Alkyl;
   oder
R6 und R6', oder R43 und R43' zusammen optional Oxo;
R8 H, (C₁-C₈)-Alkyl;
L2 Bindung, C(R44)(R45);
R44, R45;
   unabhängig voneinander H, (C₁-C₈)-Alkyl;
A 5-6 gliedriger aromatischer Ring, der bis zu 2 Heteroatome ausgewählt aus der Gruppe Stickstoff, Sauerstoff und Schwefel beinhalten kann, und substituiert sein kann mit einem oder mehreren der Substituenten H, F, Cl, Br, I, OH, CF₃, NO₂, CN, OCF₃, O-(C₁-C₆)-Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60);
im Fall L2 = Bindung kann C(O)NR8 mit einem ortho-ständigen Substituenten von A über eine Brücke enthaltend ein oder zwei Elemente aus der Gruppe Kohlenstoff und Stickstoff verbunden sein, so dass insgesamt ein 9 bis 10-gliedriger bicyclischer Ring entsteht;
R54, R55, R56, R57, R58, R59, R60
   unabhängig voneinander H, (C₁-C₈)-Alkyl;
   oder
R54 und R55, R56 und R57
   bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R61, R62, R63, R64
   unabhängig voneinander H, (C₁-C₈)-Alkyl;
L3 C(R62)(R63)O, und
B ein 4 bis 10-gliedriger mono-, bi- oder spirocyclischer nicht-aromatischer Ring, welcher 1 bis 3 Heteroatome beinhaltet, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
bedeuten.

Die Verbindungen der Formel I zeichnen sich dadurch aus, dass sie gegenüber strukturell ähnlichen Verbindungen mit MCH-antagonistischer Wirkung eine verbesserte Löslichkeit in wässrigen Medien (insbesondere in physiologisch relevanten Puffersystemen) bei gleichzeitiger hoher Aktivität aufweisen. Weiter zeichnen sich bevorzugte erfindungsgemäße Verbindungen durch eine geringe Blockade des hERG-Kanals aus. Des weiteren weisen bevorzugte erfindungsgemäße Verbindungen gegenüber Verbindungen des Standes der Technik eine verbesserte metabolische Stabilität auf.

Die Alkyl-, Alkenyl- und Alkinylreste in den Substituenten R1, R2, R3, R4, R5, R6, R6', R7, R7', R8, R9, R10, R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R43', R44, R45, R54, R55, R56, R57, R58, R59, R60, R61, R62, R63, R64 können sowohl geradkettig, verzweigt und/oder optional substituiert sein mit Substituenten wie (C₁-C₄)-Alkoxy oder Halogen. Dies trifft auch zu, sofern die Alkyl-, Alkenyl- und Alkinylreste Teil einer anderen Gruppe sind, z.B. Teil einer Alkoxygruppe (wie (C₁-C₄)-Alkoxy-(C₁-C₄)-alkyl)). Geeignete Halogene sind Fluor, Chlor, Brom und Iod, bevorzugt Fluor, Chlor und Brom, besonders bevorzugt Fluor.

Beispiele für Alkylgruppen sind: Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise mit 1, 2, 3 oder 4 identischen oder unterschiedlichen Resten, wie (C₁-C₄)-Alkoxy oder Halogen. Beispiele für mit Halogen substituierte Alkylgruppen sind fluorierte Alkylgruppen wie CF₃, CHF₂, CH₂F, 3-Fluorprop-1-yl, 2,2,1,1-Tetrafluorethyl. Dabei können die zusätzlichen Substituenten in jeder beliebigen Position des Alkylrestes auftreten. Bevorzugt sind die Alkylreste -soweit nicht anders definiert- unsubstituiert.

Unter Cycloalkyl ist im Sinne der vorliegenden Anmeldung Cycloalkyl sowie Cycloalkyl-alkyl-(Alkyl, das wiederum mit Cycloalkyl substituiert ist) zu verstehen, wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt. Bevorzugt sind die Cycloalkylreste -soweit nicht anders definiert- unsubstituiert.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl.

Unter Cycloalkenyl sind im Sinne der vorliegenden Anmeldung Cycloalkenylreste sowie Cycloalkenyl-Alkylreste (Alkyl, das mit Cycloalkenyl substituiert ist) zu verstehen, die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste und Cycloalkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Für Alkinylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt. Bevorzugt sind die Alkenyl- und Alkinylreste -soweit nicht anders definiert- unsubstituiert.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Die Bezeichnung Aryl umfasst in der vorliegenden Erfindung beispielsweise auch bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl. Bevorzugt sind die Arylreste -soweit nicht anders definiert- unsubstituiert. Besonders bevorzugt ist Aryl Phenyl oder Naphthyl.

Unter Heteroarylresten sind Reste zu verstehen, die von monocyclischen oder bicyclischen Aromaten abgeleitet sind, die Ringheteroatome, bevorzugt N, O oder S, enthalten. Im Übrigen gilt für die Heteroarylreste das bezüglich der Arylreste Aufgeführte.

Unter einem "Tricyclus" werden Strukturen mit 3 Ringen verstanden, die durch mehr als eine Bindung miteinander verbunden sind. Beispiele solcher Systeme sind kondensierte Systeme mit 3 Ringen und Spirocyclen mit ankondensiertem Ringsystem.

Unter einer polycyclischen Gruppe (bi-, tri- oder spirocyclisches Ringgerüst) ist im Sinne der vorliegenden Anmeldung eine Gruppe zu verstehen, die von Spiranen, kondensierten Ringsystemen oder Brücken-Ringsystemen abgeleitet ist. Die Spirane zeichnen sich dadurch aus, dass zwei Ringe nur ein Kohlenstoffatom gemeinsam aufweisen und die Ringebenen der beiden Ringe senkrecht aufeinander stehen. Bei den kondensierten Ringsystemen sind zwei Ringe so miteinander verknüpft, dass sie zwei Atome gemeinsam aufweisen. Bei dieser Art der Verknüpfung handelt es sich um eine "*ortho*-Kondensation". Bei den Brücken-Ringsystemen handelt es sich um Ringsysteme, die eine Brücke aus Kohlenstoff und/oder Heteroatomen zwischen zwei nicht benachbarten Atomen eines Rings aufweisen.

Unter einem "chemisch sinnvollen Rest" ist im Sinne der vorliegenden Erfindung ein Rest zu verstehen, der bei Raumtemperatur und Normaldruck stabil ist. Bevorzugt sind im Sinne der vorliegenden Erfindung unter einem "chemisch sinnvollen Rest" in der Definition der Gruppe A in den Verbindungen der Formel I Gruppen zu verstehen, die keine Heteroatom-Heteroatom-Bindungen zwischen den einzelnen Gliedern der Gruppen aufweisen.

Unter einem "nicht aromatischen" Ring ist im Sinne der vorliegenden Anmeldung bevorzugt ein Ring zu verstehen, der gesättigt oder teilweise ungesättigt ist. Dabei weist ein teilweise ungesättigter Ring gemäß der vorliegenden Anmeldung eine oder gegebenenfalls mehrere Doppelbindungen auf, wobei der teilweise ungesättigte Ring jedoch nicht aromatisch ist. Von dem Ausdruck "nicht aromatisch" sind im Sinne der vorliegenden Anmeldung auch "nicht heteroaromatische" Ringe umfasst.
Die Verbindungen der Formel I können ein oder mehrere Assymmetriezentren enthalten. Daher können die Verbindungen der Formel I in Form ihrer Racemate, Enantiomeren angereicherten Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

Können Reste oder Substituenten mehrfach in den Verbindungen der Formel I auftreten, so können sie alle unabhängig voneinander die angegebenen Bedeutungen haben und gleich oder verschieden sein.

Bevorzugt weisen die Symbole in der Formel I unabhängig voneinander die folgenden Bedeutungen auf:
R1, R2
   unabhängig voneinander H, (C₁-C₈)-Alkyl, (C₁-C₄)-Alkoxy-(C₁-C₄)-alkyl, (C₃-C₈)-Alkenyl, (C₃-C₈)-Alkinyl, CO(R9), (C(R10)(R11))_{q}-R12, CO(C(R13)(R14))ᵣ-R15, CO-O(C₁-C₈)-Alkyl, CO(C(R13)(R14))ᵣN(R16)(R17);
   bevorzugt bedeutet R1:
   H, (C₁-C₈)-Alkyl, (C(R10)(R11))_{q}-R12, (C₁-C₄)-Alkoxy-(C₁-C₄)-alkyl, (C₃-C₈)-Alkenyl, (C₃-C₈)-Alkinyl, CO-(C₁-C₈)-Alkyl, CO-O(C₁-C₈)-Alkyl,
   CO(C(R13)(R14))ᵣN(R16)(R17);
   bevorzugt bedeutet R2:
   (C₁-C₈)-Alkyl, (C(R10)(R11))_{q}-R12, (C₁-C₄)-Alkoxy-(C₁-C₄)-alkyl, (C₃-C₈)-Alkenyl, (C₃-C₈)-Alkinyl;
   oder
R1 und R2 bilden zusammen mit dem Stickstoffatom, an das sie gebunden sind, einen 4 bis 10-gliedrigen mono-, bi- oder spirocyclischen Ring welcher außer dem Stickstoffatom 0 bis 3 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, CN, (C₁-C₆)-Alkyl, (C₃-C₈)-Cycloalkyl, O-(C₁-C₈)-Alkyl, (C₁-C₄)-Alkoxy-(C₁-C₄)-alkyl, Hydroxy-(C₁-C₆)-alkyl, Oxo, CO(R18), CON(R19)(R20), Hydroxy, COO(R21), N(R22)CO(C₁-C₆)-Alkyl, N(R23)(R24) oder SO₂(C₁-C₆)-Alkyl; besonders bevorzugt bedeuten R1, R2:
   (C₁-C₈)-Alkyl, (C(R10)(R11))_{q}-R12, (C₁-C₄)-Alkoxy-(C₁-C₄)-alkyl; oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, (C₁-C₆)-Alkyl, O-(C₁-C₈)-Alkyl, (C₃-C₈)-Cycloalkyl, (C₁-C₄)-Alkoxy-(C₁-C₄)-alkyl, Hydroxy-(C₁-C₆)-alkyl, Oxo, CO(R18), Hydroxy, N(R22)CO(C₁-C₆)-Alkyl, oder SO₂(C₁-C₆)-Alkyl;
   ganz besonders bevorzugt bilden R1 und R2 zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, (C₁-C₆)-Alkyl, O-(C₁-C₈)-Alkyl, (C₃-C₈)-Cycloalkyl, (C₁-C₄)-Alkoxy-(C₁-C₄)-alkyl, Hydroxy-(C₁-C₆)-alkyl, Oxo, CO(R18), Hydroxy, N(R22)CO(C₁-C₆)-Alkyl, oder SO₂(C₁-C₆)-Alkyl;
R10, R11
   unabhängig voneinander H, (C₁-C₆)-Alkyl, Hydroxy-(C₁-C₂)-Alkyl, F, OH;
R9, R13, R14, R16, R17, R18, R19, R20, R21, R22, R23, R24,
   unabhängig voneinander H, (C₁-C₆)-Alkyl;
   oder
R16 und R17, R23 und R24
   bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
   q, r unabhängig voneinander 0, 1, 2, 3, 4, 5, 6; bevorzugt 0, 1, 2, 3, 4;
R12, R15
   unabhängig voneinander H, OH, F, O-(C₁-C₆)-Alkyl, S-(C₁-C₆)-Alkyl, O-phenyl, CN, COO(R25), N(R26)CO(C₁-C₆)-Alkyl, N(R27)(R28), CON(R29)(R30), SO₂(C₁-C₆)-Alkyl, 3-12 gliedriger mono-, bi- oder spirocyclischer Ring, der ein bis vier Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, N(R31)(R32), COO(R33), SO₂(C₁-C₆)-Alkyl und COOH enthalten kann;
   bevorzugt H, OH, F, O-(C₁-C₆)-Alkyl, N(R26)CO(C₁-C₆)-Alkyl, SO₂(C₁-C₆)-Alkyl, 3-12 gliedriger mono-, bi- oder spirocyclischer Ring, der ein bis drei Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, N(R31)(R32) und SO₂(C₁-C₆)-Alkyl enthalten kann;
R25, R26, R27, R28, R29, R30, R31, R32, R33
   unabhängig voneinander H, (C₁-C₈)-Alkyl;
   oder
R27 und R28, R29 und R30, R31 und R32
   bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
L1 C(R34)(R35), C(R36)(R37)C(R38)(R39), (C₃-C₆)-Cycloalkyl;
   bevorzugt C(R34)(R35);
   optional kann R1 mit einem der Reste R34, R35, R36, R37, R38 oder R39 verbunden sein, so dass ein 5-6 gliedriger Ring entsteht;
R34, R35, R36, R37, R38, R39
   unabhängig voneinander H, (C₁-C₈)-Alkyl;
R3, R4, R5
   unabhängig voneinander H, F, Cl, Br, I, OH, CF₃, NO₂, CN, OCF₃, O-(C₁-C₆)-Alkyl, S-(C₁-C₆)-Alkyl, O-(C₁-C₄)-Alkoxy-(C₁-C₄)-alkyl, (C₁-C₆)-Alkyl, CON(R40)(R41), CO(R42);
   bevorzugt unabhängig voneinander H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl, CO(C₁-C₆)-Alkyl;
   besonders bevorzugt unabhängig voneinander H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl;
   ganz besonders bevorzugt unabhängig voneinander H, F, Cl, O-(C₁-C₆)-Alkyl, (C₁-C₆)-Alkyl;
   insbesonders besonders bevorzugt H;
   wobei bevorzugt mindestens zwei, oder alle Reste R3, R4 und R5 H bedeuten;
R40, R41, R42
   unabhängig voneinander H, (C₁-C₈)-Alkyl;
   oder
R40 und R41
   bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
X C(R43)(R43');
R6, R6', R7, R7', R43, R43'
   unabhängig voneinander H, F, (C₁-C₈)-Alkyl, OH, O-(C₁-C₆)-Alkyl;
   bevorzugt H;
   oder
   R6 und R6', oder R43 und R43' zusammen optional Oxo;
R8 H, (C₁-C₈)-Alkyl;
L2 Bindung, C(R44)(R45);
   bevorzugt Bindung;
R44, R45;
   unabhängig voneinander H, (C₁-C₈)-Alkyl;
A 5-6 gliedriger aromatischer Ring, der bis zu 2 Heteroatome ausgewählt aus der Gruppe Stickstoff, Sauerstoff und Schwefel beinhalten kann, und substituiert sein kann mit einem oder mehreren der Substituenten H, F, Cl, Br, I, OH, CF₃, NO₂, CN, OCF₃, O-(C₁-C₆)-Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60); bevorzugt H, F, Cl, Br, I, OH, CF₃, NO₂, CN, OCF₃, O-(C₁-C₆)-Alkyl, (C₁-C₄)-Alkoxy-(C₁-C₄)-alkyl, (C₁-C₆)-Alkyl; besonders bevorzugt H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl; ganz besonders bevorzugt H, F, Cl, O-(C₁-C₆)-Alkyl, (C₁-C₆)-Alkyl;
   insbesondere ganz besonders bevorzugt H;
   bevorzugt ist der 5-6 gliedrige aromatische Ring ausgewählt aus der Gruppe bestehend aus 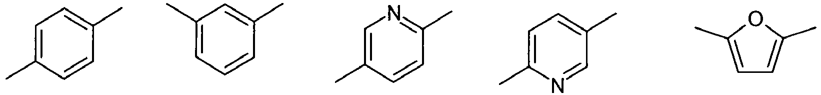 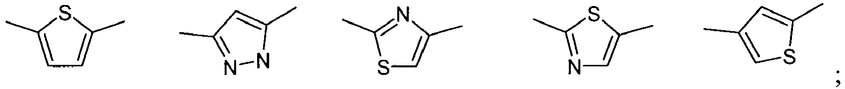 besonders bevorzugt 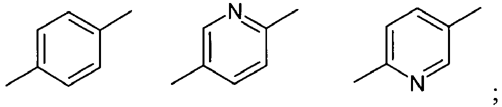
im Fall L2 = Bindung kann C(O)NR8 mit einem ortho-ständigen Substituenten von A über eine Brücke enthaltend ein oder zwei Elemente aus der Gruppe Kohlenstoff und Stickstoff verbunden sein, so dass insgesamt ein 9 bis 10-gliedriger bicyclischer Ring entsteht; bevorzugt enthält die Brücke zwei Kohlenstoff-Elemente, so dass insgesamt ein Isochinolinon oder ein Dihydroisochinolinon entsteht;
R54, R55, R56, R57, R58, R59, R60
   unabhängig voneinander H, (C₁-C₈)-Alkyl;
   oder
R54 und R55, R56 und R57
   bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R61, R62, R63, R64
   unabhängig voneinander H, (C₁-C₈)-Alkyl;
L3 C(R62)(R63)O, und
B ein 4 bis 10-gliedriger mono-, bi- oder spirocyclischer nicht-aromatischer Ring, welcher 1 bis 3 Heteroatome beinhaltet, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy; besonders bevorzugt das kombinierte Element B-L3, das ausgewählt ist aus der Gruppe 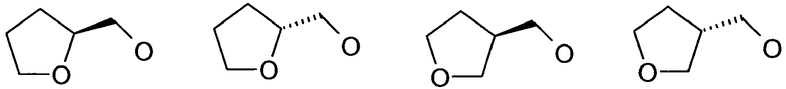 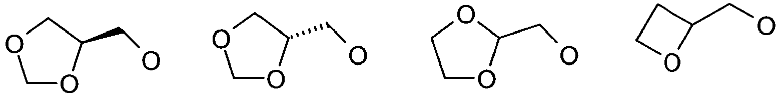 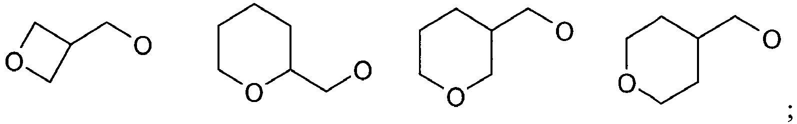 bevorzugt 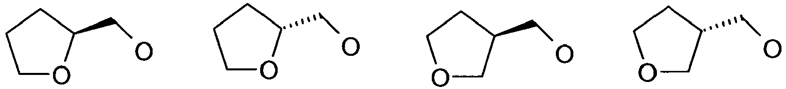 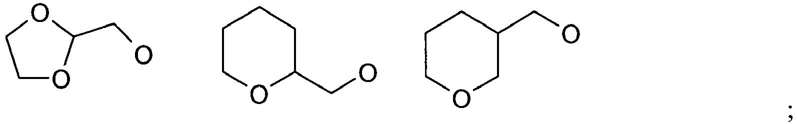 besonders bevorzugt 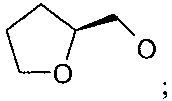
bedeuten.

Ein besonderer Aspekt der Erfindung sind die Verbindungen der Formel II 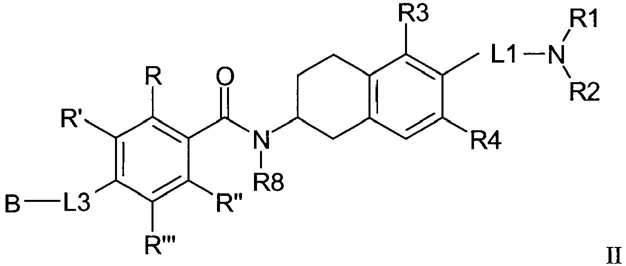 worin die Variablen R1, R2, L1, R3, R4 und R8 die oben angegebenen Bedeutungen haben und
R, R', R", R'"
   unabhängig voneinander H, F, Cl, Br, I, OH, CF₃, NO₂, CN, OCF₃, O-(C₁-C₆), Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60);
   bevorzugt unabhängig voneinander H, F, Cl, Br, I, OH, CF₃, NO₂, CN, OCF₃, O-(C₁-C₆)-Alkyl, (C₁-C₄)-Alkoxy-(C₁-C₄)-alkyl, (C₁-C₆)-Alkyl;
   besonders bevorzugt unabhängig voneinander H, F, Cl, Br, CF₃, CN, OCF₃, O-(C₁-C₆)-Alkyl, (C₁-C₄)-Alkoxy-(C₁-C₄)-alkyl, (C₁-C₆)-Alkyl;
   ganz besonders bevorzugt unabhängig voneinander H, F, Cl, O-(C₁-C₆)-Alkyl, (C₁-C₆)-Alkyl;
   insbesondere ganz besonders bevorzugt H;
L3 CH₂O;
B 4 bis 6-gliedriger nicht-aromatischer Ring, welcher 1 bis 2 Sauerstoffatome beinhaltet, wobei das Ringsystem zusätzlich substituiert sein kann mit einem oder mehreren der folgenden Substituenten: F, (C₁-C₆)-Alkyl, O-(C₁-C₈)-Alkyl, (C₁-C₄)-Alkoxy-(C₁-C₄)-alkyl, Oxo, Hydroxy bevorzugt (C₁-C₆)-Alkyl oder Hydroxy;
das kombinierte Element B-L3
bevorzugt ausgewählt aus der Gruppe 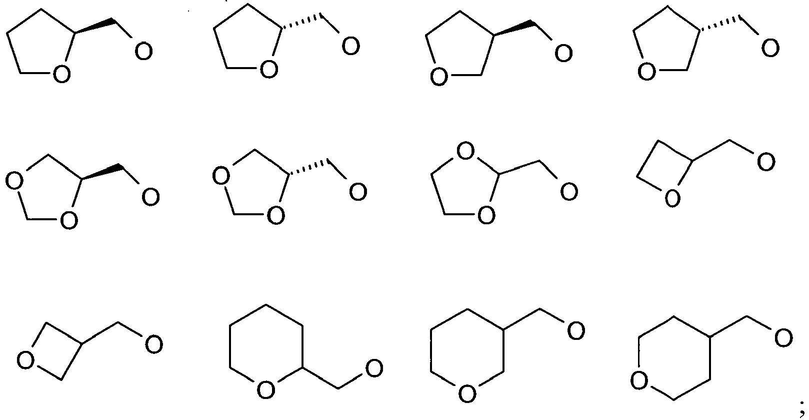 besonders bevorzugt 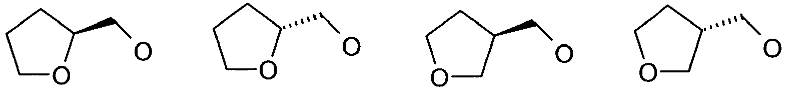 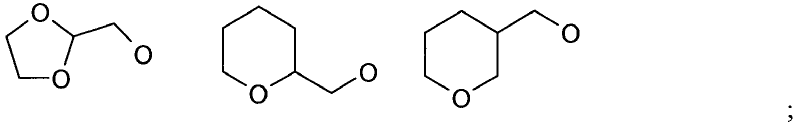 insbesondere bevorzugt 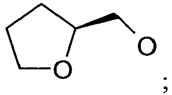 bedeuten.
Ein weiterer besonderer Aspekt der Erfindung sind die Verbindungen der Formel IIa 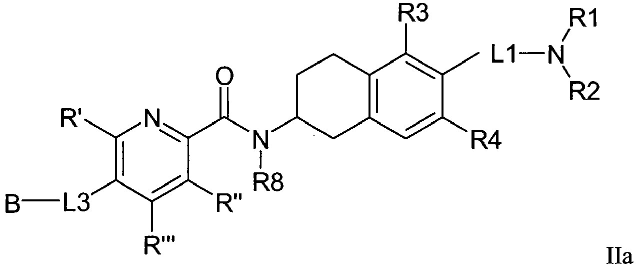 worin die Variablen R1, R2, L1, R3, R4, R8, R', R", R"', L3 und B
die oben angegebenen Bedeutungen haben.

Weiterhin offenbart sind Verbindungen der Formel III 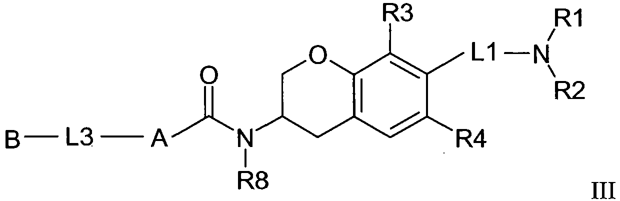 worin R1, R2, R3, R4, R8, A, L1, L3 und B die für Formel I angegebenen Bedeutungen haben.

In einem anderen besonderen Aspekt betrifft die Erfindung Verbindungen der Formel IV 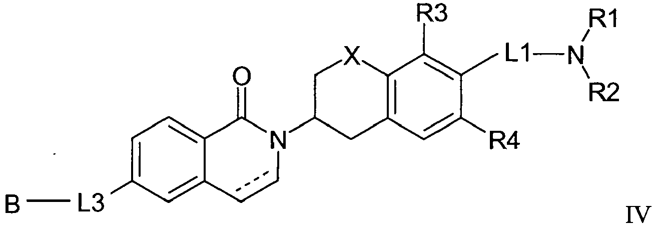 worin R1, R2, R3, R4, X, L1, L3 und B die für Formel I angegebenen Bedeutungen haben. Die unterbrochene Linie zeigt eine optionale Doppelbindung an, so dass sowohl Dihydroisochinolinone als auch Isochinolinone von der Formel IV umfasst werden.

Die erfindungsgemäßen Verbindungen der allgemeinen Formel I können analog zu dem Fachmann bekannten Verfahren hergestellt werden. Geeignete Verfahren zur Herstellung der erfindungsgemäßen Verbindungen der allgemeinen Formel I sind nachstehend beispielhaft erwähnt (siehe insbesondere Methoden A, B, C, D, E, F, G, H, I, J, K, L, M, N, O, P und Schemata 1 bis 3).
Bevorzugte Ausführungsformen der genannten Schritte ebenso wie die Herstellung der in den Schritten eingesetzten Ausgangssubstanzen sind dem Fachmann bekannt und in den genannten Schemata und Methoden sowie Beispielen beispielhaft erwähnt.

Diese Erfindung bezieht sich weiterhin auf die Verwendung von Verbindungen der Formel I und ihren pharmazeutischen Zusammensetzungen als MCH-Rezeptor-Liganden. Die erfindungsgemäßen MCH-Rezeptor-Liganden eignen sich insbesondere als Modulatoren der Aktivität des MCH R1.
Die Rolle von MCH in der Regulation des Energiehaushalts ist inzwischen gut dokumentiert (Qu, D. et al.; Nature 1996, 380, 243-7; Shimada, M. et al. Nature 1998,396, 670-4; Chen, Y. et al. Endocrinology 2002, 143, 2469-77; Endocrinology 2003, 144, 4831-40; Übersichten: G. Hervieu, Expert Opin. Ther. Targets 2003, 7, 495-511; Shi, Y., Peptides 2004, 25, 1605-11; Pissios, P. et al., Endocrine Rev. 2006, 27, 606-20 ; Luthin, D. R., Life Sei. 2007, 81, 423-440). Auch gibt es Hinweise, dass MCH-Antagonisten zentral bedingte Störungen wie z.B. Angstneurosen und Depressionen günstig beeinflussen können (Borowsky, B. et al.; Nature Medicine 2002, 8, 825-30; Übersichten: Hervieu, G., Expert Opin. Ther. Targets 2003, 7, 495-511; Chaki, S. et al., Drug Dev. Res. 2005, 65, 278-290; Dyck, B., Drug Dev. Res. 2005, 65, 291-300; Shimazaki, T., CNS Drugs 2006, 20, 801-11; Drugs Fut. 2007, 32, 809-822).

Besonders geeignet sind solche Verbindungen zur Behandlung und/oder Prävention von
1. Obesitas
2. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glukose-Toleranz,
   - Schutz der ß-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid- Konzentrationen
   - niedrige HDL-Cholesterin Konzentration
4. Fettleber, insbesondere nichtalkoholische Fettleber und deren Verlaufsformen
   - Steatose
   - Steatohepatitis
   - Zirrhose.
5. Verschiedene andere Zuständen, die mit dem Metabolischen Syndrom assoziert sein können, sind wie:
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
6. Psychiatrische Indikationen wie
   - Depressionen
   - Angstzustände
   - Störungen des zirkadianen Rhythmus
   - Affektionsstörungen
   - Schizophrenie
   - Suchtkrankheiten
7. Schlafstörungen wie
   - Schlafapnoe
   - Narkolepsie
8. Entzündliche Erkrankungen wie
   - Entzündliche Darmerkrankung
   - Crohn'sche Krankheit

### Galenik

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg (typischerweise von 0,01 mg bis 50 mg) pro Tag pro Kilogramm Körpergewicht, z.B. 0,1 -10 mg/kg/Tag. Eine intravenöse Dosis kann z.B. im Bereich von 0,001 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.
Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge mindestens einer Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die mindestens eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen mindestens einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man mindestens eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich als selektive MCH1R-Antagonisten durch ihre geringe Toxizität, die geringe Beeinflussung von metabolisierenden Enzymen und ihre geringen Nebenwirkungen aus. Insbesondere zeichnen sich bevorzugte erfindungsgemäße Verbindungen durch eine geringe Blockade des hERG-Kanals aus. Weiterhin sind bevorzugte Verbindungen der Formel I in wässrigen Systemen merklich löslich und damit für eine pharmazeutische Entwicklung besonders geeignet. Auch wird die pharmakologische Wirkung in in vivo Prüfmodellen aus gut verträglichen Vehikeln heraus nach oraler Gabe erreicht.

Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Als weitere Wirkstoffe für die Kombinationspräparate sind geeignet:
Alle Antidiabetika, die in der Roten Liste 2007, Kapitel 12 genannt sind; alle Abmagerungsmittel/Appetitzügler, die in der Roten Liste 2007, Kapitel 1 genannt sind; alle Diuretika, die in der Roten Liste 2007, Kapitel 36 genannt sind; alle Lipidsenker, die in der Roten Liste 2007, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen. Die meisten der nachfolgend aufgeführten Wirkstoffe sind in USP Dictionary of USAN and International Drug Names, US Pharmacopeia, Rockville 2006, offenbart.
Antidiabetika umfassen Insulin und Insulinderivate, wie z.B. Lantus^{®} (siehe www.lantus.com) oder HMR 1964 oder Levemir® (insulin detemir), Humalog^{(R)} (Insulin Lispro), Humulin^{(R)}, VIAject™, SuliXen^{(R)} oder solche, wie sie in WO2005005477 (Novo Nordisk) beschrieben sind, schnell wirkende Insuline (siehe US 6,221,633), inhalierbare Insuline, wie z. B. Exubera ^{®}, Nasulin™, oder orale Insuline, wie z. B. IN-105 (Nobex) oder Oral-lyn ™ (Generex Biotechnology) oder Technosphere^{(R)} Insulin (MannKind) oder Cobalamin™ orales Insulin oder Insuline, wie sie in WO2007128815, WO2007128817, WO2008034881, WO2008049711 beschrieben sind oder Insuline, die transdermal verabreicht werden können;
GLP-1-Derivate und GLP-1 Agonisten wie z.B. Exenatide oder spezielle Zubereitungen davon, wie sie z.B. in WO2008061355 beschrieben sind, Liraglutide, Taspoglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), AVE-0010, BIM-51077 (R-1583, ITM-077), PC-DAC:Exendin-4 (ein Exendin-4 Analogon, welches kovalent an rekombinantes menschliches Albumin gebunden ist), CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1 (GLP-1 gebunden an einen Nanocarrier), Agonisten wie sie z.B. bei D. Chen et al., Proc. Natl. Acad. Sei. USA 104 (2007) 943 beschrieben sind, solche wie sie in WO2006124529, WO2007124461 beschrieben sind, Peptide wie z.B. Obinepitide (TM-30338), Amylinrezeptor Agonisten, wie sie z.B. in WO2007104789 beschrieben sind, Analoga des humanen GLP-1, wie sie in WO2007120899, WO2008022015, WO2008056726 beschrieben sind, sowie oral wirksame hypoglykämische Wirkstoffe.
Antidiabetika umfassen auch Agonisten des Glukose-abhängigen insulinotropen Polypeptids (GIP) Rezeptors wie sie z.B. in WO2006121860 beschrieben sind.
Antidiabetika umfassen auch das Glukose-abhängige insulinotrope Polypeptid (GIP) wie auch analoge Verbindungen wie sie z.B. in WO2008021560 beschrieben sind.
Antidiabetika umfassen auch Analoga und Derivate des Fibroblastenwachstumsfaktors 21 (FGF-21, fibroblast growth factor 21).
Die oral wirksamen hypoglykämischen Wirkstoffe umfassen vorzugsweise Sulfonylharnstoffe, Biguanidine,
Meglitinide,
Oxadiazolidindione,
Thiazolidindione,
PPAR- und RXR-Modulatoren,
Glukosidase-Inhibitoren,
Hemmstoffe der Glykogenphosphorylase,
Glukagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. Pinacidil, Cromakalim, Diazoxid oder solche wie sie bei R. D. Carr et al., Diabetes 52, 2003, 2513-2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Modulatoren der natrium-abhängigen Glukosetransporter 1 oder 2 (SGLT1, SGLT2),
Hemmstoffe der 11-beta-Hydroxysteroid-Dehydrogenase- (11ß-HSD1),
Inhibitoren der Protein-Tyrosin-Phosphatase-1B (PTP-1B),
Nikotinsäurerezeptoragonisten,
Inhibitoren der hormon-sensitiven bzw. endothelialen Lipasen,
Hemmstoffen der Acetyl-CoA Carboxylase (ACC1 und/oder ACC2) oder
Inhibitoren der GSK-3 beta.
Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Farnesoid X Rezeptor (FXR) Modulatoren,
Fibrate,
Cholesterinresreptionsinhibitoren,
CETP-Inhibitoren,
Gallensäureresorptionsinhibitoren,
MTP-Inhibitoren,
Agonisten des Estrogenrezeptors gamma (ERR□ Agonisten),
Sigma-1 Rezeptorantagonisten,
Antagonisten des Somatostatin 5 Rezeptors (SST5 Rezeptor);
Verbindungen, die die Nahrungsmitteleinnahme verringern und
Verbindungen, die die Thermogenese erhöhen.

Bei einer Ausführungsform der Erfindung wird die Verbindung der Formel I in Kombination mit Insulin verabreicht.
Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid, Gliclazide oder Glimepirid, verabreicht.
Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331, WO2008050987 beschrieben).

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide verabreicht.
Bei einer weiteren Ausführungsform wird die Verbindung der Formel I mit einer Kombination von Mitiglinide mit einem Glitazon, z.B. Pioglitazon Hydrochlorid, verabreicht.
Bei einer weiteren Ausführungsform wird die Verbindung der Formel I mit einer Kombination von Mitiglinide mit einem alpha-Glukosidaseinhibitor verabreicht.
Bei einer weiteren Ausführungsform wird die Verbindung der Formel I in Kombination mit antidiabetischen Verbindungen, wie sie in WO2007095462, WO2007101060, WO2007105650 beschrieben sind, verabreicht.

Bei einer weiteren Ausführungsform wird die Verbindung der Formel I in Kombination mit antihypoglykämischen Verbindungen, wie sie in WO2007137008 beschrieben sind, verabreicht. Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.
Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon), DRL-17564, DRF-2593 (Balaglitazon), INT-131, T-2384 oder solchen, wie sie in WO2005086904, WO2007060992, WO2007100027, WO2007103252, WO2007122970, WO2007138485, WO2008006319, WO2008006969, WO2008010238, WO2008017398, WO2008028188 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043, WO2008006043, WO2008006044, WO2008012470, WO2008035359 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat), MBX-213, KY-201 oder wie in WO 00/64888, WO 00/64876, WO03/020269, WO2004024726, WO2007099553, US2007276041, WO2007085135, WO2007085136, WO2007141423, WO2008016175, WO2008053331 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, WO2007071766, WO2007101864, US2007244094, WO2007119887, WO2007141423, US2008004281, WO2008016175 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505 oder solchen wie sie in WO2008035359 beschrieben sind, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht.

Bei einer Ausführungsform wird die Verbindung der Formel I in Kombination mit einem α-Glukosidase-Inhibitor, wie z.B. Miglitol oder Acarbose oder solchen, wie sie z.B. in WO2007114532, WO2007140230, US2007287674, US2008103201 beschrieben sind, verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit GlukagonRezeptor-Antagonisten, wie z.B. A-770077 oder NNC-25-2504 oder wie in WO2004100875, WO2005065680, WO2006086488, WO2007047177, WO2007106181, WO2007111864, WO2007120270, WO2007120284, WO2007123581, WO2007136577, WO2008042223 beschrieben, verabreicht.

Bei einer weiteren Ausführungsform wird die Verbindung der Formel I in Kombination mit einer Antisense-Verbindung, z.B. ISIS-325568, verabreicht, welche die Produktion des Glukagonrezeptors inhibiert.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942, WO2008005914, WO2008005964, WO2008043701, WO2008044777, WO2008047821, US2008096877, WO2008050117, WO2008050101, WO2008059625 beschrieben sind, verabreicht.

Bei einer Ausführungsform wird die Verbindung der Formel I in Kombination mit einem Inhibitor der Glukoneogenese, wie sie z. B. in FR-225654, WO2008053446 beschrieben sind, verabreicht.

Bei einer Ausführungsform wird die Verbindung der Formel I in Kombination mit Inhibitoren der Fructose-1,6-bisphosphatase (FBPase) wie z.B. MB-07729, CS-917 (MB-06322) oder MB-07803 oder solchen wie sie in WO2006023515, WO2006104030, WO2007014619, WO2007137962, WO2008019309, WO2008037628 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200 (Melogliptin), GW-825964X, KRP-104, DP-893, ABT-341, ABT-279 oder ein anderes Salz davon, S-40010, S-40755, PF-00734200, BI-1356, PHX-1149, Alogliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, US2006890898, US2006803357, US2006303661, WO2007015767 (LY-2463665), WO2007024993, WO2007029086, WO2007063928, WO2007070434, WO2007071738, WO2007071576, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603, WO2007142253, WO2007148185, WO2008017670, US2008051452, WO2008027273, WO2008028662, WO2008029217, JP2008031064, JP2008063256, WO2008033851, WO2008040974, WO2008040995, WO2008064107 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas^{(R)}, einer festen Kombination von Vildagliptin mit Metförmin Hydrochlorid, verabreicht.

Bei einer Ausführungsform wird die Verbindung der Formel I in Kombination mit einer festen Kombination von eines Salzes von Sitagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761, WO2008045484 beschrieben sind, verabreicht.
Bei einer Ausführungsform wird die Verbindung der Formel I in Kombination mit Agonisten des glucose-abhängigen insulinotropischen Rezeptors (GDIR) wie z. B. APD-668 verabreicht. Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin oder Dapagliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116, WO2007143316, WO2007147478, WO2008001864, WO2008002824, WO2008013277, WO2008013280, WO2008013321, WO2008013322, WO2008016132, WO2008020011, JP2008031161, WO2008034859, WO2008042688, WO2008044762, WO2008046497, WO2008049923, WO2008055870, WO2008055940 oder von A. L. Handion in Expert Opin. Ther. Patents (2005) 15(11), 1531-1540 beschrieben sind, verabreicht.

Bei einer Ausführungsform wird die Verbindung der Formel I in Kombination mit Hemmstoffen der 11-beta-Hydroxysteroid-Dehydrogenase-1 (11ß-HSD1), wie z. B. BVT-2733, JNJ-25918646, INCB-13739, INCB-20817, DIO-92 ((-)-Ketoconazol) oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810, WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427, WO2007139992, WO2007144394, WO2007145834. WO2007145835, WO2007146761, WO2008000950, WO2008000951, WO2008003611, WO2008005910, WO2008006702, WO2008006703, WO2008011453, WO2008012532, WO2008024497, WO2008024892, WO2008032164, WO2008034032, WO2008043544, WO2008044656, WO2008046758, WO2008052638, WO2008053194 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058, US2008004325, WO2008033455, WO2008033931, WO2008033932, WO2008033934 beschrieben sind, verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A (Laropiprant) oder MK-0524 oder solchen Verbindungen, wie sie in WO2004041274, WO2006045565, WO2006045564, WO2006069242, WO2006085108, WO2006085112, WO2006085113, WO2006124490, WO2006113150, WO2007017261, WO2007017262, WO2007017265, WO2007015744, WO2007027532, WO2007092364, WO2007120575, WO2007134986, WO2007150025, WO2007150026, WO2008016968, WO2008051403 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.

Bei einer Ausführungsform der Erfindung wird die Verbindung der Formel I in Kombination mit Nicotinsäure oder einem anderen Nicotinsäurerezeptoragonisten und einem Prostaglandin DP Rezeptorantagonisten, wie z.B. solchen wie sie in WO2008039882 beschrieben sind, verabreicht.

Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform wird die Verbindung der Formel I in Kombination mit Modulatoren des GPR40, wie sie z.B. in WO2007013689, WO2007033002, WO2007106469, US2007265332, WO2007123225, WO2007131619, WO2007131620, WO2007131621, US2007265332, WO2007131622, WO2007136572, WO2008001931, WO2008030520, WO2008030618, WO2008054674, WO2008054675 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1, PSN-821, MBX-2982 oder solchen wie sie z. B. in WO2005061489 (PSN-632408), WO2004065380, WO2007003960-62 und WO2007003964, WO2007116229, WO2007116230, WO2008005569, WO2008005576, WO2008008887, WO2008008895, WO2008025798, WO2008025799, WO2008025800, WO2007035355, WO2006083491, WO200807692, WO2008076243 beschrieben sind, verabreicht.

Bei einer weiteren Ausführungsform wird die Verbindung der Formel I in Kombination mit Modulatoren des GPR120, wie sie z.B. in EP1688138 beschrieben sind, verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178, WO2007119837 beschrieben, verabreicht.

Bei einer Ausführungsform wird die Verbindung der Formel I in Kombination mit Inhibitoren der endothelialen Lipase, wie z. B. in WO2006111321, WO2006131233, WO2006131232, WO2006131231, WO2007042178, WO2007045392, WO2007045393, WO2007110216, WO2007110215 beschrieben, verabreicht.

Bei einer Ausführungsform wird die Verbindung der Formel I in Kombination mit einem Phospholipase A2 Inhibitor wie z.B. Darapladib oder A-002 oder solchen, wie sie in WO2008048866, WO20080488867 beschrieben sind, verabreicht.

Bei einer Ausführungsform wird die Verbindung der Formel I in Kombination mit Myricitrin, einem Lipase-Inhibitor (WO2007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110, US2007281949, WO2008002244, WO2008002245, WO2008016123, WO2008023239, WO2008044700, WO2008056266, WO2008057940 beschrieben.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Phosphoinositidkinase-3 (PI3K), wie z.B. solchen, wie in WO2008027584 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, WO2007093264, WO2008009335 beschrieben, verabreicht.

Bei einer Ausführungsform wird die Verbindung der Formel I in Kombination mit einem Modulator des Glucocorticoidrezeptors, wie z. B. in WO2008057855, WO2008057856, WO2008057857, WO2008057859, WO2008057862 beschrieben, verabreicht.

Bei einer Ausführungsform wird die Verbindung der Formel I in Kombination mit einem Inhibitor der Protein Kinase C beta (PKC beta), wie z. B. Ruboxistaurin, verabreicht.

Bei einer weiteren Ausführungsform wird die Verbindung der Formel I in Kombination mit einem Aktivator der AMP-aktivierten Proteinkinase (AMPK), wie sie z. B. in WO2007062568, WO2008006432, WO2008016278, WO2008016730 beschrieben sind, verabreicht.

Bei einer Ausführungsform wird die Verbindung der Formel I in Kombination mit einem Inhibitor der Ceramidkinase, wie sie z. B. in WO2007112914, WO2007149865 beschrieben sind, verabreicht.

Bei einer weiteren Ausführungsform wird die Verbindung der Formel I in Kombination mit einem Inhibitor der MAPK-interagierenden Kinase 1 oder 2 (MNK1 oder 2), wie sie z.B. in WO2007104053, WO2007115822, WO2008008547 beschrieben sind, verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022057, WO2004022553, WO2005097129, WO2005113544, US2007244140 beschrieben sind, verabreicht.

Bei einer anderen Ausführungsform wird die Verbindung der Formel I in Kombination mit Inhibitoren der NF-kappaB (NFKB) Aktivierung, wie sie z. B. Salsalate verabreicht.

Bei einer weiteren Ausführungsform wird die Verbindung der Formel I in Kombination mit Inhibitoren der ASK-1 (apoptosis signal-regulating kinase 1), wie sie z. B. in WO2008016131 beschrieben sind, verabreicht.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699, BMS-644950 oder solchen, wie sie in US2007249583 beschrieben sind, verabreicht.

Bei einer weiteren Ausführungsform der Erfindung wird die Verbindung der Formel I in Kombination mit einem Farnesoid X Rezeptor (FXR) Modulatoren, wie z.B. WAY-362450 oder solchen wie in WO2003099821, WO2005056554, WO2007052843, WO2007070796, WO2007092751, JP2007230909, WO2007095174, WO2007140174, WO2007140183, WO2008000643, WO2008002573, WO2008025539, WO2008025540 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965, WO2008041003, WO2008049047 beschrieben, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit Fibraten, wie z.B. dem Cholinsalz von Fenofibrat (SLV-348), verabreicht.

Bei einer Ausführungsform der Erfindung wird die Verbindung der Formel I in Kombination mit Fibraten, wie z.B. dem Cholinsalz von Fenofibrat und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358, WO2008033431, WO2008033465, WO2008052658, WO2008057336 beschrieben, verabreicht.

Bei einer Ausführungsform der Erfindung wird die Verbindung der Formel I in Kombination mit einem NPC1L1-Antagonisten, wie z.B. solchen, wie sie in WO2008033464, WO2008033465 beschrieben sind, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer Ausführungsform der Erfindung ist der weitere Wirkstoff ein Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer Ausführungsform der Erfindung wird die Verbindung der Formel I in Kombination mit einem CETP-Inhibitor, wie z.B. Torcetrapib, Anacetrapib oder JTT-705 (Dalcetrapib) oder solchen wie sie in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906, WO2008006257, WO2008009435, WO2008018529, WO2008058961, WO2008058967 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9, DE 10 2006 053635, DE 10 2006 053637, WO2007009655-56, WO2008058628, WO2008058629, WO2008058630, WO2008058631 beschrieben, verabreicht.

Bei einer Ausführungsform wird die Verbindung der Formel I in Kombination mit Agonisten des GPBAR1 (G-protein-coupled-bile-acid-receptor-1; TGR5), wie sie z.B. in WO2007110237, WO2007127505, WO2008009407 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.

Bei einer Ausführungsform der Erfindung wird die Verbindung der Formel I in Kombination mit Colesevelam Hydrochlorid und Metformin oder einem Sulfonylharnstoff oder Insulin verabreicht.

Bei einer Ausführungsform der Erfindung wird die Verbindung der Formel I in Kombination mit einem Phytosterole enthaltenden Kaugummi (Reductol^{™}) verabreicht.

Bei einer Ausführungsform der Erfindung wird die Verbindung der Formel I in Kombination mit einem Inhibitor des mikrosomalen Triglycerid-Transfer-Proteins (MTP-Inhibitor), wie z.B. Implitapide, BMS-201038, R-103757, AS-1552133, SLx-4090, AEGR-733 oder solchen wie in WO2005085226, WO2005121091, WO2006010423, WO2006113910, WO2007143164, WO2008049806, WO2008049808, beschrieben, verabreicht.

Bei einer weiteren Ausführungsform der Erfindung wird die Verbindung der Formel I in Kombination mit einer Kombinbation eines Cholesterolabsorptionsinhibitors, wie z.B. Ezetimibe, und einem Inhibitor des Triglycerid-Transfer-Proteins (MTP-Inhibitor), wie z.B. Implitapide, wie in WO2008030382 beschrieben, verabreicht.

Bei einer Ausführungsform der Erfindung wird die Verbindung der Formel I in Kombination mit einem antihypertriglyceridämischen Wirkstoff, wie z.B. solchen wie sie in WO2008032980 beschrieben sind, verabreicht.

Bei einer anderen Ausführungsform der Erfindung wird die Verbindung der Formel I in Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382, verabreicht.

Bei einer weiteren Ausführungsform der Erfindung wird die Verbindung der Formel I in Kombination mit einem Inhibitor der Leber-Carnitin Palmitoyltransferase-1 (L-CPT1), wie sie z.B. in WO2007063012, WO2007096251 (ST-3473), WO2008015081, US2008103182 beschrieben sind, verabreicht.

Bei einer weiteren Ausführungsform der Erfindung wird die Verbindung der Formel I in Kombination mit einem Modulator der Serin-Palmitoyltransferase (SPT), wie sie z.B. in WO2008031032, WO2008046071 beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943, WO2008003424 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738, WO2008020607 beschrieben, verabreicht.

Bei einer anderen Ausführungsform der Erfindung wird die Verbindung der Formel I in Kombination mit einem HDL-Cholesterol-erhöhenden Agens, wie z.B. solchen wie sie in WO2008040651 beschrieben sind, verabreicht.

Bei einer Ausführungsform der Erfindung wird die Verbindung der Formel I in Kombination mit einem ABCA1 Expressionsverstäker, wie sie z.B. in WO2006072393 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A1 Rezeptor Agonisten (Adenosin A1 R), wie sie z.B. in EP1258247, EP1375508, WO2008028590 beschrieben sind, verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in US2007270433, WO2008027585 beschrieben sind, verabreicht.

Bei einer Ausführungsform wird die Verbindung der Formel I in Kombination mit Hemmstoffen der Acetyl-CoA Carboxylase (ACC1 und/oder ACC2) wie z. B. solchen wie in WO199946262, WO200372197, WO2003072197, WO2005044814, WO2005108370, JP2006131559, WO2007011809, WO2007011811, WO2007013691, WO2007095601-603, WO2007119833 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in WO2007100833) verabreicht.

Bei einer weiteren Ausführungsform wird die Verbindung der Formel I in Kombination mit Modulatoren der Xanthin-Oxidoreductase (XOR) verabreicht.

Bei einer anderen Ausführungsform wird die Verbindung der Formel I in Kombination mit Inhibitoren der löslichen Epoxidhydrolase (sEH), wie sie z.B. in WO2008051873, WO2008051875 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure- {4-[(4-amino-quinazolin-2-ylamino)-methyl]-cyclohexylmethyl}-amid Hydrochlorid (CGP 71683A);
NPY-5 Rezeptorantagonisten wie L-152804 oder die Verbindung "NPY-5-BY" der Firma Banyu oder wie sie z. B. in WO2006001318, WO2007103295, WO2007125952, WO2008026563, WO2008026564, WO2008052769 beschrieben sind;
NPY-4-Rezeptorantagonisten wie sie z. B. in WO2007038942 beschrieben sind;
NPY-2-Rezeptorantagonisten wie sie z. B. in WO2007038943 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166, WO2008003947 beschrieben sind;
Derivaten des Peptids Obestatin wie sie WO2006096847 beschrieben sind;
CB1R (Cannabinoid Rezeptor 1) Antagonisten wie z.B. Rimonabant, Surinabant (SR147778), SLV-319 (Ibipinabant), AVE-1625, Taranabant (MK-0364) oder Salze davon, Otenabant (CP-945,598), V-24343 oder solche Verbindungen wie sie in z. B. EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006018662, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007018460, WO2007016460, WO2007020502, WO2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863, WO2007119001, WO2007120454, WO2007121687, WO2007123949, US2007259934, WO2007131219, WO2007133820, WO2007136571, WO2007136607, WO2007136571, US7297710, WO2007138050, WO2007139464, WO2007140385, WO2007140439, WO2007146761, WO2007148061, WO2007148062, US2007293509, WO2008004698, WO2008017381, US2008021031, WO2008024284, WO2008031734, WO2008032164, WO2008034032, WO2008035356, WO2008036021, WO2008036022, WO2008039023, WO2998043544, WO2008044111, WO2008048648, EP1921072-A1, WO2008053341, WO2008056377, WO2008059207, WO2008059335 beschrieben sind;
Cannabinoid Rezeptor 1 / Cannabinoid Rezeptor 2 (CB1/CB2) modulierende Verbindungen wie z.B. delta-9-Tetrahydrocannabivarin oder solchen wie sie z.B. in WO2007001939, WO2007044215, WO2007047737, WO2007095513, WO2007096764, WO2007112399, WO2007112402 beschrieben sind;
Modulatoren der FAAH (fatty acid amide hydrolase) wie sie z.B. in WO2007140005, WO2008019357, WO2008021625, WO2008023720, WO2008030532 beschrieben sind;
Vanilloid-1-Rezeptor Modulatoren (Modulatoren des TRPV1), wie sie z.B. in WO2007091948, WO2007129188, WO2007133637, WO2008007780, WO2008010061, WO2008007211, WO2008010061, WO2008015335, WO2008018827, WO2008024433, WO2008024438, WO2008032204, WO2008050199, WO2008059370 beschrieben sind;
Antagonisten oder inverse Agonisten der Opioidrezeptoren, wie sie z.B. in WO2008021849, WO2008021851, WO2008032156 beschrieben sind;
Agonisten des Prostaglandinrezeptors, wie z.B. Bimatoprost oder solchen Verbindungen wie sie in WO2007111806 beschrieben sind;
MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763, WO2007141343, WO2008007930, WO2008017852, WO2008039418 beschrieben sind;
Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374;WO2007122591, WO2007126934, WO2007126935, WO2008008517, WO2008008518, WO2008008551, WO2008020405, WO2008026149, WO2008038251 beschrieben sind);
Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, US2005171181 (z.B. PF-00389027), WO2006107661, WO2007003804, WO2007016496, WO2007020213, WO2007049798, WO2007055418, WO2007057329, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111, WO2007137955, US2007281923, WO2007137968, WO2007138431, WO2007146122, WO2008005338, WO2008012010, WO2008015125, WO2008045371 beschrieben sind);
Histamin H1 /Histamin H3 Modulatoren, wie z. B. Betahistin bzw. seinem Dihydrochlorid;
Modulatoren des Histamin H3 Transporters oder der Histamin H3 / Serotonin Transporter wie sie z.B. in WO2008002816, WO2008002817, WO2008002818, WO2008002820 beschrieben sind;
Histamin H4 Modulatoren wie sie z.B. in WO2007117399 beschrieben sind;
CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CRF1-Antagonisten, wie sie in WO2007105113, WO2007133756, WO2008036541, WO2008036579 beschrieben sind);
CRF BP-Antagonisten (z.B. Urocortin);
Urocortin-Agonisten;
Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2002038544, WO2007048840-843, WO2008015558 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71 (AMG-071, AMG-076), GW-803430, GW-856464, NGD-4715, ATC-0453, ATC-0759 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916, WO2007141200, WO2007142217, US2007299062, WO2007146758, WO2007146759, WO200800116, WO2008016811, WO2008020799, WO2008022979, WO2008038692, WO2008041090, WO2008044632, WO2008047544, JP2008088120, WO2008065021, WO2008068265, WO2008061109, WO2008076562, WO2008071646 beschrieben sind);
CCK-A (CCK-1) Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034, WO2007120655, WO2007120688, WO2007120718 beschrieben sind;
Serotonin-Wiederaufnahme-Inhibitoren (z.B. Dexfenfluramine) oder solchen wie sie in WO2007148341, WO2008034142 beschrieben sind;
gemischte Serotonin-/Dopamin-Wiederaufnahme-Inhibitoren (z.B. Bupropion) oder solche wie sie in WO2008063673 beschrieben sind oder feste Kombinationen von Bupropion mit Naltrexon oder Bupropion mit Zonisamid;
gemischte Wiederaufnahmeinhibitoren wie z.B. DOV-21947;
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine) oder solchen wie sie z.B. in WO2006085118 beschrieben sind;
Norepinephrin-Wiederaufnahme-Inhibitoren wie sie z.B. in US2008076724 beschrieben sind;
5-HT2A Rezeptor Antagonisten wie sie z.B. in WO2007138343 beschrieben sind;
5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213, WO2008007661, WO2008007664, WO2008009125, WO2008010073 beschrieben sind);
5-HT6 Rezeptor Modulatoren, wie z.B. E-6837, BVT-74316 oder PRX-07034 oder solche wie sie z.B. in WO2005058858, WO2007054257, WO2007107373, WO2007108569, WO2007108742-744, WO2008003703, WO2008027073, WO2008034815, WO2008054288 beschrieben sind;
Agonisten des Estrogenrezeptors gamma (ERR□ Agonisten), wie sie z.B. in WO2007131005, WO2008052709 beschrieben sind;
Sigma-1 Rezeptorantagonisten, wie sie z.B. in WO2007098953, WO2007098961, WO2008015266, WO2008055932, WO2008055933 beschrieben sind;
Muscarin 3 Rezeptor (M3R) Antagonisten, wie sie z.B. in WO2007110782, WO2008041184 beschrieben sind;
Bombesin-Rezeptor Agonisten (BRS-3 Agonisten), wie sie z.B. in WO2008051404, WO2008051405, WO2008051406 beschrieben sind;
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734, WO2007127457, WO2008008286 beschrieben sind;
Growth Hormone Secretagogue Receptor Modulatoren wie z.B. JMV-2959, JMV-3002, JMV-2810, JMV-2951 oder solchen, wie sie in WO2006012577 (z.B. YIL-781 oder YIL-870), WO2007079239 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311, WO2007141502, WO2007141517, WO2007141538, WO2007141545, WO2007144571, WO2008011130, WO2008011131, WO2008039007, WO2008048991 beschrieben;
Inhibitoren der Monoacylglycerolacyltransferase (2-Acylglycerol-O-Acyltransferase; MGAT) wie sie z.B. in WO2008038768 beschrieben sind;
Inhibitoren der Fettsäuresynthase (FAS) wie z.B. C75 oder solchen, wie in WO2004005277, WO2008006113 beschrieben;
Inhibitoren der Stearoyl-CoA delta9 Desaturase (SCD1) wie sie z.B. in WO2007009236, WO2007044085, WO2007046867, WO2007046868, WO20070501124, WO2007056846, WO2007071023, WO2007130075, WO2007134457, WO2007136746, WO2007143597, WO2007143823, WO2007143824, WO2008003753, WO2008017161, WO2008024390, WO2008029266, WO2008036715, WO2008043087, WO2008044767, WO2008046226, WO2008056687 beschrieben sind;
hypoglykämische/hypertriglyceridämische Indolinverbindungen wie sie in WO2008039087 beschrieben sind;
Inhibitoren des "Adipocyte fatty acid-binding protein aP2" wie z.B. BMS-309403; Aktivatoren der Adiponectinsekretion, wie z.B. in WO2006082978 beschrieben;
Promotoren der Adiponectinproduktion, wie z.B. in WO2007125946, WO2008038712 beschrieben;
Oxyntomodulin oder Analoga davon;
Oleoyl-Estron
oder Agonisten oder partiellen Agonisten des Schilddrüsenhormonrezeptors (thyroid hormone receptor agonists) wie z. B: KB-2115 (Eprotirome), QRX-431 (Sobetirome) oder DITPA oder solche, wie in WO20058279, WO200172692, WO200194293, WO2003084915, WO2004018421, WO2005092316, WO2007003419, WO2007009913, WO2007039125, WO2007110225, WO2007110226, WO2007128492, WO2007132475, WO2007134864, WO2008001959 beschrieben
oder Agonisten des Schilddrüsenhormonrezeptors beta (TR-beta) wie z. B. MB-07811 oder MB-07344, verabreicht.

Bei einer Ausführungsform der Erfindung wird die Verbindung der Formel I in Kombination mit einer Kombination von Epotirome mit Ezetimibe verabreicht.

Bei einer Ausführungsform der Erfindung wird die Verbindung der Formel I in Kombination mit einem Inhibitor der Site-1 Protease (S1P), wie z.B. PF-429242, verabreicht.

Bei einer weiteren Ausführungsform der Erfindung wird die Verbindung der Formel I in Kombination mit einem RNAi Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel I in Kombination mit Lycopin verabreicht.

Bei einer Ausführungsform der Erfindung wird die Verbindung der Formel I in Kombination mit einem Antioxidans, wie z.B. OPC-14117, AGI-1067 (Succinobucol), Probucol, Tocopherol, Ascorbinsäure, β-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.
Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948 beschrieben, verabreicht.

Bei einer anderen Ausführungsform wird die Verbindung der Formel I in Kombination mit Topiramat oder einem Derivat davon, wie es in WO2008027557 beschrieben ist, verabreicht.

Bei einer weiteren Ausführungsform wird die Verbindung der Formel I in Kombination mit einer festen Kombination von Topiramat mit Phentermin (Qnexa^{™}) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer anderen Ausführungsform wird die Verbindung der Formel I in Kombination mit einem Aldosteronsynthaseinhibitor und einem Antagonisten des Glucocorticoidrezeptors, einem Cortisolsyntheseinhibitor und/oder einem Antagonisten des Corticotropin-freisetzenden Faktors (corticotropin releasing factor), wie z.B. in EP1886695 beschrieben, verabreicht.

Bei einer Ausführungsform wird die Verbindung der Formel I in Kombination mit einem Agonisten des RUP3 Rezeptors, wie z. B. in WO2007035355, WO2008005576 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in WO2007119463 beschrieben, verabreicht.

Bei einer Ausführungsform wird die Verbindung der Formel I in Kombination mit einem "c-Jun N-terminal kinase" Inhibitor (JNK-Inhibitor), wie z. B. in WO2007125405, WO2008028860 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in WO2005090336, WO2006071609, WO2006135826, WO2007105766 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1 (einer NAD⁺-abhängigen Proteindeacetylase); dieser Wirkstoff kann z.B. Resveratrol in geeigneten Formulierungen sein, oder solche Verbindungen wie sie in WO2007019416 (z.B. SRT-1720) genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2007107587, WO2007111994 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in WO2007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulator"; SARM), wie sie z.B. in WO2007099200, WO2007137874 beschrieben sind, verabreicht.

Bei einer weiteren Ausführungsform wird die Verbindung der Formel I in Kombination mit einem AGE (advanced glycation endproduct) Inhibitor, wie sie z.B. in JP2008024673 beschrieben sind, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere Wirkstoff Metreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.
Bei einer Ausführungsform ist der weitere Wirkstoff Fenfluramin oder Dexfenfluramin.

Bei noch einer Ausführungsform ist der weitere Wirkstoff Sibutramin oder solche Derivate wie sie in WO2008034142 beschrieben sind.
Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104) oder Derivate davon (JP2008106008).

Bei einer Ausführungsform ist der weitere Wirkstoff ein nasal verabreichter Calciumkanalblocker wie z.B. Diltiazem oder solche, wie sie in US 7,138,107 beschrieben sind.

Bei einer Ausführungsform ist der weitere Wirkstoff ein Inhibitor des Natrium-Calcium-Ionen-Austausches wie z.B. solche, wie sie in WO2008028958 beschrieben sind.

Bei einer weiteren Ausführungsform ist der weitere Wirkstoff ein Blocker von Calciumkanälen wie z.B. des CaV3.2 wie sie in WO2008033431, WO2008033447, WO2008033356, WO2008033460, WO2008033464, WO2008033465, WO2008033468 beschrieben sind.

Bei einer Ausführungsform ist der weitere Wirkstoff ein Blocker des "T-type calcium channel" wie sie z.B. in WO2008033431 beschrieben sind.

Bei einer Ausführungsform ist der weitere Wirkstoff ein Inhibitor des KCNQ-Kaliumkanal-2 bzw. -3 wie z.B. solche, wie sie in US2008027049, US2008027090 beschrieben sind.

Bei einer Ausführungsform ist der weitere Wirkstoff ein Inhibitor des Kalium Kvl.3 Ionenkanals wie z.B. solchen, wie sie in WO2008040057, WO2008040058, WO2008046065 beschrieben sind.

Bei einer anderen Ausführungsform ist der weitere Wirkstoff ein Modulator des MCP-1 Rezeptors (monocyte chemoattractant protein-1 (MCP-1)) wie z.B. solche, wie sie in WO2008014360, WO2008014381 beschrieben sind.

Bei einer Ausführungsform ist der weitere Wirkstoff ein Modulator des Somatostatinrezeptors 5 (SSTR5) wie z.B. solche, wie sie in WO2008019967, US2008064697 beschrieben sind.

Bei einer Ausführungsform ist der weitere Wirkstoff ein Modulator des Somatostatinrezeptors 2 (SSTR2) wie z.B. solche, wie sie in WO2008051272 beschrieben sind.

Bei einer Ausführungsform ist der weitere Wirkstoff ein Erythropoietin-mimetisches Peptid, welches als Erythropoietin (EPO) Rezeptoragonist agiert. Solche Moleküle sind z.B. in WO2008042800 beschrieben.

Bei einer weiteren Ausführungsform ist der weitere Wirkstoff ein Anorektikum/eine hypoglykämische Verbindung wie z.B. solche, wie sie in WO2008035305, WO2008035306, WO2008035686 beschrieben sind.

Bei einer Ausführungsform ist der weitere Wirkstoff ein Induktor der Liponsäuresynthetase wie z.B. solche, wie sie in WO2008036966, WO2008036967 beschrieben sind.

Bei einer Ausführungsform ist der weitere Wirkstoff ein Stimulator der endothelialen Nitric-Oxid-Synthase (eNOS) wie z.B. solche, wie sie in WO2008058641 beschrieben sind.

Bei einer Ausführungsform ist der weitere Wirkstoff ein Modulator des Kohlenhydrat- und/oder Lipidstoffwechsels wie z.B. solche, wie sie in WO2008059023, WO2008059024, WO2008059025, WO2008059026 beschrieben sind.

Bei einer weiteren Ausführungsform ist der weitere Wirkstoff ein Angiotensin II Rezeptorantagonist wie z.B. solche, wie sie in WO2008062905 beschrieben sind.

Bei einer Ausführungsform ist der weitere Wirkstoff ein Agonist des Sphingosin-1-Phosphatrezeptors (S1P) wie z.B. solche, wie sie in WO2008064315 beschrieben sind.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

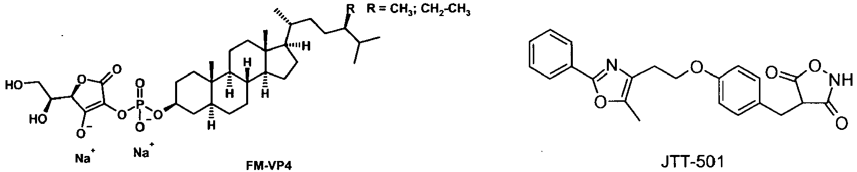

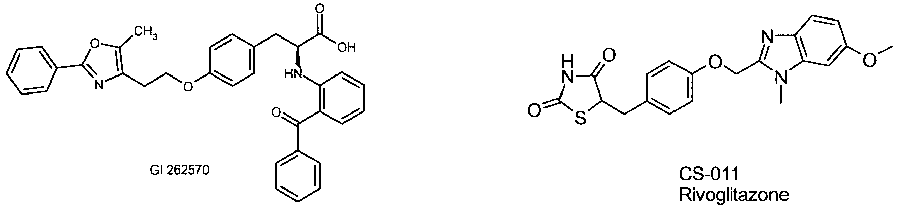

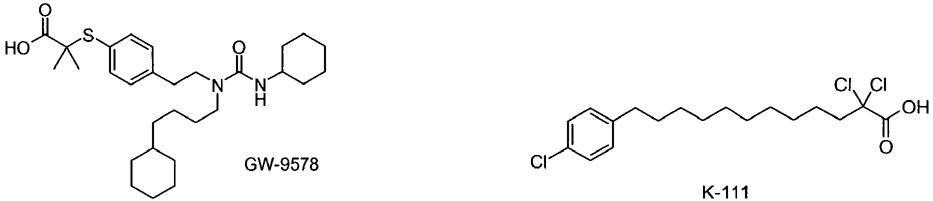

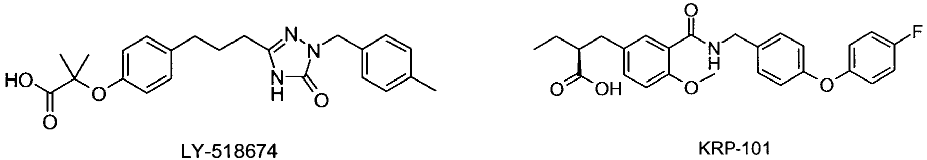

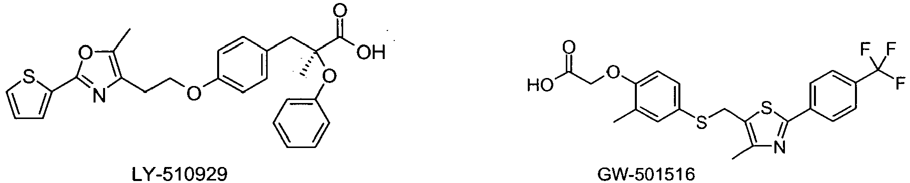

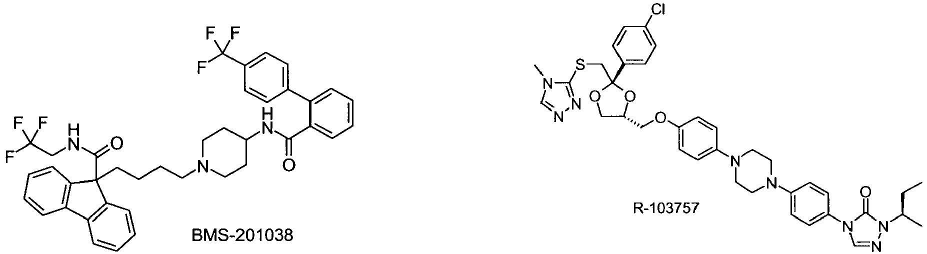

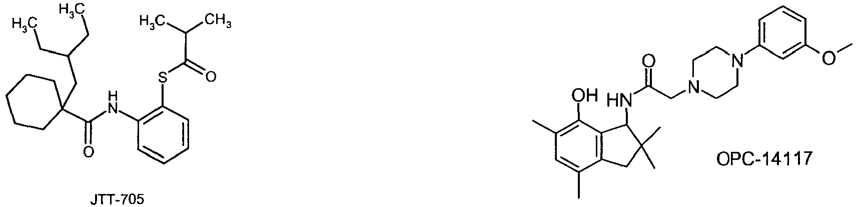

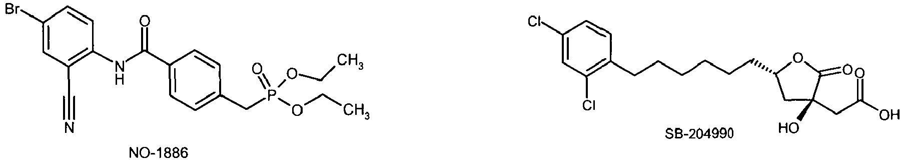

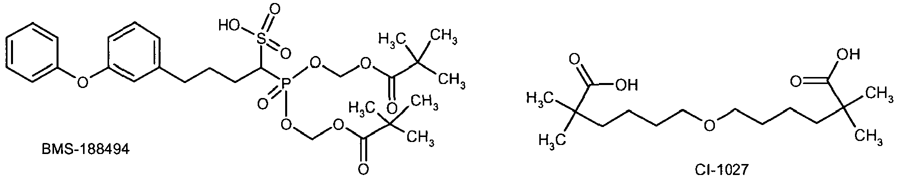

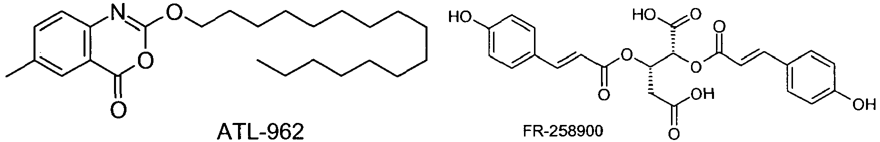

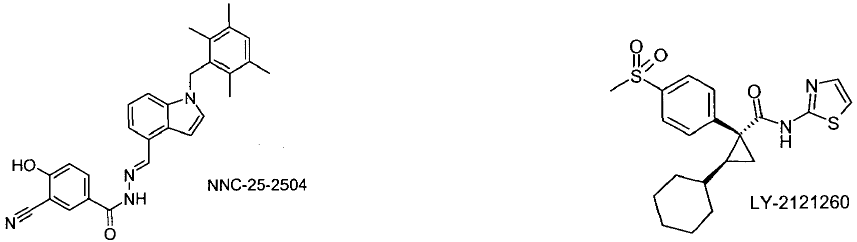

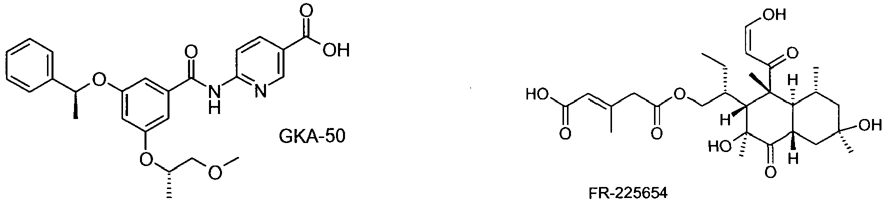

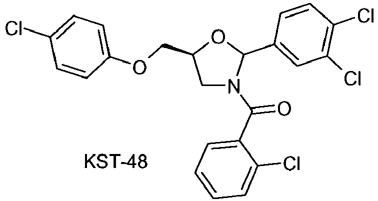

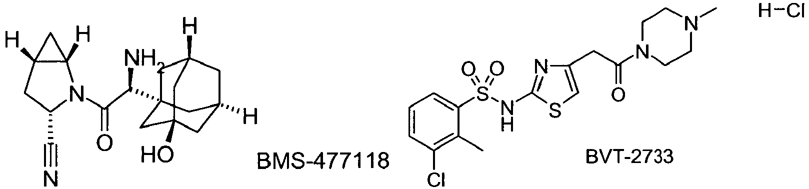

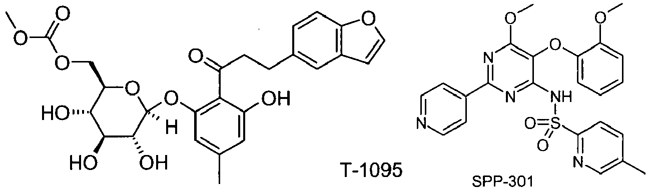

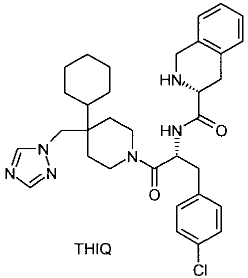

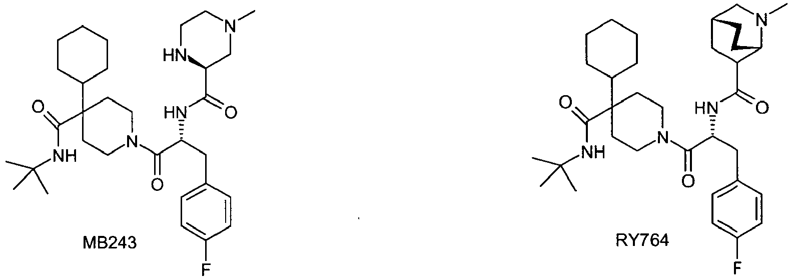

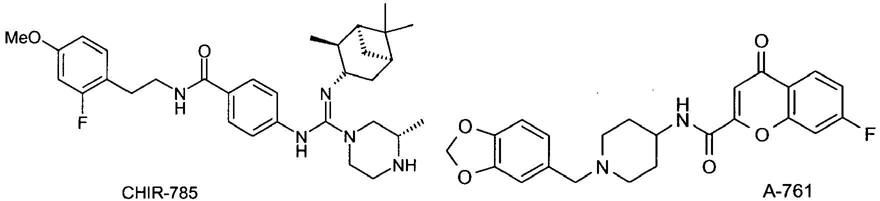

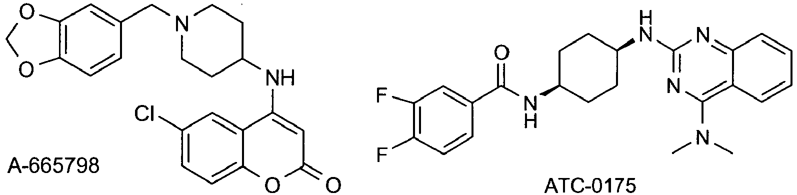

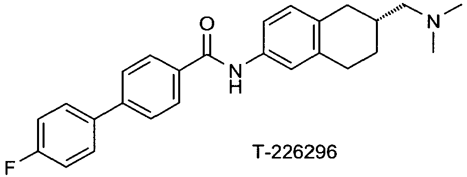

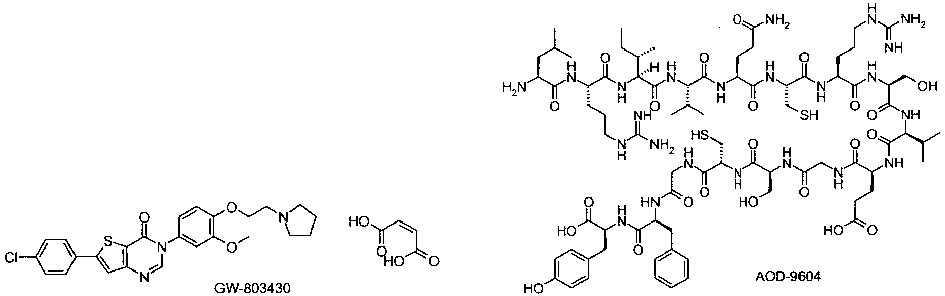

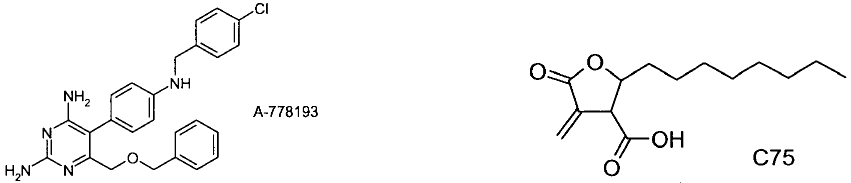

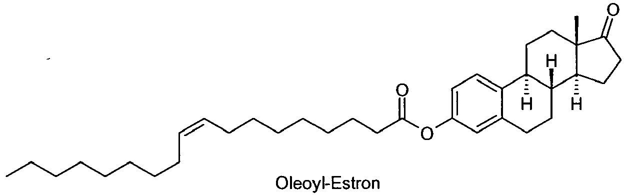

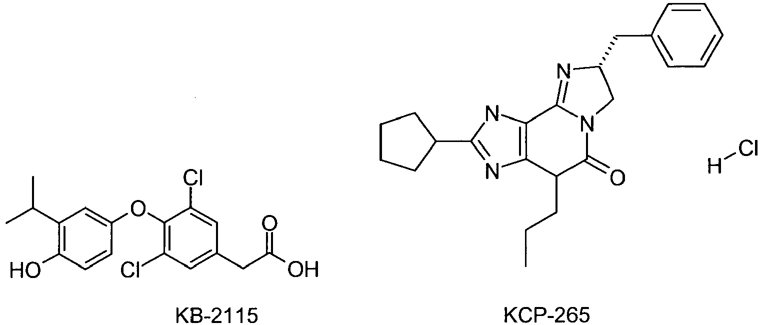

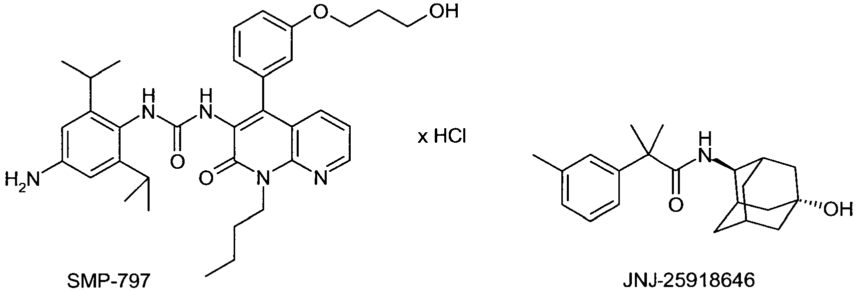

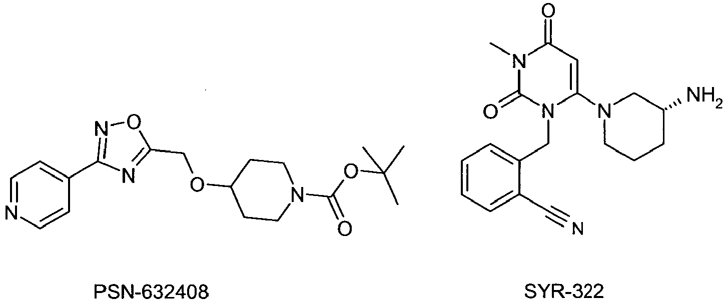

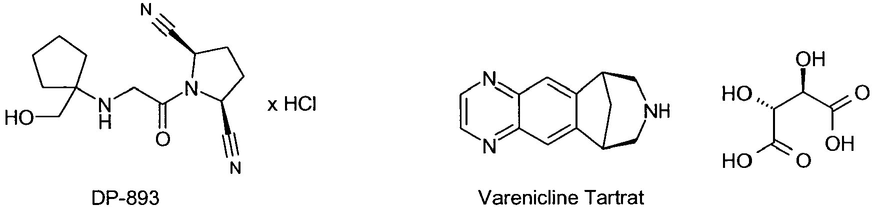

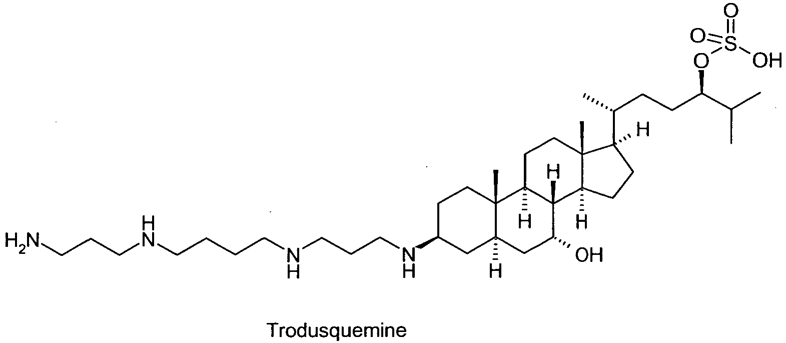

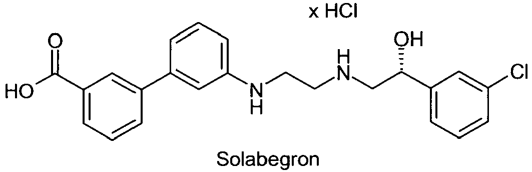

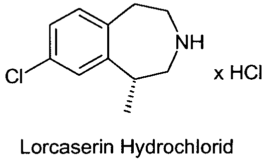

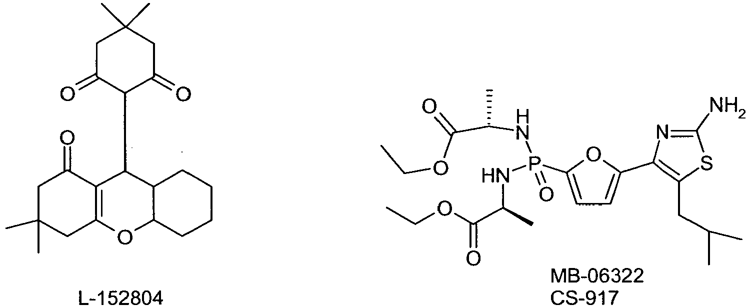

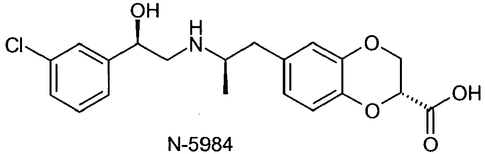

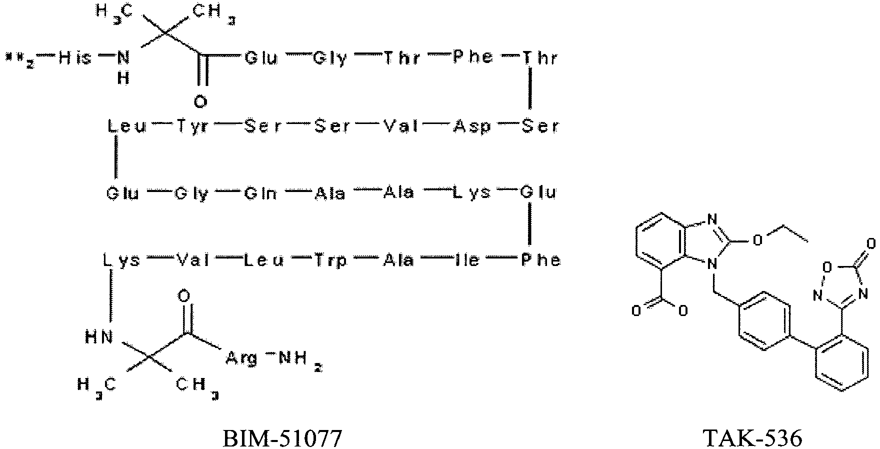

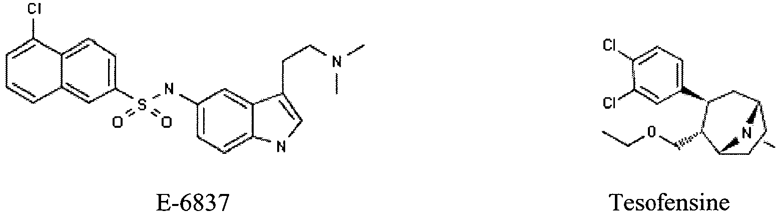

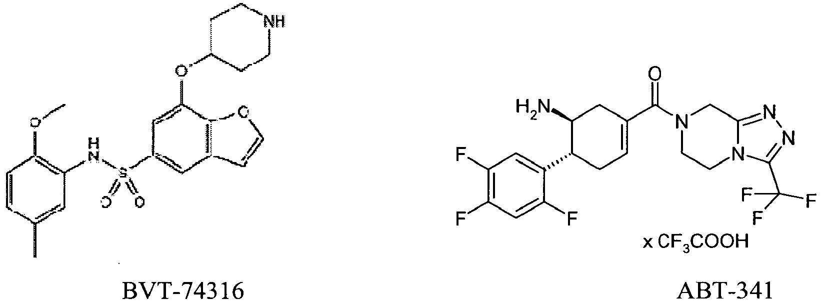

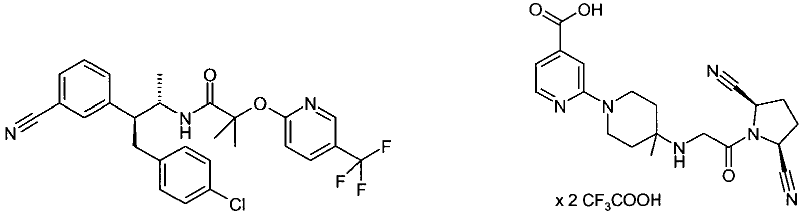

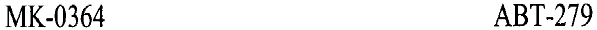

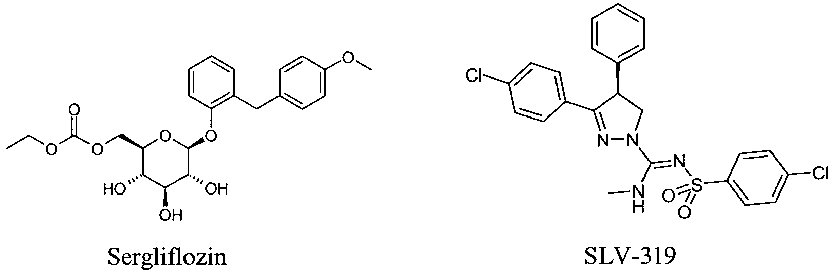

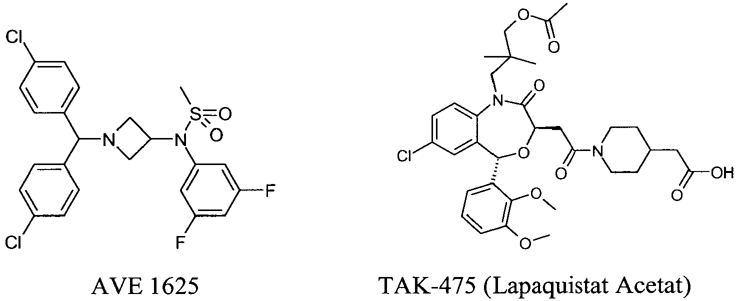

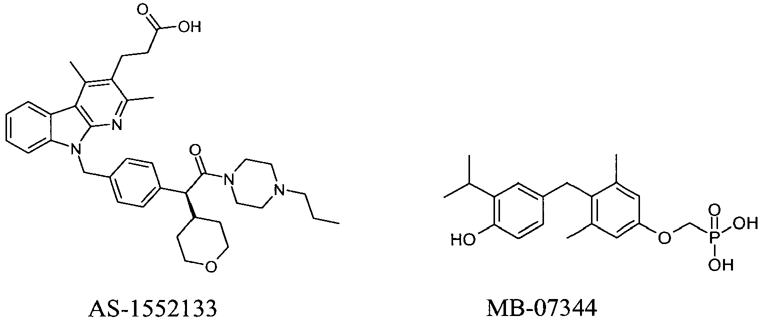

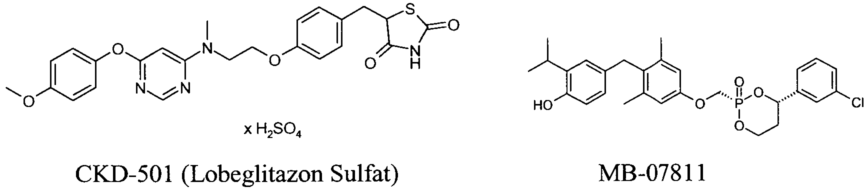

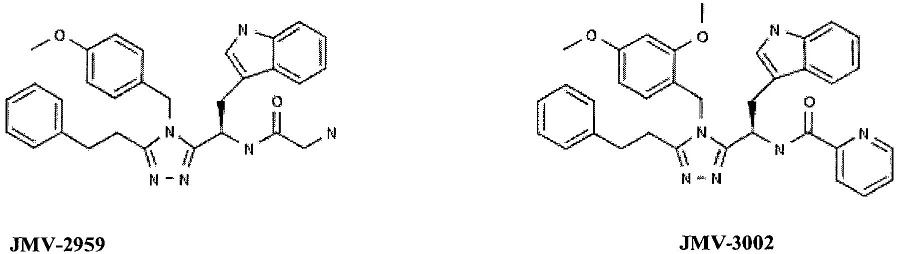

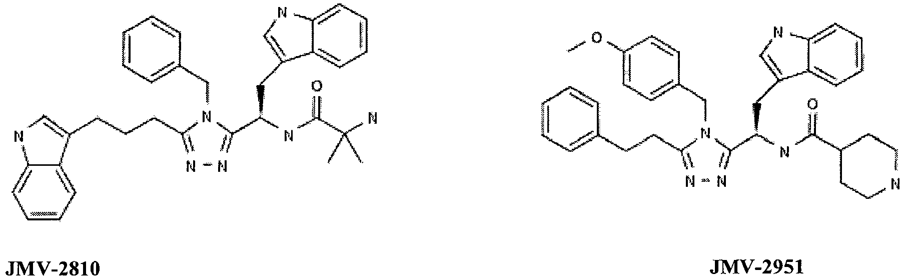

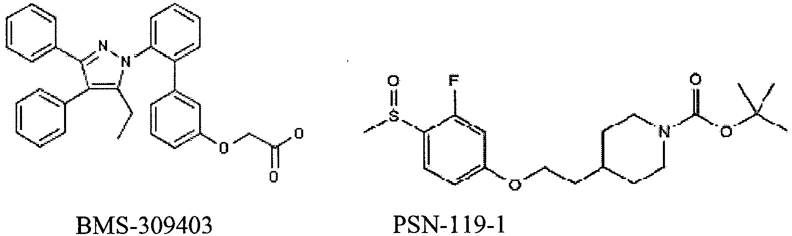

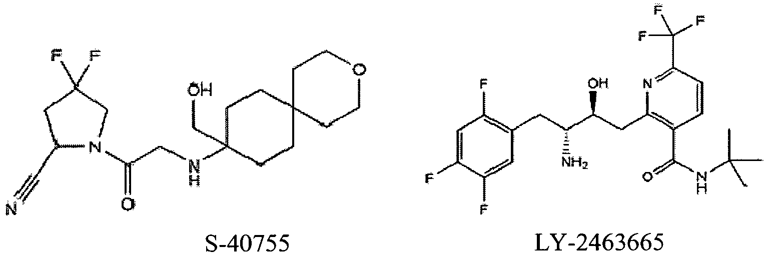

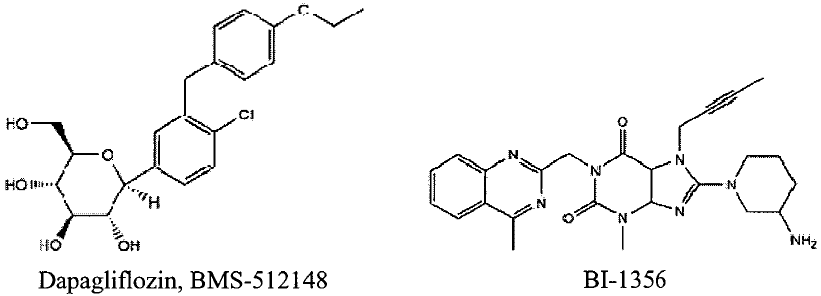

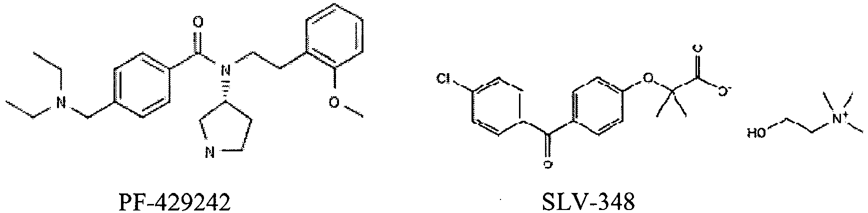

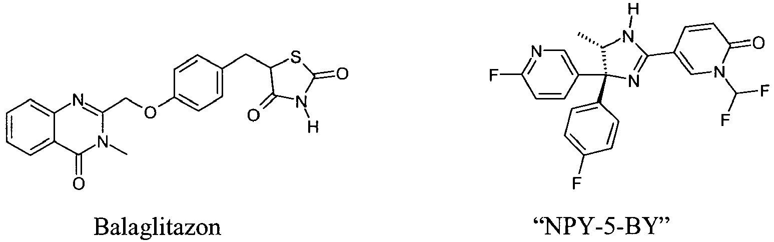

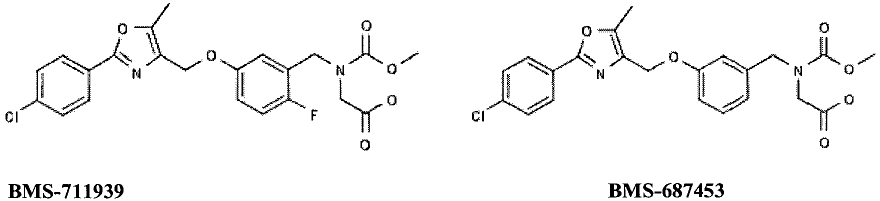

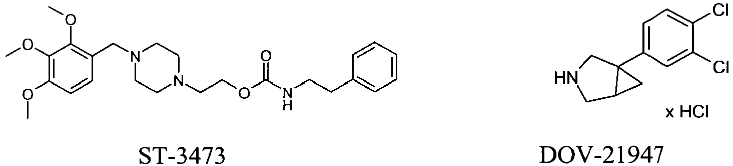

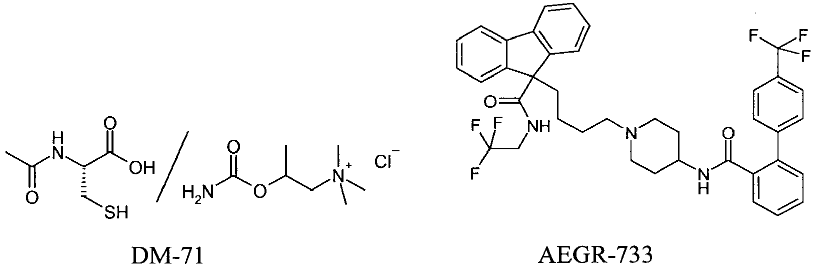

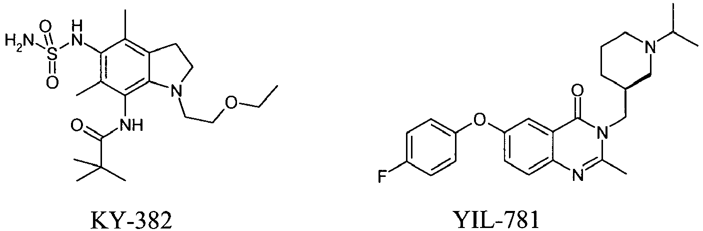

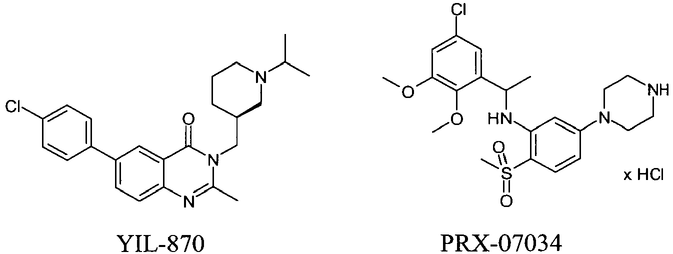

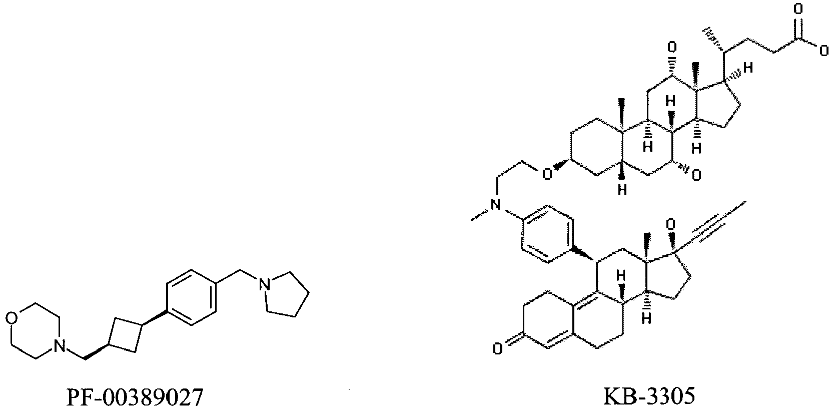

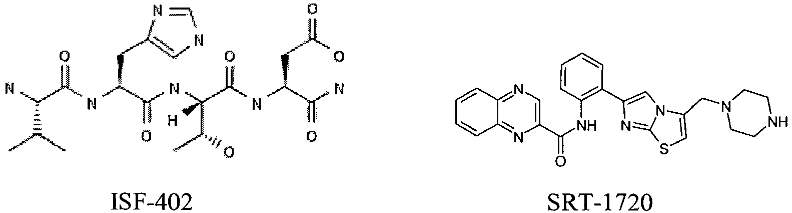

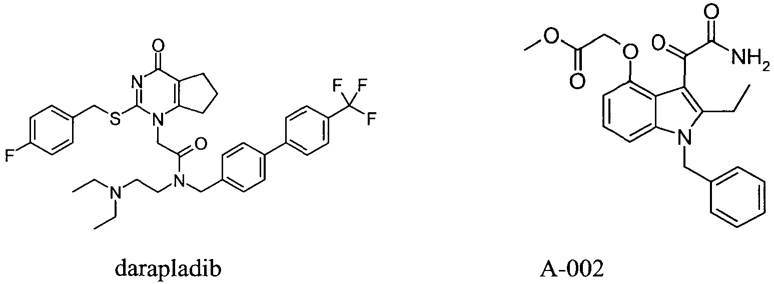

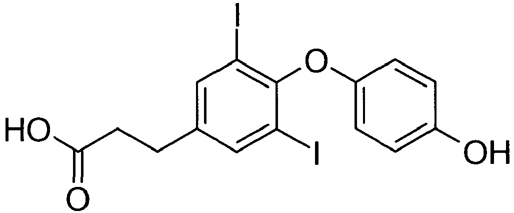

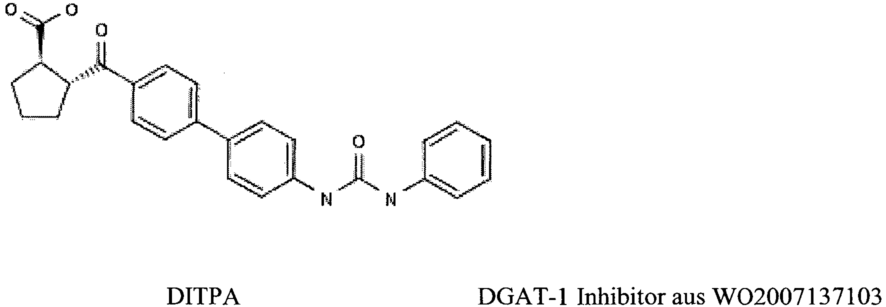

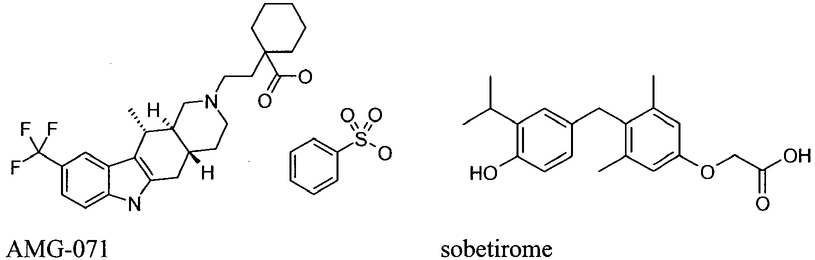

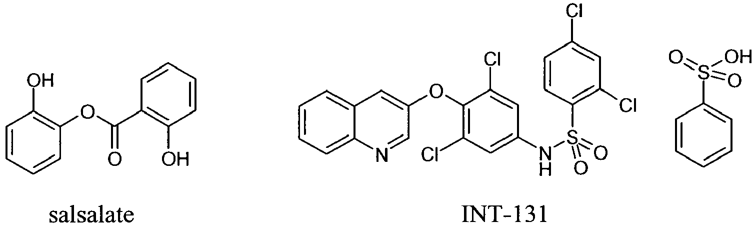

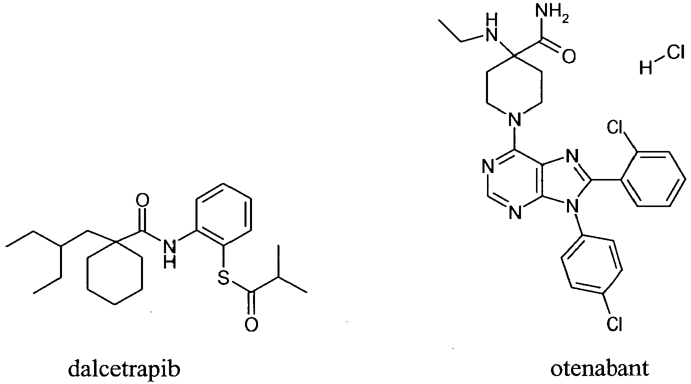

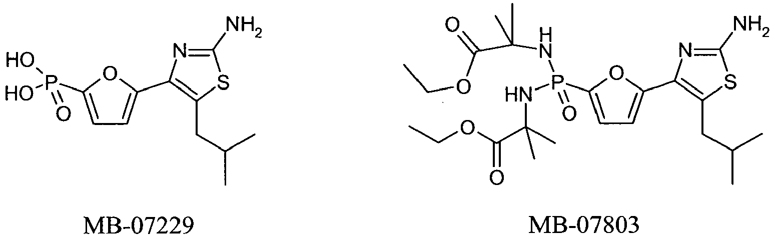

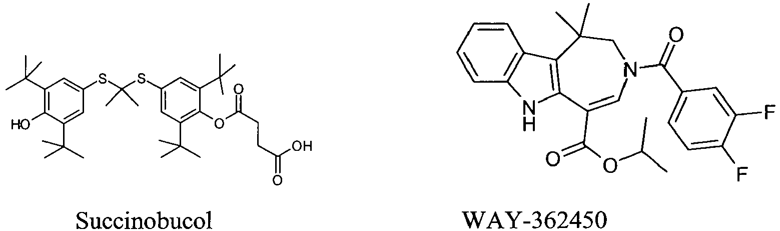

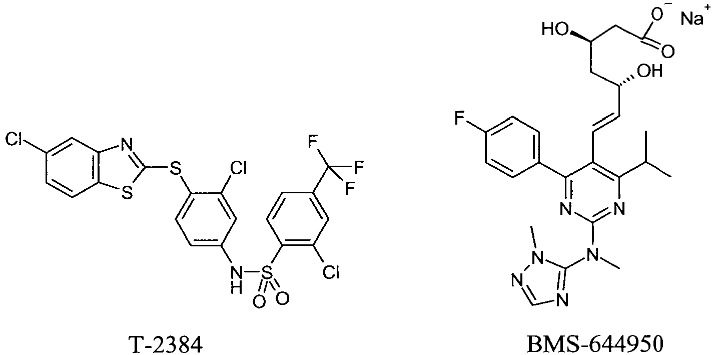

Weiterhin sind folgende Wirkstoffe für Kombinationspräparate geeignet:
Alle Antiepileptika, die in der Roten Liste 2007, Kapitel 15 genannt sind;
alle Antihypertonika, die in der Roten Liste 2007, Kapitel 17 genannt sind;
alle Hypotonika, die in der Roten Liste 2007, Kapitel 19 genannt sind;
alle Antikoagulantia, die in der Roten Liste 2007, Kapitel 20 genannt sind;
alle Arteriosklerosemittel, die in der Roten Liste 2007, Kapitel 25 genannt sind;
alle Betarezeptoren-, Calciumkanalblocker und Hemmstoffe des Renin-Angiotensin-Systems, die in der Roten Liste 2007, Kapitel 27 genannt sind;
alle Diuretika und Durchblutungsfördernde Mittel, die in der Roten Liste 2007, Kapitel 36 und 37 genannt sind;
alle Entwöhnungsmittel/Mittel zur Behandlung von Suchterkrankungen, die in der Roten Liste 2007, Kapitel 39 genannt sind;
alle Koronarmittel und Magen-Darm-Mittel, die in der Roten Liste 2007, Kapitel 55 und 60 genannt sind;
alle Migränemittel, Neuropathiepräparate und Parkinsonmittel, die in der Roten Liste 2007, Kapitel 61, 66 und 70 genannt sind.

Bei einer Ausführungsform werden die Verbindungen der Formel I in Kombination mit Medikamenten mit Wirkungen auf das Herz-Kreislauf und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit antientzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

### Prüfmodelle

Anhand verschiedener Prüfmodelle kann die Eignung der erfindungsgemäßen Verbindungen als pharmazeutischer Wirkstoff getestet werden. Im Folgenden werden beispielhaft Beschreibungen solcher Prüfmodelle gegeben.

### Beeinflussung des MCH-Rezeptors in vitro; Bestimmung von funktionellen IC50-Werten von MCH R1-Antagonisten

Die Klonierung der cDNA für den humanen MCH-Rezeptor, Herstellung einer rekombinanten HEK293-Zellinie, welche den humanen MCH-Rezeptor exprimiert, sowie funktionelle Messungen mit der rekombinanten Zellinie erfolgten sinngemäß wie von Audinot et al. (J. Biol. Chem. 2001, 276, 13554-13562) beschrieben. Im Unterschied zur Literaturstelle wurde jedoch für die Konstruktion des Expressionsvektors das Plasmid pEAK8 der Fa. EDGE Biosystems (USA) verwendet. Als Wirt für die Transfektion diente eine transformierte HEK-Zellinie namens "PEAK Stable Cells" (ebenfalls von EDGE Biosystems). Die funktionellen Messungen des zellulären Calciumflusses nach Agonistenzugabe (MCH) in Gegenwart von erfindungsgemäßem Ligand erfolgten mit Hilfe des FLIPR-Gerätes der Fa. Molecular Devices (USA), unter Verwendung von Vorschriften des Geräteherstellers. Die erfindungsgemäßen Verbindungen zeigen eine signifikante Inhibierung (>30%) des durch den Agonisten induzierten Signals bei einer Konzentration von 100 µM, bevorzugt bei 10 µM, besonders bevorzugt bei 1 µM, ganz besonders bevorzugt bei 100 nM und ganz ganz besonders bevorzugt bei 10 nM.
Neben der funktionellen Aktivität kann nach Audinot et al. (Br. J. Pharmacol. 2001, 133, 371-378) auch die Affinität der für den MCH1R bestimmt werden. Bevorzugte erfindungsgemäße Verbindungen zeigen einen IC50-Wert von unter 1 µM, besonders bevorzugt von unter 100 nM, ganz besonders bevorzugt von unter 10 nM und ganz ganz besonders bevorzugt von unter 1 nM.

### Milchaufnahme weiblicher NMRI Mäuse

Die Prüfung der anorektischen Wirkung erfolgt an weiblichen NMRI Mäusen. Nach 24-stündigem Futterentzug wird oder die Prüfsubstanz intraperitoneal oder bevorzugt oral über eine Schlundsonde verabreicht. In Einzelhaltung und bei freiem Zugang zu Trinkwasser wird den Tieren 30 Minuten nach Präparatgabe Kondensmilch angeboten. Der Kondensmilchverbrauch wird halbstündlich 7 Stunden lang bestimmt und das Allgemeinbefinden der Tiere beobachtet. Der gemessene Milchverbrauch wird mit den Vehikel-behandelten Kontrolltieren verglichen.
Das Vehikel hat selbst keinen Einfluss auf die Futteraufnahme. Bevorzugte verträgliche Vehikel für die Applikation sind z. B. Hydroxyethylzellulose (0,5% in Wasser) oder Solutol HS15 (5% in Hydroxyethylzellulose (0,5% in Wasser)).

### Futter- und Wasseraufnahme weiblicher Wistar-Ratten

Alternativ zur Prüfung der anorektischen Wirkung an NMRI Mäusen können auch analog weibliche Wistar-Ratten mit einem Gewicht von ca. 220-250g verwendet werden. Die Tiere werden vor Studienbeginn an die Versuchsumgebung gewöhnt. In einer Ausführungsform haben die Tiere bis zum Beginn des Versuchs freien Zugang zu Futter und Wasser. In einer anderen Ausführungsform wird den Tieren der Zugang zu Futter 24 Stunden vor der Applikation entzogen. Für die Untersuchung der Prüfsubstanz werden die Tiere bei freiem Zugang zu Futter und Wasser einzeln aufgestallt. Futter- und Wasseraufnahme werden mit einem computergestützten System (TSE Drinking & Feeding Monitor) kontinuierlich alle 30 Minuten über einen Zeitraum von 22 Stunden erfasst. Der gemessene Futter- und Wasserverbrauch wird mit den Vehikel-behandelten Kontrolltieren verglichen.

### Körpergewichtsentwicklung an diät-induziert adipösen und standard-gefütteren Mäusen

Für diese Untersuchungen werden männliche C57BL6J-Mäuse im Alter von 5 Wochen (Alter des Absetzens) zum einen an eine Standard Haltungsdiät bzw. eine fett- und damit energiereiche Diät gewöhnt. Nach 12 Wochen haben die normalgefütterten, schlanken Mäuse typischerweise ein Körpergewicht von etwa 25 g, die fettgefütterten eines von etwa 35 g erreicht. Die Tiere werden einzeln aufgestallt und individuell die Futter- und Wasseraufnahme bestimmt. Während des Versuches besteht freier Zugang zu Futter und Wasser.
Die Prüfsubstanzen werden in einem Vehikel oral verabreicht und immer im Vergleich zur parallel mitgeführten Vehikel-Kontrolle geprüft. Das Vehikel hat selbst keinen Einfluss auf die Futteraufnahme, üblicherweise handelt es sich um Hydroxyethylzellulose (0,5% in Wasser) oder Solutol HS15 (5% in Hydroxyethylzellulose (0,5% in Wasser)). Zu jeder Gruppe von diät induziert adipösen Mäusen wird eine korrespondierende Gruppe von schlanken Mäusen geführt.
Futter- und Wasseraufnahme werden in der ersten Woche täglich, danach einmal pro Wochen durch Rückwaage des angebotenen Futters bzw. Wassers bestimmt. Das Körpergewicht wird täglich gemessen.
Vor und am Ende der Behandlung werden Blutproben gewonnen, um Serumparameter zu bestimmen, die Hinweise auf Veränderungen des Intermediärstoffwechsels liefern. Weiterhin kann am lebenden Tier der Fettanteil des Körpers mittels einer Impedanz-Messung bestimmt (TOBEC-Methode) werden.

Für die beabsichtigten Wirkungen auf Parameter wie Nahrungsaufnahme und Körpergewichtsentwicklung ist es wünschenswert, dass ein Antagonist des MCH1R eine ausreichende Hirngängigkeit (z. B. bestimmt als Verhältnis der zu einem Zeitpunkt erreichten Verbindungsspiegel im Hirngewebe und im Blutserum) aufweist (siehe dazu z. B. J. Pharmacol. Exp. Thera. 2008, 324, 206-213). Bevorzugte erfindungsgemässe Verbindungen haben ein Verhältnis von Hirn- zu Serumspiegeln von mindestens 0,3. Weiter bevorzugte Verbindungen weisen ein Verhältnis von mindestens 0,6 auf. Besonders bevorzugte Verbindungen zeigen ein Verhältnis von mindestens 1,0.

### Mikrokern-Test (in vitro)

Ziel des Mikrokern-Tests (in vitro) ist es, zu prüfen, ob eine Testverbindung das Potential besitzt, die Bildung von Mikrokernen (kleine Membran gebundene DNA-Fragmente) in verschiedenen Zelllinien oder primären Kulturen, mit oder ohne metabolische Aktivierung durch S9-Leberhomogenat, hervorzurufen. Das Testsystem erlaubt die Unterscheidung zwischen dem clastogenen und aneugenen Potential einer Testverbindung durch ein immunochemisches Labelling der Kinetochore oder durch Anfärben der DNA-Fragmente nach dem FISH-(fluorescence in situ hybridization) Verfahren.
Kurzbeschreibung: Die Zellen werden auf einer 96-well Mikrotiterplatte mit der Testverbindung behandelt. Die Behandlungszeit beträgt typischerweise 3 Stunden mit metabolischer Aktivierung beziehungsweise 24 Stunden ohne metabolische Aktivierung. Vierundzwanzig Stunden nach dem Ende der Behandlung werden die Zellen isoliert, fixiert und angefärbt. Die Zytotoxizität der Testverbindung wird beurteilt nach dem relativen Zellwachstum ausgedrückt als Prozentsatz des Wachstums oder unter Berücksichtigung der Verdopplungszeit als Population Doubling im Vergleich zur Negativkontrolle. Die höchste Testkonzentration sollte nicht weniger als 30% überlebende Zellen zeigen, oder sollte die Konzentration sein, bei der ein Niederschlag der Testverbindung beobachtet wird. Doppelbestimmungen sollten bei jeder Testkonzentration durchgeführt werden. Eine genaue ausführliche Versuchsbeschreibung findet sich bei Kirsch-Volders et al. (Mutation Res. 2003, 540, 153-163).
Auswertung: Der strukturelle bzw. numerische Chromosomenschaden wird angegeben als die Erhöhung der Zahl der Zellen mit Mikrokernen in einem Ensemble von 1000 Zellen bei drei analysierbaren Testkonzentrationen. Der Test wird in folgenden Fällen als positiv betrachtet:
a) der Anstieg der Zahl der Zellen mit Mikrokernen ist signifikant im Vergleich zur Negativkontrolle (Lösungsmittel bzw. unbehandelt), oder
b) die Zahl der Mikrokerne ist in einem biologisch relevanten Rahmen konzentrationsabhängig erhöht im Vergleich zur Negativkontrolle.

Eine Positivkontrolle muss einen deutlichen statistisch signifikanten Effekt zeigen im Vergleich zur Negativkontrolle.
Bevorzugte Verbindungen der Erfindung sind negativ im Mikrokerntest.

### AMES II-Test

Ziel des AMES II-Tests ist es zu prüfen, ob eine Testverbindung mutagenes Potential besitzt. Kurzbeschreibung: Auf einer 384-well Mikrotiterplatte wird ein gemischter Bakterienstamm (Mixed strains, 6 verschiedene Salmonella typhimurium Stämme mit jeweils einer Missence-Punktmutation im Histidinoperon) sowie der Salmonella typhimurium Stamm TA98 zum Nachweis von frameshift-Mutationen mit verschiedenen Konzentrationen der Testsubstanz mit oder ohne metabolische Aktivierung durch den Zusatz von S9-Leberhomogenat behandelt (genaue Versuchsbeschreibungen finden sich in der Literatur: P. Gee, D.M. Maron, B.N. Ames; Proc. Natl. Acad. Sci. USA 1994, 91, 11606 bzw. Flückiger-Isler et al.; Mutation Res. 2004, 558, 181 und zit. Lit.).
Mutagene Testverbindungen verursachen Rückmutationen und stellen so die Funktionalität der endogenen Histidinbiosynthese wieder her. Dadurch sind mutierte Bakterien in der Lage sich zu teilen und zu Bakterienkolonien auszuwachsen.
Auswertung: Kommt es zu einem verstärkten Bakterienwachstum bedingt durch Mutationen der Bakterien, so werden Enzyme im Wachstumsmedium verdaut. Dadurch sinkt der pH-Wert im Medium und es erfolgt ein Farbumschlag des zugesetzten Indikators (Bromkresolpurpur) von lila nach gelb. Der Test wird als positiv betrachtet wenn die Anzahl der Wells pro Konzentration, bei denen ein Farbumschlag beobachtet wird, signifikant gegenüber dem Kontrollwert ansteigt.
Bevorzugte Verbindungen der Erfindung sind im AMES II-Test negativ.

### Zytotoxizitätstests

### a) LDH-Freisetzung

Ziel des Tests auf LDH (Laktat-Dehydrogenase)- Freisetzung ist es zu prüfen, ob eine Verbindung die Integrität der Zellwand schädigt und so den Zelltod verursachen kann. Kurzbeschreibung: Gemessen wird auf colorimetrischem Weg die LDH-Aktivität, die durch Zellschädigung vom Zytosol in den Zellüberstand gelangt. Die Zellen werden mit der Testverbindung behandelt. Fünfzig Mikroliter des Kulturüberstandes werden abgenommen und mit der Reaktionslösung (LDH-Kit, Roche, Mannheim) nach den Angaben des Herstellers zusammengebracht. LDH katalysiert die Umwandlung von Laktat in Pyruvat. Dabei wird NAD+ zu NADH/H+ reduziert. Letzteres wiederum reduziert unter dem Einfluss der zugesetzten Diaphorase ein ebenfalls zugesetztes gelbes Tetrazoliumsalz zu rotem Formazan. Auswertung: Die Quantifizierung des Formazans erfolgt durch Messung der Absorption bei 492 nM (z. B. mit TECAN SPECTRAFluor Plus).
Bevorzugte Verbindungen der Erfindung zeigen keine signifikante Erhöhung der LDH-Aktivität bei Konzentrationen unter 10 µM. Besonders bevorzugte Verbindungen zeigen keine Erhöhung unterhalb einer Konzentration von 50 µM. Noch weiter bevorzugte Verbindungen zeigen keine Erhöhung unterhalb einer Konzentration von 250 µM.

### b) Intrazellulärer ATP-Gehalt

Ziel des Tests ist es, den intrazellulären Gesamt-ATP-Gehalt, der ein Maß für den Energiespiegel und damit die Vitalität einer Zelle ist, zu bestimmen.
Kurzbeschreibung: 100 µL Zellkulturmedium werden in einem Well einer Mikrotiterplatte mit 100 µL des CellTiter-Glo Reagenzes versetzt (folgend den Angaben des Herstellers: Promega Technical Bulletin No. 228, CellTiter-Glo Luminesent Cell Viability Assay). Die Kulturen werden für 2 Minuten bei Raumtemperatur geschüttelt und dann noch 10 Minuten inkubiert bis sich das Lumineszenzsignal stabilisiert hat.
Auswertung: Die Lumineszenz wird über eine Sekunde integrierend aufgenommen (z. B. mit TECAN SPECTRAFluor Plus).
Bevorzugte Verbindungen der Erfindung zeigen keine signifikante Absenkung der ATP-Spiegel bei Konzentrationen unter 10 µM. Besonders bevorzugte Verbindungen zeigen keine Absenkung unterhalb einer Konzentration von 50 µM. Noch weiter bevorzugte Verbindungen zeigen keine Absenkung unterhalb einer Konzentration von 250 µM.

### c) Neutral-Rot-Aufnahme

Ziel des Tests ist es, die Aufnahme von Neutral-Rot (NR) in die Lysosomen / Endosomen und Vakuolen lebender Zellen, die ein quantitatives Maß für die Zahl und Vitalität der Zellen ist, zu messen.
Kurzbeschreibung: Die Zellen werden mit 150 µL einer vorgewärmten Phosphatpufferlösung (PBS) gewaschen und mit 100 µL des NR Mediums für 3 Stunden in einer angefeuchteten Atmosphäre mit 7,5% Kohlendioxid bei 37°C inkubiert. Nach der Inkubation wird das NR-Medium entfernt und die Zellen mit 150 µL PBS gewaschen. Nach dem Entfernen der PBS werden genau 150 µL einer Ethanol / Eisessig-Lösung zugesetzt. Nach 10 Minuten Schütteln ist der Farbstoff aus den Zellen zu einer homogenen Farbstoff-Lösung extrahiert. Eine genaue Beschreibung des Tests findet sich in der Literatur (E. Borenfreund, J.A. Puerner, Toxicol. Lett. 1985, 24(2-3), 119-124).
Auswertung: Die Absorption der Farbstoff-Lösung wird bei 540 nM mittels eines Mikrotiterplattenlesegeräts als Differenz zur Absorption der Ethanol / Eisessig-Lösung bestimmt.

### HERG-Kanal-Blockade

Ziel des Tests ist, den Konzentrationsbereich zu bestimmen, in dem die Testverbindung den kardialen hERG-Kanal blockiert. Eine Blockade des hERG-Kanals, der verantwortlich ist für den Ikr-Strom im menschlichen Herzen, ist mit potentiell tödlichen Arrythmien assoziert.
Die den hERG-Kanal codierende cDNA wurde zur Expression in den pCDNA3-Vektor (Invitrogen) geklont. Eizellen des chinesischen Hamsters (CHO, American Type Culture Collection, Rockville, MD) wurden mittels Lipofectamine (GIBCO/BRL, Grand Island, NY) mit der hERG cDNA transfiziert und mittels G418 (GIBCO/BRL, Grand Island, NY; 500 µg/mL) selektioniert. CHO-Zellen mit stabiler Expression des hERG-Kanals wurden in einer Atmosphäre von 95% Luft / 5% Kohlendioxid auf einem HAM F-12 Medium kultiviert, das angereichert war mit 10% nativem Rinderserum, 1X Penicilin/Streptomycin und 500 µg/mL G418.
Die für den Patch-Clamp-Versuch ausgesuchten Zellen werden 18-24 Stunden vor dem Versuch auf einen Plastik-Träger ausgesät. HERG-Kanal-Ströme werden bei Raumtemperatur nach der Ganzzellen-Variante der Patch-Clamp-Technik mit einem Axopatch 200B Verstärker (Axon Instruments, Foster City, CA) aufgezeichnet. Die Elektroden (3-6 Megaohm Widerstand) werden hergestellt aus TW150F Glaskapillaren (World Precision Instruments, Sarasota, FL) and mit der Pipettenlösung (120 mM Kaliumaspartat, 20 mM KCl, 4 mM Na2ATP, 5 mM HEPES, 1 mM MgCl2; auf pH 7,2 gestellt mit KOH) gefüllt. Die hERG-Kanal-Ströme werden ausgelöst durch einen positiven Spannungimpuls (20 mV) gefolgt von einem negativen Impuls (-40 mV) und werden aufgezeichnet für die spätere Analyse. Sobald der hERG-Kanal-Strom der Zelle, die mit der Kontrolllösung (130 mM NaCl, 5 mM KCl, 2,8 mM NaOAc, 1 mM MgCl2, 10 mM HEPES; 10 mM Glukose, 1 mM CaCl2; auf pH 7,4 gestellt mit NaOH) gespült wird, stabil ist, wird die Zelle mit der Testverbindung, gelöst in obiger Kontrolllösung (durch Verdünnen einer 10 oder 100 mM DMSO-Lösung der Testverbindung, so dass der DMSO-Anteil nicht mehr als 0,1% beträgt) durchspült. Der Strom wird ständig verfolgt bis keine Änderungen mehr auftreten. Der gleiche Vorgang wird mit steigenden Konzentrationen der Testverbindung wiederholt. Für jede Konzentration und für jede Zelle wird die Maximalamplitude des hERG-Stroms in picoAmpere (pA) gemessen. Die Maximalamplitude in pA für jede Konzentration der Testverbindung wird verglichen mit der der reinen Kontrolllösung in derselben Zelle und berechnet als % des Kontrollwertes. Auswertung: Die Testverbindung wird bei verschiedenen Konzentrationen in 3-5 CHO-Zellen, die den hERG-Kanal exprimieren, getestet. Der IC50-Wert ergibt sich durch Anwendung der nicht-linearen Regression mittels kleinster Fehlerquadrate (GraphPAD Software, San Diego, CA).

### Generelle Selektivität

Um das Risiko unerwünschter Nebenwirkungen zu minimieren, ist es wünschenswert die unselektive Beeinflussung von biologisch wichtigen Funktionseinheiten (z. B. Rezeptoren, Ionenkanäle und Enzyme; für Auflistungen siehe z. B. Whitebread, S. et al.; Drug Discovery Today 2005, 10, 1421-33 und Rolland, C. et al.; J. Med. Chem. 2005, 48, 6563-6574) durch einen pharmazeutischen Wirkstoff möglichst gering zu halten. Generelle Selektivitätstests in einer Vielzahl von in vitro Testsystemen können durch verschiedene spezialisierte Anbieter (z. B. Cerep, Panlabs) durchgeführt werden.
Die erfindungsgemäßen Verbindungen der Formel I weisen als selektive MCH1R-Antagonisten Selektivitätsfaktoren von mindestens 30, bevorzugt von 100, stärker bevorzugt von 300 und noch stärker bevorzugt von 1000 gegenüber der Affinität zu anderen Proteinen auf. Beispiele solcher Proteine sind Serotonin-Rezeptor-Subtypen (z. B. der 5-HT2a Rezeptor), Muscarin-Rezeptor-Subtypen (z. B. der M1-Rezeptor), adrenerge Rezeptorsubtypen (z. B. AR alphala), Natrium- und Calciumkanäle (z. B. der Calciumkanal vom L-Typ).

### Löslichkeiten in wässrigen Systemen

Ausreichende Löslichkeit einer Substanz in wässrigen Lösungsmittelsystemen ist eine wichtige Vorraussetzung für eine (reproduzierbare) pharmakologische Wirkung. Löslichkeiten in wässrigen Systemen können nach verschiedenen Verfahren bestimmt werden. Geeignet sind z. B. Fällungsverfahren aus Lösungen ("kinetische Löslichkeit") und Verfahren, die die Auflösung einer festen Probe bis zur Gleichgewichtseinstellung untersuchen ("thermodynamische Löslichkeit").

### a) Kinetische Löslichkeit

Auf einer 96-well Mikrotiterplatte wird eine DMSO-Lösung der Testverbindung (2,5 mM; 0,5 µL) zu 200 µL einer wässrigen Testlösung (z. B. Phosphate buffered Saline, 10x, 1M, Sigma eingestellt auf 10 mM, pH 7,4) pipettiert und die Trübung bei der so erhaltenen theoretischen Konzentration der Testverbindung von 6,25 µM mittels eines Nephelometers (z. B. Nephelostar Galaxy, BMG Labtech) gemessen. Danach wird die Konzentration der Testverbindung in der wässrigen Testlösung durch Zugabe von weiterer DMSO-Lösung (2,5 mM; 0,5 µL) auf theoretisch 12,5 µM erhöht und die Trübungsmessung erneut durchgeführt. Weitere Zugaben von DMSO-Lösungen (1 µL, 2,5 mM; 0,5 µL, 10 mM; dann 9-mal 1 µL, 10 mM ergebend theoretische Konzentrationen von 25 µM, 50 µM, 100 µM, 150 µM, 200 µM, 250 µM, 300 µM, 350 µM, 400 µM, 450 µM und 500 µM) mit zwischenzeitlicher Trübungsmessung komplettieren den Messprozess.
Auswertung: Die Trübungswerte des Nephelometers werden gegen die theoretische Konzentration der Testverbindung in der wässrigen Testlösung aufgetragen. Sobald bei einer theoretischen Konzentration eine signifikante Trübung detektiert wird (z. B. 5-fach über dem Kontrollwert der wässrigen Testlösung), wird der darunter liegende Konzentrationswert als Löslichkeitsgrenze der Testverbindung in der Testlösung angegeben. Als maximal möglicher Messbereich ergeben sich damit die Werte <6,25 µM, 6,25 - 500 µM und >500 µM. Bevorzugte erfindungsgemäße Verbindungen zeigen eine kinetische Löslichkeit im Phosphatpuffer (pH 7,4) von mindestens 12,5 µM; bevorzugter von mindestens 50 µM und noch stärker bevorzugt von mindestens 250 µM.

### b) Thermodynamische Löslichkeit

Mittels HPLC-UV-Messung einer Verdünnungsreihe der Testverbindung in DMSO (500 µM, 100 µM, 50 µM, 10 µM und 1 µM) wird die integrierte UV-Absorption in einer Kalibiergeraden linear mit der Konzentration korreliert. Die Testverbindung (500 µg) wird zusammen mit der wässrigen Testlösung (250 µL) in einem geschlossenen Gefäß (Fassungsvolumen: 1,5 mL) für 16 Stunden geschüttelt (Eppendorf Thermoschüttler, 1400 rpm, 25°C, Abdeckung als Lichtschutz). Anschließend wird die Probe bei maximaler Drehzahl zentrifugiert und der Überstand abschließend noch filtriert. Eine Probe des filtrierten Überstandes wird direkt mittels HPLC-UV-Messung (siehe oben) analysiert. Eine weitere Probe wird nach Verdünnen (1 Volumenteil Überstand, 39 Volumenteile Testlösung) analysiert. Auswertung: Anhand der erstellten Kalibiergeraden wird aus den erhaltenen integrierten UV-Absorptionen der Überstandsproben die Konzentration der Testverbindung im unverdünnten Überstand berechnet und als Löslichkeit der Testverbindung in der jeweiligen wässrigen Testlösung angegeben.
Beispiele für wässrige Testlösungen sind entsalztes Wasser oder wässrige Phosphatpuffer mit verschiedenen pH-Werten (z. B. pH 1,2; pH 4,0; pH 6,8; pH 7,4; pH 9,0), die nach Standardverfahren aus der kommerziellen Lösung (Phosphate buffered saline, 10x, Sigma) hergestellt werden können durch Verdünnen bzw. Einstellen mit Phosphorsäure oder Natronlauge.
Bevorzugte erfindungsgemäße Verbindungen zeigen eine Löslichkeit im Phosphatpuffer (pH 7,4) von mindestens 12,5 µM; bevorzugter von mindestens 50 µM und noch stärker bevorzugt von mindestens 250 µM.

### Permeabilität

Der Test auf Permeabilität wird in CACO-2/TC7 Zellen durchgeführt, die für 21 Tage auf Becton Dickinson-Filtern (24-well, nicht beschichtet) kultiviert wurden (DMEM / Glutamax I / Gibco mit hohem Glukoseanteil, HEPES 25 mM, 1% NEAA, 10% FBS, 40 µg/mL Gentamycin; 37°C Umgebungstemperatur; 95% Luftfeuchtigkeit und 10% CO2-Gehalt). Die Permeabilität wird bei einer Konzentration der Testverbindung von 20 µM (1% DMSO in HBSS) mit einem pH-Gradienten (apical: pH 6,5 und 0,5% BSA; basolateral: pH 7.4 und 5% BSA) geprüft. Die Analyse erfolgt mittels LCMS/MS. Weitere Beschreibungen des Testsystems und Referenzen zur experimentellen Durchführung finden sich bei Balimane, P.V.; Drug Discovery Today 2005, 10(5), 335-343.

### Inhibition von CYP-Enzymen

Die Inhibition von CYP-Enzymen wird an rekombinanten Enzymen (erhalten von Becton Dickinson) und fluoreszierenden Substraten (BD/Gentest) nach den Empfehlungen des Herstellers bestimmt (siehe Website http://www.bdbiosciences.com). Weitere Beschreibungen des Testsystems und Referenzen zur experimentellen Durchführung finden sich bei Zlokarnik, G.; Drug Discovery Today 2005, 10(21), 1443-1450.

### Metabolische Stabilität

Die metabolische Stabilität wird bestimmt durch Inkubation der Testverbindung (5 µM) bei 37°C mit microsomalen Leberfraktionen (1 mg/mL Protein mit 0,1 % w/v BSA; 1 mM NADPH, 0,5% DMSO). Die Analyse bei 0 und 20 Minuten Inkubationszeit erfolgt mittels LCMS/MS. Weitere Beschreibungen des Testsystems und Referenzen zur experimentellen Durchführung finden sich bei Plant, N.; Drug Discovery Today 2004, 9(7), 328-336 und Lau, Y.Y. et al.; Pharmaceutical Res. 2002, 19(11), 1606-1610.

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Zum Beispiel kann eine Aminosäure der Struktur Z1 zunächst selektiv geschützt werden (z. B. nach Methode D mit Boc20). Die folgende Reaktion mit einem Amin (HNR1R2) kann vorteilhaft unter Verwendung eines allgemein bekannten Kupplungsreagenzes (z. B. nach Methode A-1 mit HATU oder nach Methode A-2 mit EDC/HOBt) durchgeführt werden. Entfernung der Schutzgruppe (z. B. nach Methode C mit Salzsäure) und anschließende Reduktion (z. B. nach Methode B mit Lithiumaluminiumhydrid) ergibt ein Amin der Struktur Z2 (wobei R8 = H). Entfernt man eine Carbamatschutzgruppe vor der Reduktion nicht, wird Z2 mit R8 = Methyl erhalten. In einem letzten Schritt können die erfindungsgemäßen Verbindungen der Formel Ia durch Reaktion des Amins Z2 mit einer Säure der Struktur B-L3-A-L2-CO2H (z. B. nach Methode A-1 oder A-2) erhalten werden (Schema 1).

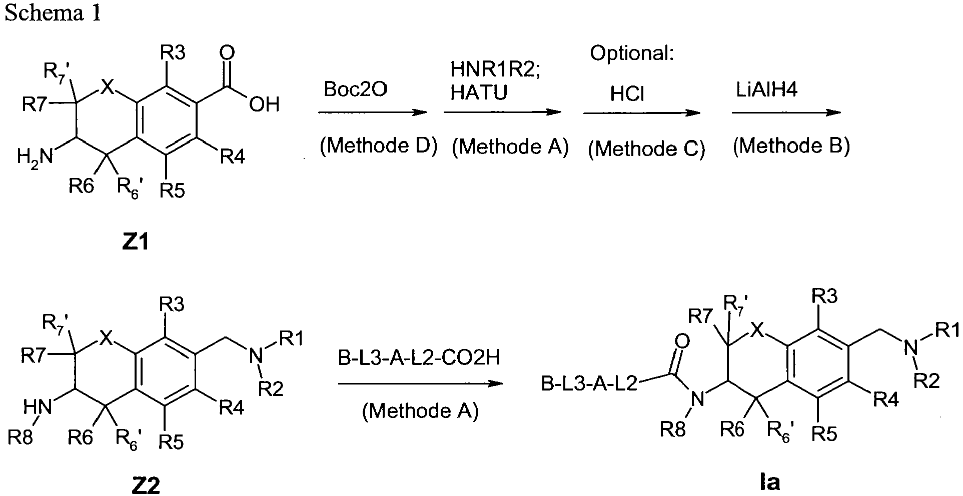

Weitere Verbindungen des Typs Ia können durch Umsetzung der Intermediate Z2 mit Carbonsäuren der Struktur HO-A-L2-COOH (z. B. nach Methode A-2) und anschließende Alkylierung mit entsprechenden Alkylierungsmitteln (z. B. nach Methode F mit Alkylbromiden, Alkyliodiden oder Alkylsulfonsäureestern) erhalten werden.
Alternativ können Verbindungen der Formel I aus den Ketonen Z3 erhalten werden, welche kommerziell erhältlich sind, oder nach bekannten Methoden (siehe z. B. Synthesis 2004, 121; J. Org. Chem. 1995, 60, 4324) hergestellt werden können. Säurekatalysierte Kondensation der Ketone Z3 mit Amiden (B-L3-A-L2-CONH2) und anschließende (optional asymmetrische) katalytische Hydrierung der erhaltenen Enamide unter bekannten Bedingungen (siehe z. B. Adv. Synth. Catal. 2003, 345, 230; Tetrahedron: Asymmetry 1999, 10, 3467; J. Org. Chem. 1995, 60, 4324) ergibt die Arylbromide Z4. Diese können nach Literaturvorschriften in die Arylcarbonylverbindungen Z5 umgewandelt werden (siehe z. B. J. Am. Chem. Soc. 2000, 122, 6935; J. Med. Chem. 2005, 48, 1948; Angew. Chem. Int. Ed. 2006, 45, 154). Abschließende reduktive Aminierung führt zu den Verbindungen Ib (Schema 2).

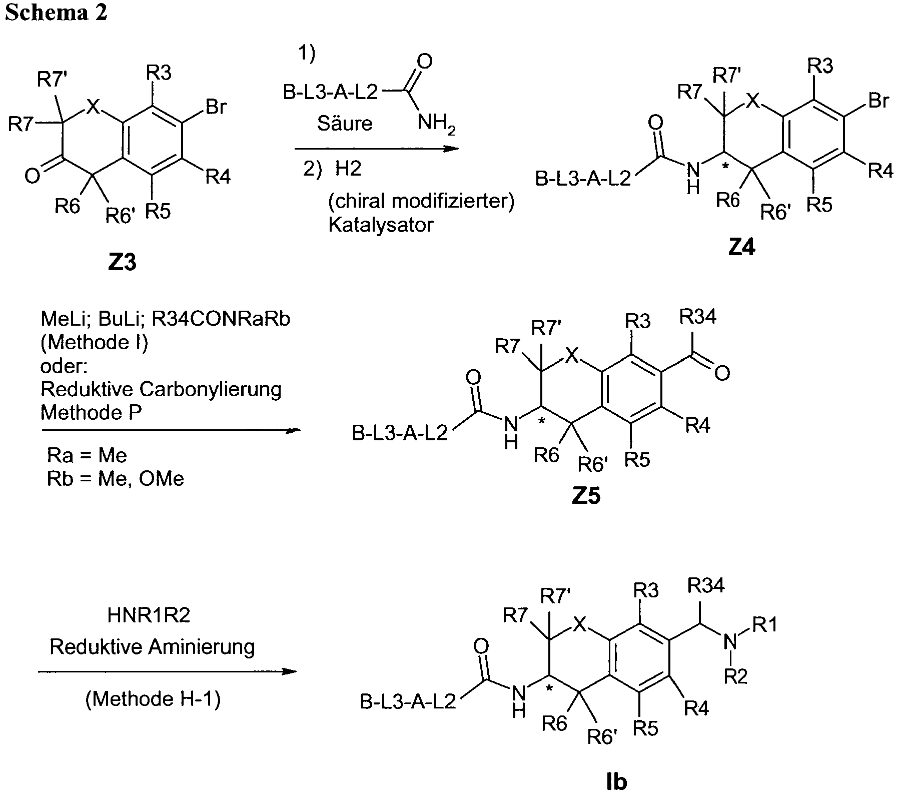

Der Aufbau stereochemisch definierter Verbindungen des Typs Ib* gelingt z. B. durch Kondensation der Intermediate Z5' mit chiralen Sulfinylamiden (z. B. nach Methode M), Addition von Grignard-Reagenzien (z. B. nach Methode L), Hydrolyse (z. B. nach Methode K) und optionaler reduktiver Alkylierung z. B. nach Methode H-2 (Schema 2-1).

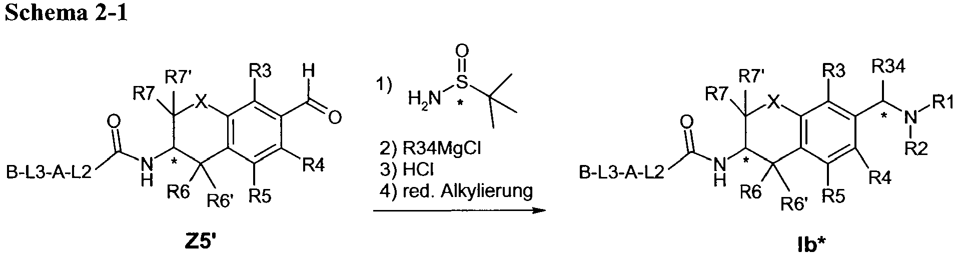

Weitere Intermediate des Typs Z4 können durch nachträgliche Abwandlung von Substituenten erhalten werden. So können z. B. Methoxygruppen (B-L3 = MeO) durch Reagenzien wie Bromwasserstoff oder Bortribromid (z. B. nach Methode N) gespalten und die erhaltenen aromatischen Hydroxyverbindungen mit entsprechenden Alkylierungsmitteln (z. B. nach Methode F mit Alkylbromiden, Alkyliodiden oder Alkylsulfonsäureestern) umgesetzt werden. Die Intermediate Z4 können auch zur Synthese anderer Verbindungen der Formel I verwendet werden. Dazu können z. B. die durch sequentielle Behandlung von Z4 mit MeLi und dann n-BuLi erhaltenen Dianionen mit Ketonen (R34COR35) umgesetzt werden. Die erhaltenen tertiären Alkohole können unter den Bedingungen der Ritter-Reaktion (z. B. TMSCN, H2SO4/HOAc) zu Amiden umgewandelt werden, die dann, nach Hydrolyse und optionaler reduktiver Aminierung, Verbindungen der Struktur Ib-1 ergeben (Schema 2-2).
Alternativ können die Intermediate Z4 auch durch Übergangsmetallkomplexe (z. B. derer von Pd und Ni) katalysiert mit Pyridyl-verbindungen (z. B. Pyridyl-trialkylzinnverbindungen, Pyridyl-boronsäure(derivaten) oder Pyridin-N-oxiden) zur Reaktion gebracht werden. Anschließende Hydrierung mit geeigneten Katalysatoren (z. B. PtO2 in HOAc; Methode J-1) und optionale reduktive Alkylierung ergibt die Strukturen Ib-2 (Schema 2-2).
In einer anderen Variante werden die Intermediate Z4 Palladium-katalysiert mit Allylmetallverbindungen (z. B. Allyl-tributyl-zinn) umgesetzt, anschließend die Doppelbindung oxidativ gespalten (z. B. mit OsO4/NaIO4),und die so erhaltenen Aldehyde im Sinne einer reduktiven Aminierung mit Aminen HNR1R2 umgesetzt (Schema 2-2).

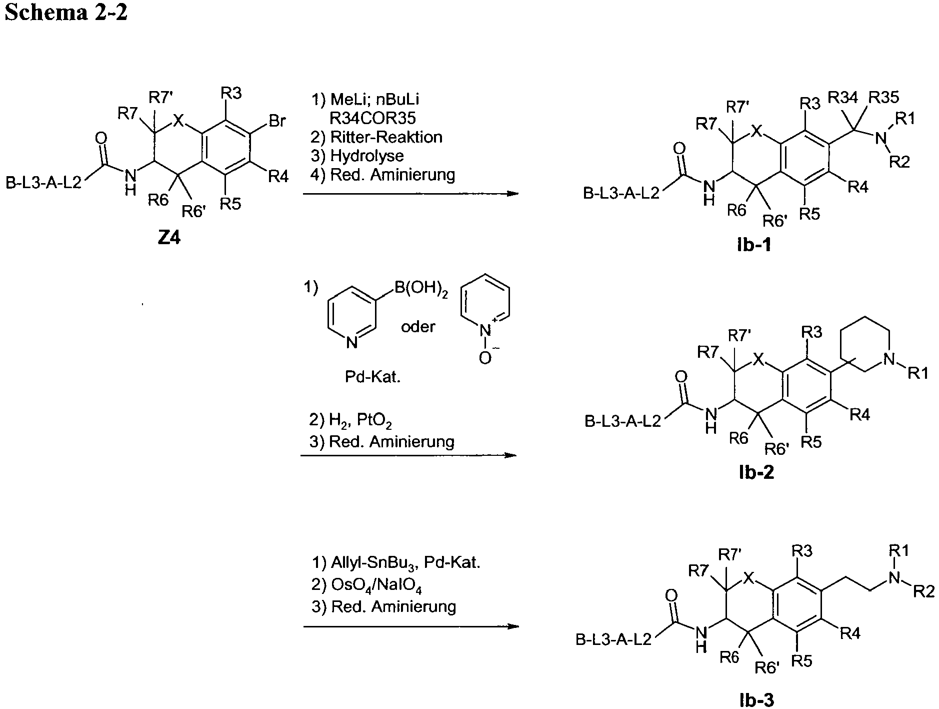

Durch Alkylierung (z. B. mit NaH, MeI) an der Amidfunktion der Intermediate Z4, Z5 und Z5' und weitere Synthese entlang der oben angegebenen Wege, sowie auch durch analoge Alkylierung der Strukturen Ib, Ib-1, Ib-2 und Ib-3 ergeben sich weitere Wege zu Verbindungen der Formel I (Variation des Substituenten R8).
Ein weiteres Herstellungsverfahren für wieder andere Verbindungen der Formel I besteht darin, Dichloride des Typs Z6 oder Isochromenone des Typs Z7 mit Aminen Z8 nach im Prinzip bekannten Verfahren umzusetzen (Schema 3). Die benötigten Dichloride Z6 können aus ortho-Methylbenzoesäuren durch doppelte Metallierung z. B. mit Lithium-diisopropylamid (LDA), Abfangen des Dianions mit Formaldehyd (z. B. in Form von Paraformaldehyd) und abschließende Dichlorierung gewonnen werden. Die Amine Z8 können z. B. nach Schema 1 (Z2 mit R8 = H) oder nach Schema 2 durch Hydrolyse der Strukturen Ib erhalten werden.

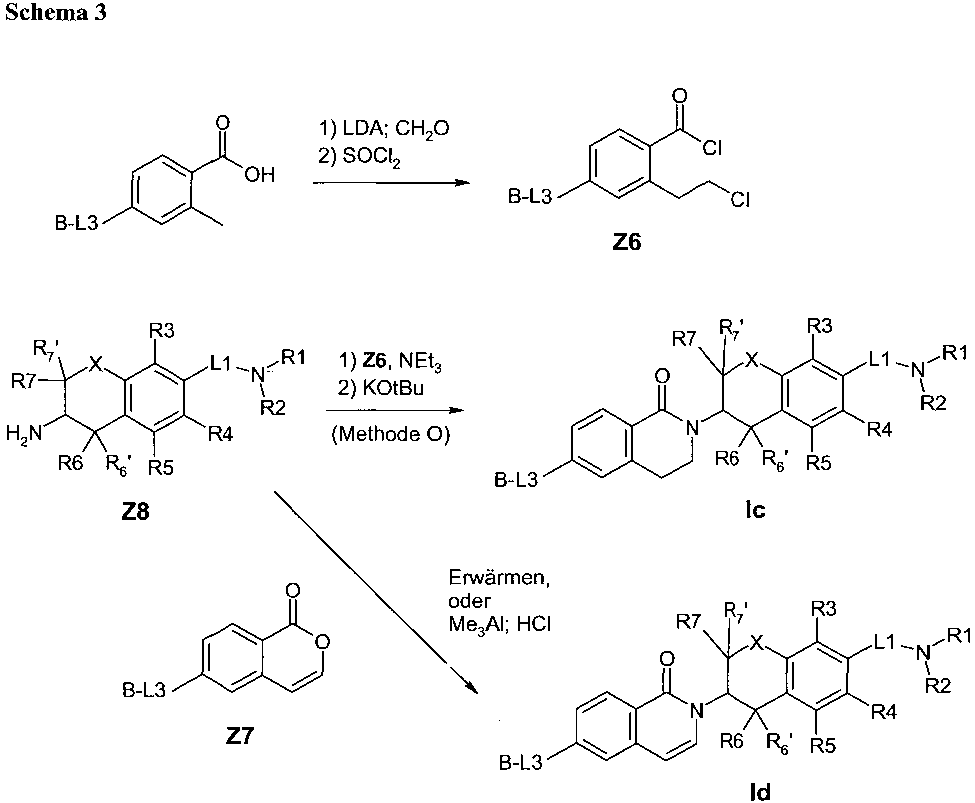

Alternativ kann zur Synthese von (Dihydro)isochinolinonen gemäß der Formel I auch die Amidbindung der Intermediate Z4 unter entsprechenden Bedingungen (z. B. mit HBr / Methansulfonsäure) gespalten werden. Die so erhaltenen Amine können mit den Dichloriden Z6 (oder den Isochromenonen Z7) umgesetzt werden. Die so hergestellten Bromide Z9 können dann analog den Intermediaten Z4 (bevorzugt über dort angegebene übergangsmetallkatalysierte Reaktionen) weiter zu erfindungsgemässen Verbindungen umgesetzt werden. Z. B. können die Bromide Z9 reduktiv carbonyliert werden und die so erhaltenen Aldehyde im Sinne einer reduktiven Aminierung zu Verbindungen des Typs Ie umgesetzt werden (Schema 3-1).

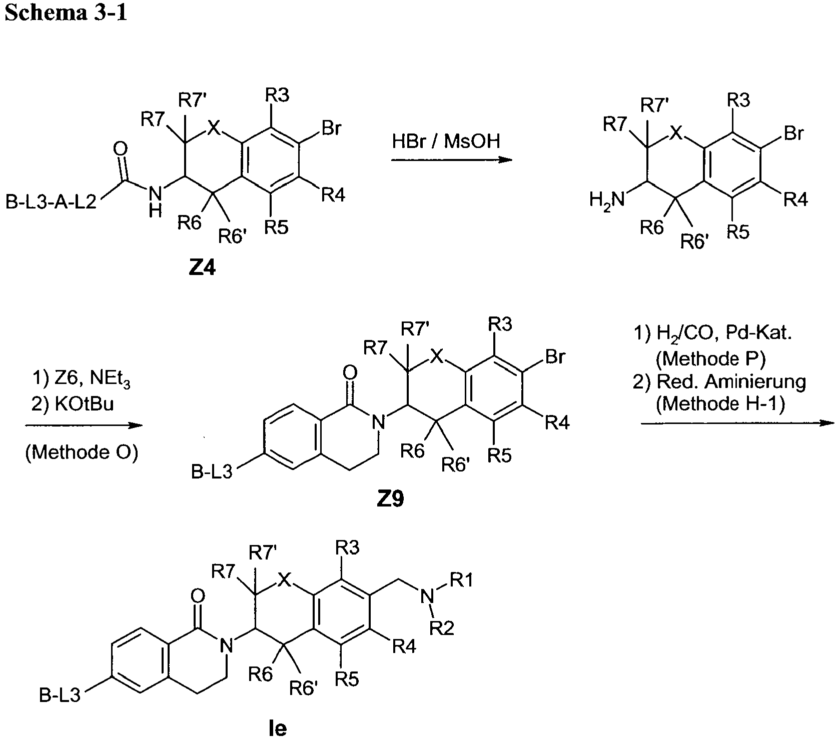

Beschreibungen der verwendeten allgemeinen Methoden finden sich exemplarisch an folgenden Stellen beschrieben:
Methode A-1, B, C, D, E, F, G im Beispiel 1;
Methode H, I im Beispiel 2;
Methode J-1 im Beispiel 4;
Methode K, L, M-1 im Beispiel 6;
Methode H-2 im Beispiel 8-1;
Methode A-2 im Beispiel 9-1;
Methode N im Beispiel 13;
Methode O im Beispiel 14;
Methode P im Beispiel 15.

### Allgemeine Erläuterungen

### a) Zeichenweise der Strukturformeln

In den Strukturformeln der gegebenen Beispiele werden zur Übersichtlichkeit bevorzugt nur Nicht-Wasserstoffatome dargestellt.

### b) Salzformen

Viele der erfindungsgemäßen Verbindungen sind Basen und können mit entsprechend starken Säuren Salze bilden. Insbesondere können die Verbindungen nach HPLC-chromatographischer Reinigung unter Verwendung eines Trifluoressigsäure enthaltenden Laufmittels als Hydrotrifluoracetate vorliegen. Diese können durch einfaches Behandeln einer Lösung der Salze z. B. mit Natriumcarbonatlösung in die gezeigten freien Basen überführt werden.

### c) Einheiten der Charakterisierungsdaten

Die Einheit der angegebenen Molekulargewichte ist "g/mol". Beobachtete Peaks im Massenspektrum sind angegeben als ganzzahliger Quotient der molaren Molekülionmasse und der Ladung des Molekülions (m/z).

### Beispiel 1

### N-((S)-6-Pyrrolidin-1-ylmethyl-1,2,3,4-tetrahydro-naphthalen-2-yl)-4-[(S)-1-(tetrahydro-furan-2-yl)methoxy]-benzamide

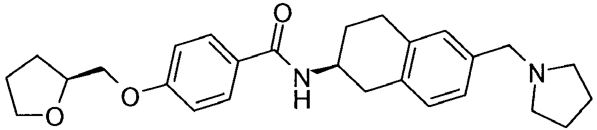

### Methode A-1

Zu einer Lösung von 4-[(S)-1-(TETRAHYDRO-FURAN-2-YL)METHOXY]-BENZOIC ACID (3,38 g) in NMP (30 mL) wurden HATU (O-(7-AZABENZOTRIAZOL-1-YL)-N,N,N',N'-TETRAMETHYLURONIUM-HEXAFLUOROPHOSPHATE; 5,78 g)) und dann Triethylamin (2,12 mL) gegeben. Es wurde eine Lösung von (S)-6-Pyrrolidin-1-ylmethyl-1,2,3,4-tetrahydro-naphthalen-2-ylamine (3,5 g) in NMP (20 mL) zugetropft. Nach 12 Stunden wurde die Reaktionsmischung mit Ethylacetat verdünnt, mit gesättigter Natriumcarbonatlösung gewaschen und eingeengt. Der Rückstand wurde durch Chromatographie an Kieselgel (Laufmittel DCM / MeOH 10:1) gereinigt. Man erhielt so das Produkt mit dem Molekulargewicht 434,58 (C27H34N2O3); MS (ESI): 435 (M+H+).

### (S)-6-Pyrrolidin-1-ylmethyl-1,2, 3,4-tetrahydro-naphthalen-2-ylamine

### Methode B

Eine Lösung von ((S)-6-Amino-5,6,7,8-tetrahydro-naphthalen-2-yl)-pyrrolidin-1-yl-methanone (0,49 g) in THF (5 mL) wurde zu einer Suspension von Lithiumaluminiumhydrid (0,60 g) in THF (10 mL) getropft. Die Mischung wurde für eine Stunde bei RT gerührt. Es wurde Wasser (0,6 mL), gefolgt von Natronlauge (16%ig; 2 mL) und wieder Wasser (2 mL) vorsichtig zugetropft. Der entstandene Niederschlag wurde abfiltriert und das Filtrat eingeengt. Der Rückstand wurde in Salzsäure (1N) aufgenommen und die Lösung mit Diethylether gewaschen. Die wässrige Phase wurde mit konzentrierter Natronlauge basisch gestellt und mit Dichlormethan (DCM) dreimal extrahiert. Die vereinigten organischen Phasen wurden über Magnesiumsulfat getrocknet und eingeengt. Man erhielt so das Produkt mit dem Molekulargewicht 230,36 (C15H22N2); MS (ESI): 231 (M+H+).
Analog wurden (S)-6-(4-Methoxy-piperidin-1-ylmethyl)-1,2,3,4-tetrahydro-naphthalen-2-ylamine und (S)-6-Azepan-1-ylmethyl-1,2,3,4-tetrahydro-naphthalen-2-ylamine dargestellt.

### ((S)-6-Amino-5,6,7,8-tetrahydro-naphthalen-2-yl)-pyrrolidin-1-yl-methanone

### Methode C

Zu einer Lösung von [(S)-6-(Pyrrolidine-1-carbonyl)-1,2,3,4-tetrahydro-naphthalen-2-yl]-carbamic acid tert-butyl ester (0,70 g) in MeOH (5 mL) wurde konzentrierte Salzsäure (5 mL) gegeben. Nach einer Stunde wurde die Mischung mit konzentrierter Natronlauge basisch gestellt und mit DCM dreimal extrahiert. Die vereinigten organischen Phasen wurden über Magnesiumsulfat getrocknet und eingeengt. Man erhielt so das Produkt mit dem Molekulargewicht 244,34 (C15H20N2O); MS (ESI): 245 (M+H+).

### [(S)-6-(Pyrrolidine-1-carbonyl)-1,2,3,4-tetrahydro-naphthalen-2-yl]-carbamic acid tert-butyl ester

Nach Methode A-1 wurde (S)-6-tert-Butoxycarbonylamino-5,6,7,8-tetrahydro-naphthalene-2-carboxylic acid mit Pyrrolidin umgesetzt. Man erhielt so das Produkt mit dem Molekulargewicht 344,46 (C20H28N2O3); MS (ESI): 345 (M+H+).

### (S)-6-tert-Butoxycarbonylamino-5,6,7,8-tetrahydro-naphthalene-2-carboxylic acid

### Methode D

Zu einer Mischung von (S)-6-Amino-5,6,7,8-tetrahydro-naphthalene-2-carboxylic acid (1,0 g), Natronlauge (32%ig; 1,1 g) und MeOH (20 mL) wurde DI-TERT-BUTYL DICARBONATE (1,92 g) gegeben. Die Mischung wurde bei 50 °C für 6 Stunden gerührt und dann Wasser (150 mL) hinzugefügt. Nach der Extraktion mit Diethylether wurde die wässrige Phase leicht angesäuert und dreimal mit DCM extrahiert. Die vereinigten organischen Phasen wurden über Magnesiumsulfat getrocknet und eingeengt. Man erhielt so das Produkt mit dem Molekulargewicht 291,35 (C16H21NO4); MS (ESI): 292 (M+H+).

### 4-[(S)-1-(Tetrahydro-furan-2-yl)methoxy]-benzoic acid

### Methode E

Eine Mischung aus 4-[(S)-1-(Tetrahydro-furan-2-yl)methoxy]-benzoic acid methyl ester (9,8 g), Natronlauge (2 N; 80 mL) und MeOH (300 mL) wurde für 12 Stunden gerührt. Organische flüchtige Anteile wurden am Rotationsverdampfer entfernt. Die verbleibende wässrige Phase wurde mit Methyl-tert.-butylether (MTBE) extrahiert und dann mit konzentrierter Salzsäure sauer gestellt. Der entstandene Niederschlag wurde abfiltriert und getrocknet. Man erhielt so das Produkt mit dem Molekulargewicht 222,24 (C12H14O4); MS (ESI): 223 (M+H+).

### 4-[(S)-1-(Tetrahydro-furan-2-yl)methoxy]-benzoic acid methyl ester

### Methode F

Eine Mischung aus Methanesulfonic acid (S)-1-(tetrahydro-furan-2-yl)methyl ester (7,6 g), 4-Hydroxy-benzoic acid methyl ester (6,4 g), Cäsiumcarbonat (20 g) und NMP (100 mL) wurde für 12 Stunden auf 75 °C erwärmt. Die abgekühlte Reaktionsmischung wurde mit Wasser versetzt und mit Ethylacetat extrahiert. Die organische Phase wurde dreimal mit Wasser gewaschen, über Magnesiumsulfat getrocknet und eingeengt. Man erhielt so das Produkt mit dem Molekulargewicht 236,27 (C13H16O4); MS (ESI): 237 (M+H+).

### Methanesulfonic acid (S)-1-(tetrahydro-furan-2-yl)methyl ester

### Methode G

Zu einer Lösung von (S)-1-(Tetrahydro-furan-2-yl)-methanol (7,95 g) in Pyridin (35 mL) wurde bei -15 °C Methansulfonsäurechlorid (7,47 g) zugegeben und die Reaktion wurde für 5 Stunden bei 0 °C gerührt. Nach der Zugabe von Wasser wurde das Gemisch mit Ethylacetat extrahiert. Die organischen Phasen wurden über Magnesiumsulfat getrocknet und eingeengt. Man erhielt so das Produkt mit dem Molekulargewicht 180,22 (C6H12O4S); MS (ESI): 181 (M+H+). Analog wurde Methanesulfonic acid (R)-1-(tetrahydro-furan-2-yl)methyl ester synthetisiert.

### Beispiel 2

### N-Methyl-N-((S)-6-pyrrolidin-1-ylmethyl-1,2,3,4-tetrahydro-naphthalen-2-yl)-4-[(S)-1-(tetrahydro-furan-2-yl)methoxy]-benzamide

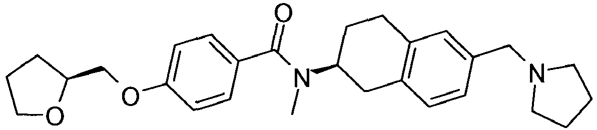

Nach Methode A-1 wurde 4-[(S)-1-(TETRAHYDRO-FURAN-2-YL)METHOXY]-BENZOIC ACID mit Methyl-((S)-6-pyrrolidin-1-ylmethyl-1,2,3,4-tetrahydro-naphthalen-2-yl)-amine umgesetzt. Man erhielt so das Produkt mit dem Molekulargewicht 448,61 (C28H36N2O3); MS (ESI): 449 (M+H+).

### Methyl-((S)-6-pyrrolidin-1-ylmethyl-1,2,3,4-tetrahydro-naphthalen-2-yl)-amine

Nach Methode B wurde [(S)-6-(Pyrrolidine-1-carbonyl)-1,2,3,4-tetrahydro-naphthalen-2-yl]-carbamic acid tert-butyl ester mit Lithiumaluminiumhydrid (10 Äquiv., 60 °C, 2 Stunden) umgesetzt. Man erhielt so das Produkt mit dem Molekulargewicht 244,38 (C16H24N2); MS (ESI): 245 (M+H+).

### Beispiel 3-1

### N-{(S)-6-[(Isobutyl-methyl-amino)-methyl]-1,2,3,4-tetrahydro-naphthalen-2-yl}-4-[(S)-1-(tetrahydro-furan-2-yl)methoxy]-benzamide

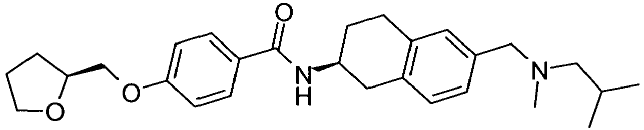

### Methode H-1

Zu einer Mischung aus N-((S)-6-Formyl-1,2,3,4-tetrahydro-naphthalen-2-yl)-4-[(S)-1-(tetrahydro-furan-2-yl)methoxy]-benzamide (0,50 g), THF (5 mL), Methanol (2 mL), Isobutylmethyl-amine (0,23 g) und Essigsäure (0,24 g) wurde polymer-gebundenes Natriumcyanoborhydrid (2,7 mmol) gegeben und die Suspension für 12 Stunden bei Raumtemperatur geschüttelt. Es wurde vom Polymer abgesaugt und das Filtrat eingeengt. Der Rückstand wurde durch präparative HPLC gereinigt. Man erhielt so das Produkt mit dem Molekulargewicht 450,63 (C28H38N2O3); MS (ESI): 451 (M+H+).

### N-((S)-6-Formyl-1,2,3,4-tetrahydro-naphthalen-2-yl)-4-[(S)-1-(tetrahydro-furan-2-yl)methoxy]-benzamide

### Methode I

Eine Mischung aus N-((S)-6-Bromo-1,2,3,4-tetrahydro-naphthalen-2-yl)-4-[(S)-1-(tetrahydro-furan-2-yl)methoxy]-benzamide (10,0 g) und THF (130 mL) wurde auf-78 °C (Trockeneisbad) gekühlt und eine Lösung von Methyllithium (18,9 mL; 1,6 M in Diethylether) zugetropft. Eine Minute nach beendeter Zugabe wurde eine Lösung von Butyllithium (13,9 mL; 2,5 M in Toluol) zugetropft. Eine Minute nach beendeter Zugabe wurde DMF (5,1 g) und nach weiteren 30 Sekunden Essigsäure (4,5 mL) zugesetzt. Nach dem Erwärmen auf Raumtemperatur wurde die Reaktionsmischung mit Wasser verdünnt und mit Ethylacetat extrahiert. Die organischen Phasen wurden über Magnesiumsulfat getrocknet und eingeengt. Man erhielt so das Produkt mit dem Molekulargewicht 379,46 (C23H25NO4); MS (ESI): 380 (M+H+). Analog wurde N-((R)-6-Formyl-1,2,3,4-tetrahydro-naphthalen-2-yl)-4-[(S)-1-(tetrahydro-furan-2-yl)methoxy]-benzamide aus N-((R)-6-Bromo-1,2,3,4-tetrahydro-naphthalen-2-yl)-4-[(S)-1-(tetrahydro-furan-2-yl)methoxy]-benzamide hergestellt.

### N-((S)-6-Bromo-1,2, 3,4-tetrahydro-naphthalen-2-yl)-4-[(S)-1-(tetrahydro-furan-2-yl)methoxy]-benzamide

Nach Methode F wurde N-((S)-6-Bromo-1,2,3,4-tetrahydro-naphthalen-2-yl)-4-hydroxy-benzamide mit Methanesulfonic acid (S)-1-(tetrahydro-furan-2-yl)methyl ester umgesetzt. Man erhielt so das Produkt mit dem Molekulargewicht 430,35 (C22H24BrNO3); MS (ESI): 430 (M+H+).

### N-((S)-6-Bromo-1,2,3,4-tetrahydro-naphthalen-2-yl)-4-hydroxy-benzamide

Eine Mischung aus N-((S)-6-Bromo-1,2,3,4-tetrahydro-naphthalen-2-yl)-4-methoxy-benzamide (3,0 g), Eisessig (2 mL) und HBr (20 mL; 48%ig in Wasser) wurde in einem verschlossenen Glasgefäß in einem Mikrowellenreaktor für 25 Minuten auf 150°C erwärmt. Der nach dem Abkühlen erhaltene Niederschlag wurde abfiltriert. Man erhielt so das Produkt mit dem Molekulargewicht 346,23 (C17H16BrNO2); MS (ESI): 346 (M+H+).
Sowohl N-((S)-6-Bromo-1,2,3,4-tetrahydro-naphthalen-2-yl)-4-methoxy-benzamide als auch N-((R)-6-Bromo-1,2,3,4-tetrahydro-naphthalen-2-yl)-4-methoxy-benzamide können nach Literaturvorschriften (J. Org. Chem. 1995, 60, 4324) erhalten werden.

Die in der Tabelle 1 zusammengefassten Beispiele wurden durch Umsetzung der entsprechenden Carbonylverbindungen (Aldehyde bzw. Ketone) mit den entsprechenden Aminen nach Methode H-1 erhalten.

**Tabelle 1.**

| Bsp. No. | Struktur | Molekular-gewicht | ESI-MS [M+H]+ |
|---|---|---|---|
| 3-2 | 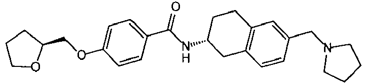 | 434.58 | 435 |
| 3-3 | 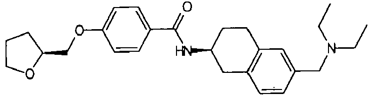 | 436.59 | 437 |
| 3-4 | 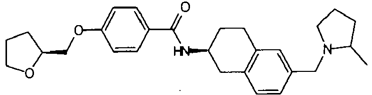 | 448.60 | 449 |
| 3-5 | 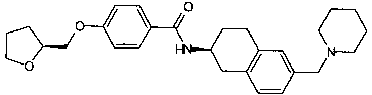 | 448.60 | 449 |
| 3-6 | 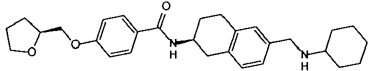 | 462.63 | 463 |
| 3-7 | 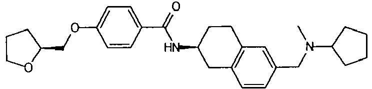 | 462.63 | 463 |
| 3-8 | 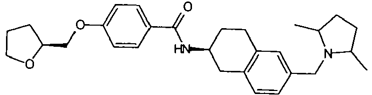 | 462.63 | 463 |
| 3-9 | 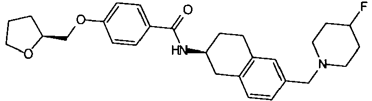 | 466.59 | 467 |
| 3-10 | 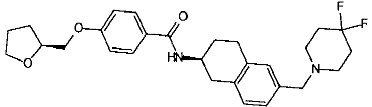 | 484.58 | 485 |
| 3-11 | 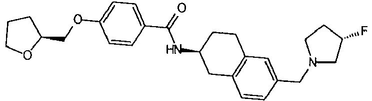 | 452.57 | 453 |
| 3-12 | 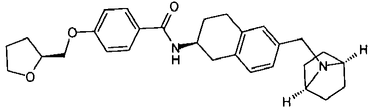 | 460.61 | 461 |
| 3-13 | 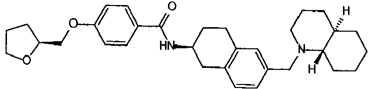 | 502.70 | 503 |
| 3-14 | 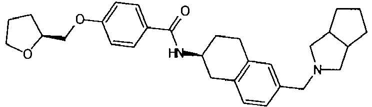 | 474.64 | 475 |
| 3-15 | 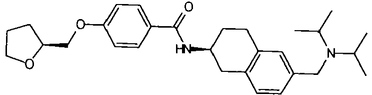 | 464.65 | 465 |
| 3-16 | 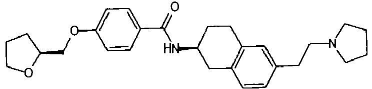 | 448.60 | 449 |
| 3-17 | 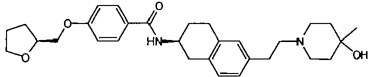 | 492.66 | 493 |
| 3-18 | 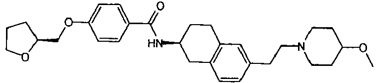 | 492.66 | 493 |
| 3-19 | 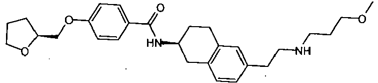 | 466.62 | 467 |
| 3-20 | 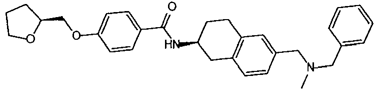 | 484.64 | 485 |
| 3-21 | 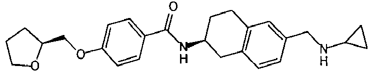 | 420.55 | 421 |
| 3-22 | 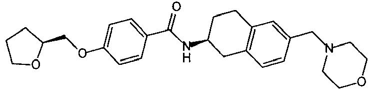 | 450.58 | 451 |
| | | | |
| 3-23 | 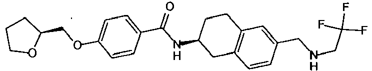 | 462.51 | 463 |
| 3-24 | 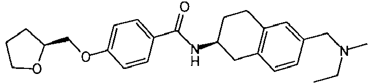 | 422.57 | 423 |
| 3-25 | 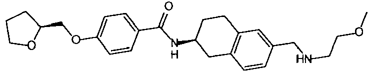 | 438.57 | 439 |
| 3-26 | 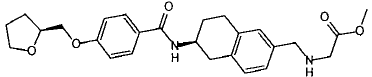 | 452.55 | 453 |
| 3-27 | 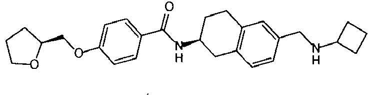 | 434.58 | 435 |
| 3-28 | 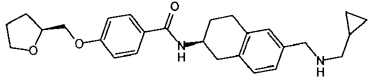 | 434.58 | 435 |
| 3-29 | 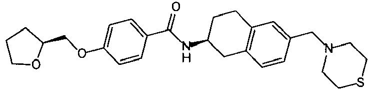 | 466.64 | 467 |
| 3-30 | 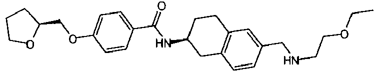 | 452.59 | 453 |
| 3-31 | 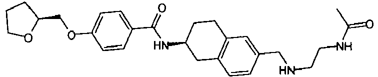 | 465.59 | 466 |
| 3-32 | 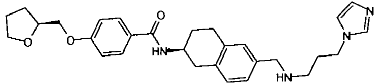 | 488.63 | 489 |
| 3-33 | 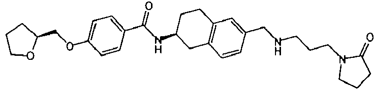 | 505.66 | 506 |
| 3-34 | 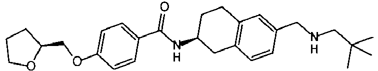 | 450.62 | 451 |
| 3-35 | 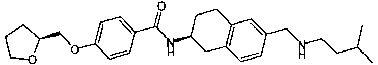 | 450.62 | 451 |
| 3-36 | 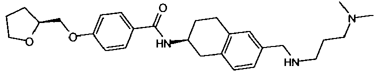 | 465.63 | 466 |
| 3-37 | 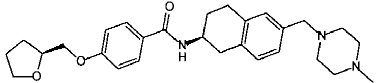 | 463.62 | 464 |
| 3-38 | 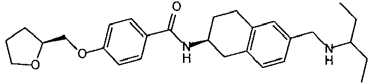 | 450.62 | 451 |
| 3-39 | 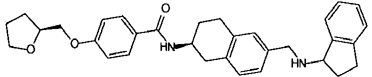 | 496.65 | 497 |
| 3-40 | 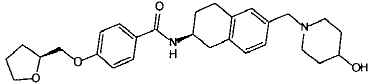 | 464.60 | 465 |
| 3-41 | 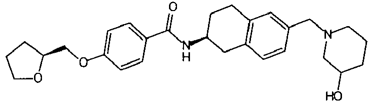 | 464.60 | 465 |
| 3-42 | 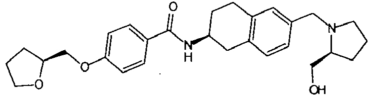 | 464.60 | 465 |
| 3-43 | 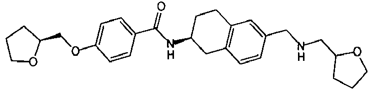 | 464.60 | 465 |
| 3-44 | 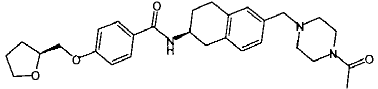 | 491.63 | 492 |
| 3-45 | 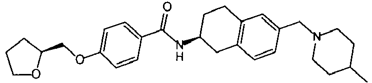 | 462.63 | 463 |
| 3-46 | 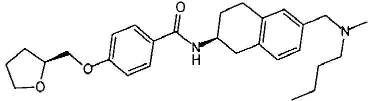 | 450.62 | 451 |
| 3-47 | 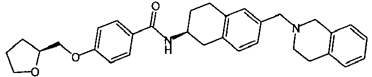 | 496.65 | 497 |
| 3-48 | 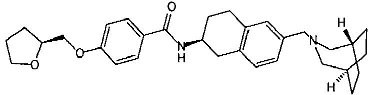 | 488.67 | 489 |
| 3-49 | 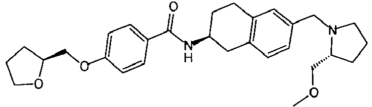 | 478.63 | 479 |
| 3-50 | 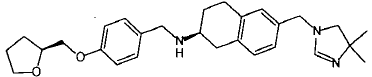 | 461.60 | 462 |
| 3-51 | 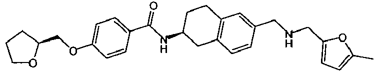 | 474.60 | 475 |
| 3-52 | 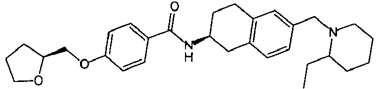 | 476.66 | 477 |
| 3-53 | 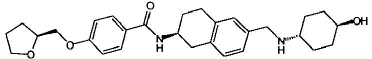 | 478.63 | 479 |
| 3-54 | 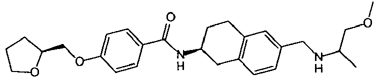 | 452.59 | 453 |
| 3-55 | 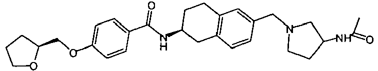 | 491.63 | 492 |
| 3-56 | 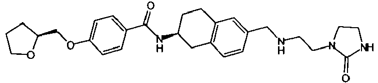 | 492.62 | 493 |
| 3-57 | 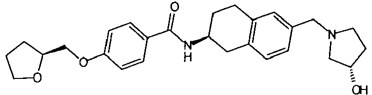 | 450.58 | 451 |
| 3-58 | 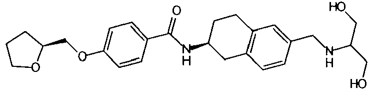 | 454.56 | 455 |
| 3-59 | 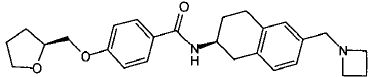 | 420.55 | 421 |
| 3-60 | 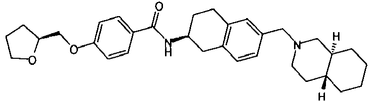 | 502.70 | 503 |
| 3-61 | 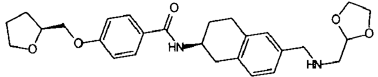 | 466.58 | 467 |
| 3-62 | 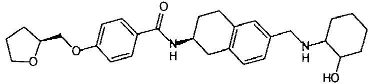 | 478.63 | 479 |
| 3-63 | 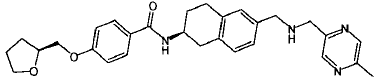 | 486.61 | 487 |
| 3-64 | 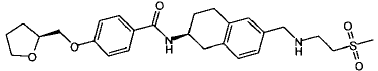 | 486.63 | 487 |
| 3-65 | 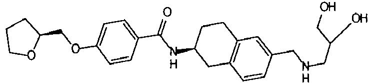 | 454.56 | 455 |
| 3-66 | 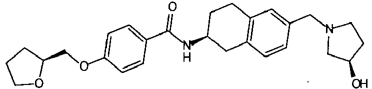 | 450.58 | 451 |
| 3-67 | 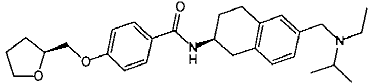 | 450.62 | 451 |
| 3-68 | 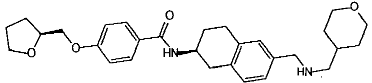 | 478.63 | 479 |
| 3-69 | 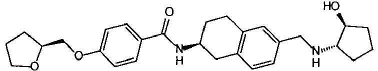 | 464.60 | 465 |
| 3-70 | 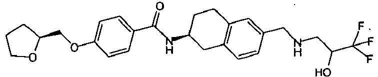 | 492.54 | 493 |
| 3-71 | 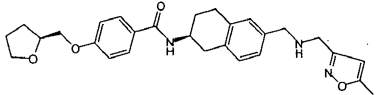 | 475.59 | 476 |
| 3-72 | 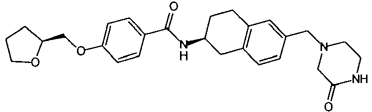 | 463.58 | 464 |
| 3-73 | 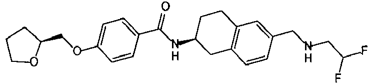 | 444.52 | 445 |
| 3-74 | 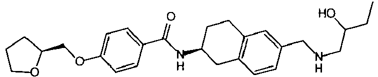 | 452.59 | 453 |
| 3-75 | 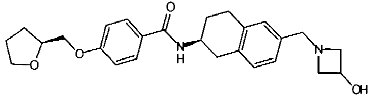 | 436.55 | 437 |
| 3-76 | 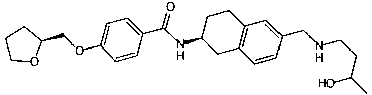 | 452.59 | 453 |
| 3-77 | 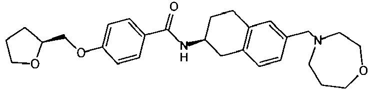 | 464.60 | 465 |
| 3-78 | 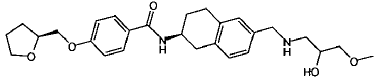 | 468.59 | 469 |
| 3-79 | 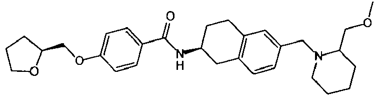 | 492.66 | 493 |
| 3-80 | 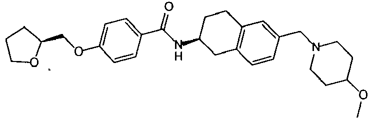 | 478.63 | 479 |
| 3-81 | 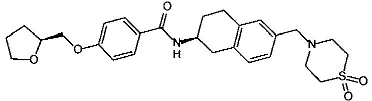 | 498.64 | 499 |
| 3-82 | 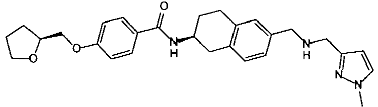 | 474.60 | 475 |
| 3-83 | 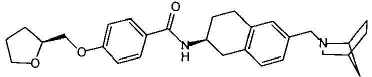 | 460.61 | 461 |
| 3-84 | 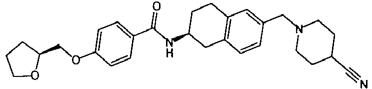 | 473.61 | 474 |
| 3-85 | 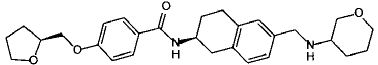 | 464.60 | 465 |
| 3-86 | 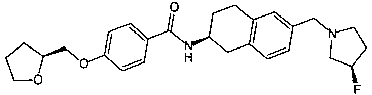 | 452.57 | 453 |
| 3-87 | 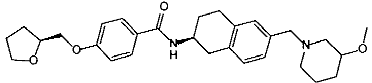 | 478.63 | 479 |
| 3-88 | 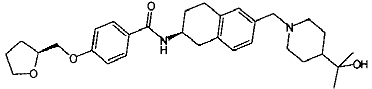 | 506.68 | 507 |
| 3-89 | 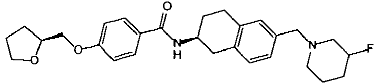 | 466.59 | 467 |
| 3-90 | 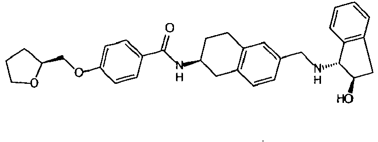 | 512.65 | 513 |
| 3-91 | 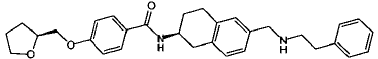 | 484.64 | 485 |
| 3-92 | 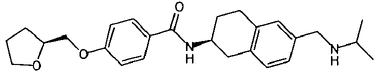 | 422.57 | 423 |
| 3-93 | 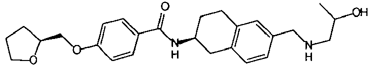 | 438.57 | 439 |
| 3-94 | 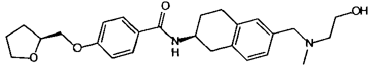 | 438.57 | 439 |
| 3-95 | 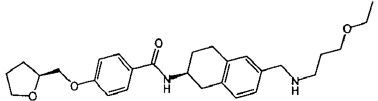 | 466.62 | 467 |
| 3-96 | 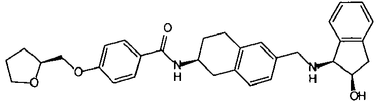 | 512.65 | 513 |
| 3-97 | 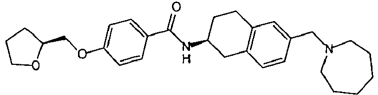 | 462.63 | 463 |
| | | | |
| 3-98 | 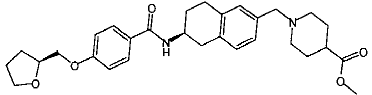 | 506.64 | 507 |
| 3-99 | 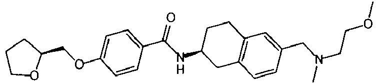 | 452.59 | 453 |
| 3-100 | 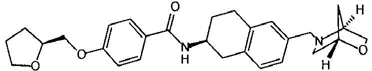 | 462.59 | 463 |
| 3-101 | 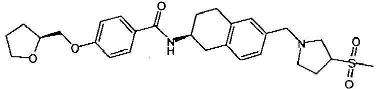 | 512.67 | 513 |
| 3-102 | 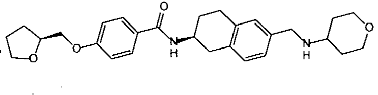 | 464.60 | 465 |
| 3-103 | 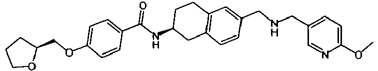 | 501.62 | 502 |
| 3-104 | 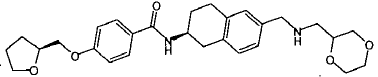 | 480.60 | 481 |
| 3-105 | 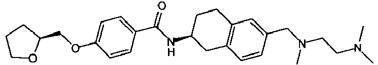 | 465.63 | 466 |
| 3-106 | 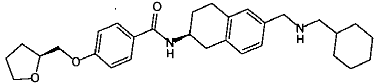 | 476.66 | 477 |
| 3-107 | 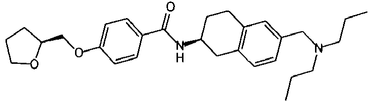 | 464.65 | 465 |
| 3-108 | 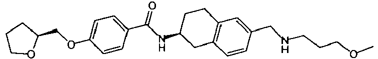 | 452.59 | 453 |
| 3-109 | 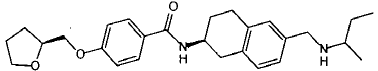 | 436.59 | 437 |
| 3-110 | 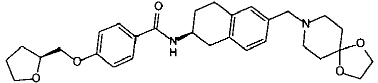 | 506.64 | 507 |
| 3-111 | 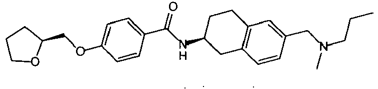 | 436.59 | 437 |
| 3-112 | 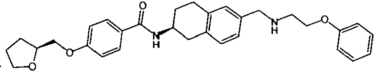 | 500.64 | 501 |
| 3-113 | 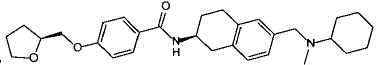 | 476.66 | 477 |
| 3-114 | 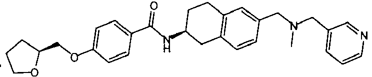 | 485.63 | 486 |
| 3-115 | 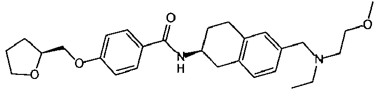 | 466.62 | 467 |
| 3-116 | 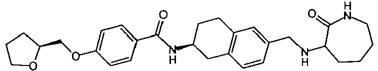 | 491.63 | 492 |
| 3-117 | 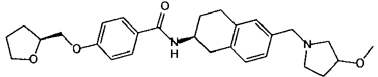 | 464.60 | 465 |
| 3-118 | 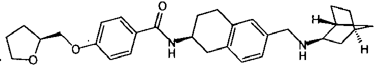 | 474.64 | 475 |
| 3-119 | 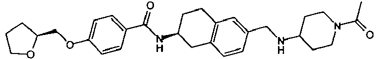 | 505.66 | 506 |
| 3-120 | 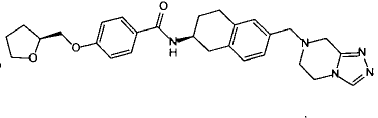 | 487.60 | 488 |
| 3-121 | 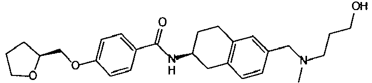 | 452.59 | 453 |
| 3-122 | 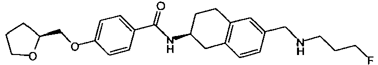 | 440.56 | 441 |
| 3-123 | 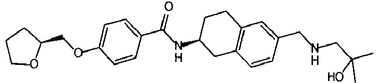 | 452.59 | 453 |
| 3-124 | 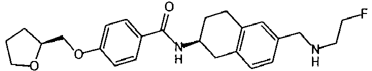 | 426.53 | 427 |
| 3-125 | 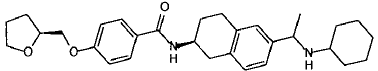 | 476.66 | 477 |
| 3-126 | 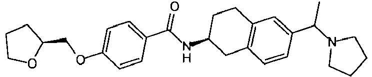 | 448.60 | 449 |
| 3-127 | 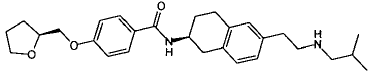 | 450.62 | 451 |
| 3-128 | 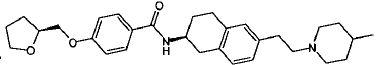 | 476.66 | 477 |
| 3-129 | 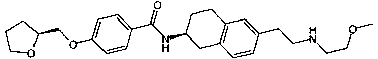 | 452.59 | 453 |
| 3-130 | 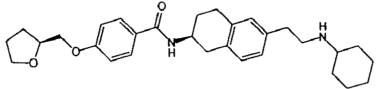 | 476.66 | 477 |
| 3-131 | 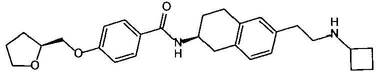 | 448.60 | 449 |
| 3-132 | 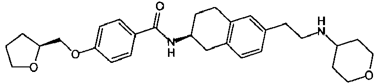 | 478.63 | 479 |
| 3-133 | 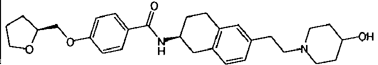 | 478.63 | 479 |
| 3-134 | 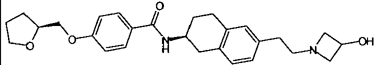 | 450.58 | 451 |
| 3-135 | 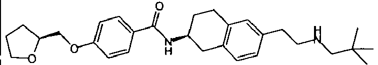 | 464.65 | 465 |
| 3-136 | 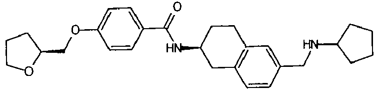 | 448.60 | 449 |
| 3-137 | 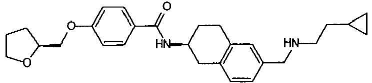 | 448.60 | 449 |
| 3-138 | 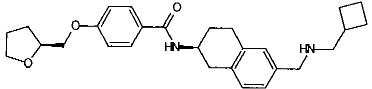 | 448.60 | 449 |
| 3-139 | 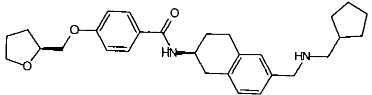 | 462.63 | 463 |
| 3-140 | 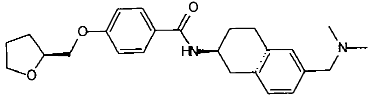 | 408.54 | 409 |
| 3-141 | 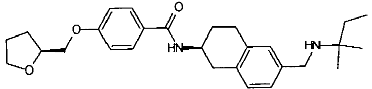 | 450.62 | 451 |
| | | | |
| 3-142 | 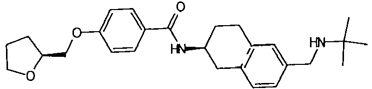 | 436.59 | 437 |
| 3-143 | 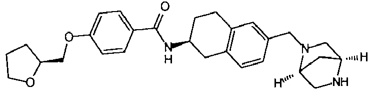 | 461.60 | 462 |
| 3-144 | 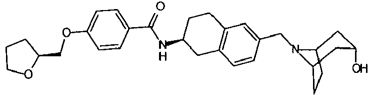 | 490.64 | 491 |
| 3-145 | 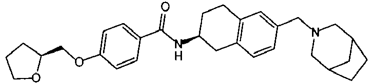 | 474.64 | 475 |
| 3-146 | 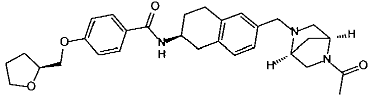 | 503.64 | 504 |
| 3-147 | 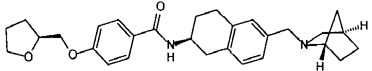 | 460.61 | 461 |
| 3-148 | 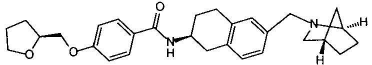 | 460.61 | 461 |
| 3-149 | 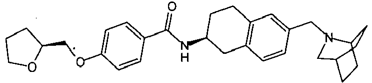 | 474.64 | 475 |
| | | | |
| 3-150 | 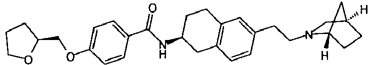 | 474.64 | 475 |
| 3-151 | 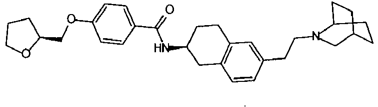 | 488.67 | 489 |
| 3-152 | 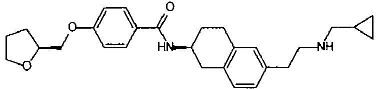 | 448.60 | 449 |
| 3-153 | 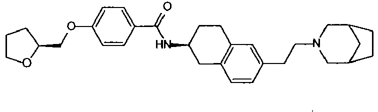 | 488.67 | 489 |
| 3-154 | 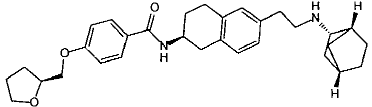 | 488.67 | 489 |
| 3-155 | 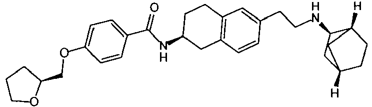 | 488.67 | 489 |
| 3-156 | 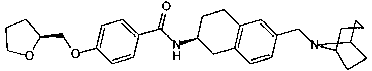 | 474.64 | 475 |
| 3-157 | 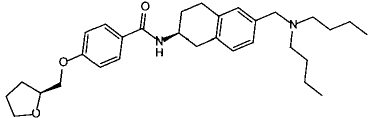 | 492.70 | 493 |
| 3-158 | 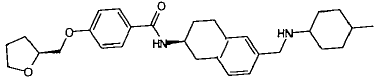 | 476.66 | 477 |
| 3-159 | 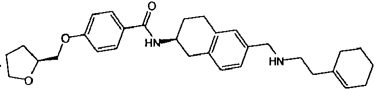 | 488.67 | 489 |
| 3-160 | 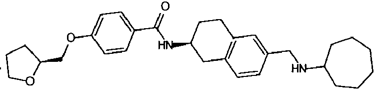 | 476.66 | 477 |
| 3-161 | 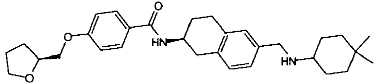 | 490.68 | 491 |
| 3-162 | 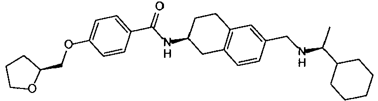 | 490.68 | 491 |
| 3-163 | 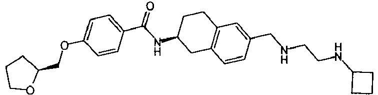 | 477.65 | 478 |
| 3-164 | 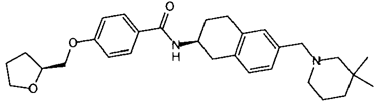 | 476.66 | 477 |
| 3-165 | 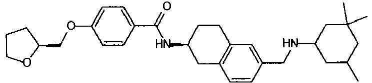 | 504.71 | 505 |
| 3-166 | 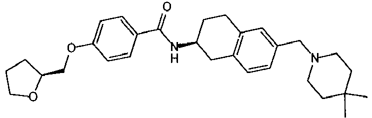 | 476.66 | 477 |
| 3-167 | 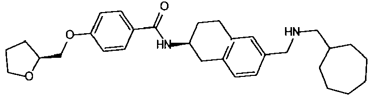 | 490.68 | 491 |
| 3-168 | 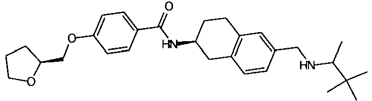 | 464.65 | 465 |
| 3-169 | 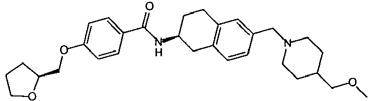 | 492.66 | 493 |
| 3-17C | 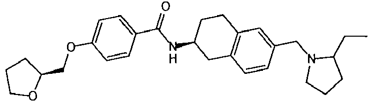 | 462.63 | 463 |
| 3-171 | 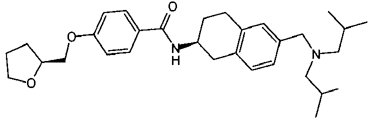 | 492.70 | 493 |
| 3-172 | 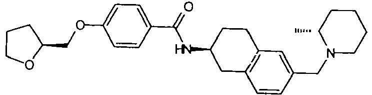 | 462.63 | 463 |
| | | | |
| 3-173 | 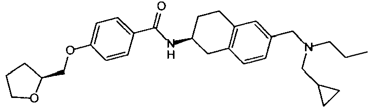 | 476.66 | 477 |
| 3-174 | 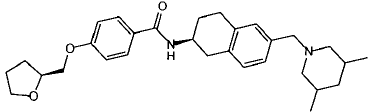 | 476.66 | 477 |
| 3-175 | 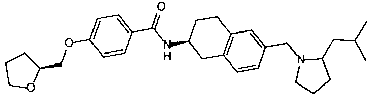 | 490.68 | 491 |
| 3-176 | 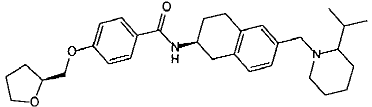 | 490.68 | 491 |
| 3-177 | 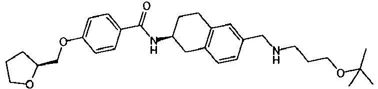 | 494.67 | 495 |
| 3-178 | 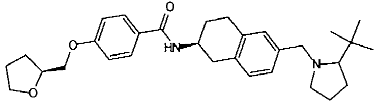 | 490.68 | 491 |
| 3-179 | 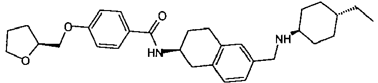 | 490.68 | 491 |
| 3-180 | 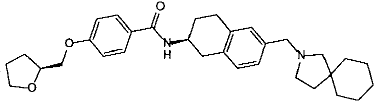 | 502.70 | 503 |
| 3-181 | 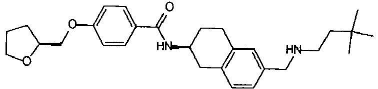 | 464.65 | 465 |
| 3-182 | 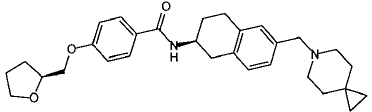 | 474.64 | 475 |
| 3-183 | 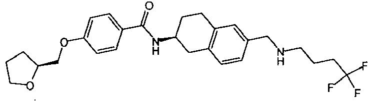 | 490.56 | 491 |
| 3-184 | 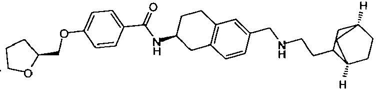 | 502.70 | 503 |
| 3-185 | 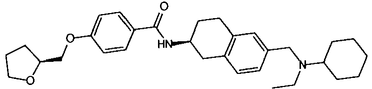 | 490.68 | 491 |
| 3-186 | 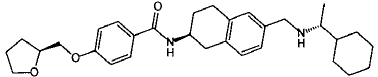 | 490.68 | 491 |
| 3-187 | 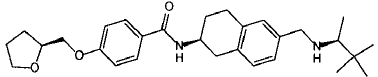 | 464.65 | 465 |
| 3-188 | 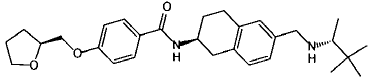 | 464.65 | 465 |
| 3-189 | 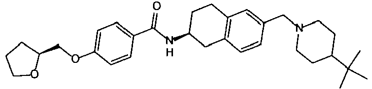 | 504.71 | 505 |
| 3-190 | 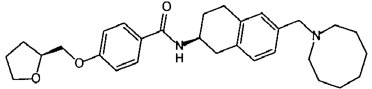 | 476.66 | 477 |
| 3-191 | 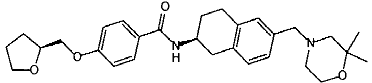 | 478.63 | 479 |
| 3-192 | 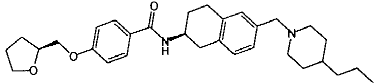 | 490.68 | 491 |
| 3-193 | 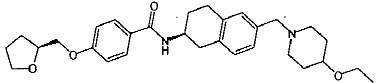 | 492.66 | 493 |
| 3-194 | 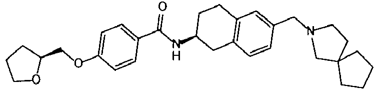 | 488.67 | 489 |
| 3-195 | 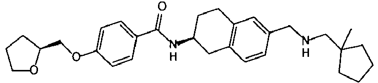 | 476.66 | 477 |
| 3-196 | 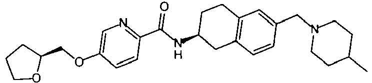 | 463,63 | 464 |
| | | | |
| 3-197 | 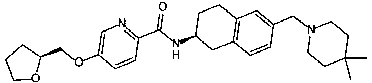 | 477,65 | 478 |
| 3-198 | 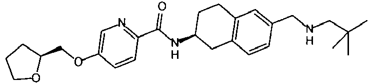 | 451,61 | 452 |
| 3-199 | 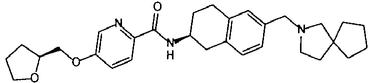 | 489,66 | 490 |
| 3-200 | 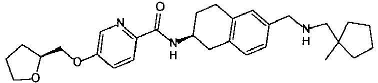 | 477,65 | 478 |
| 3-201 | 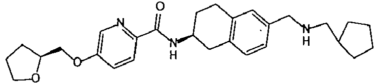 | 463,63 | 464 |
| 3-202 | 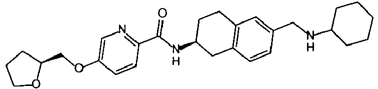 | 463,63 | 464 |
| 3-203 | 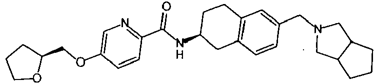 | 475,64 | 476 |
| 3-204 | 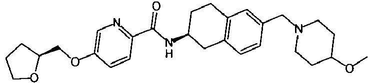 | 479,62 | 480 |
| 3-205 | 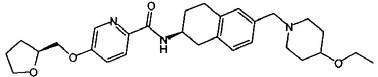 | 493,65 | 494 |
| 3-206 | 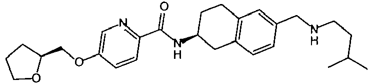 | 451,61 | 452 |
| 3-207 | 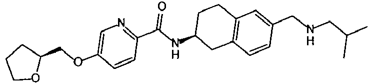 | 437,59 | 438 |
| 3-208 | 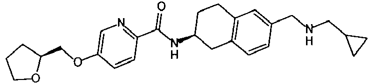 | 435,57 | 436 |
| 3-209 | 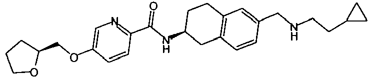 | 449,60 | 450 |
| 3-210 | 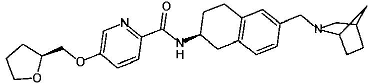 | 461,61 | 462 |
| 3-211 | 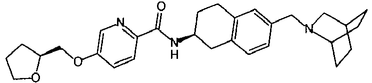 | 475,64 | 476 |
| 3-212 | 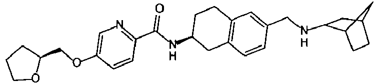 | 475,64 | 476 |
| 3-213 | 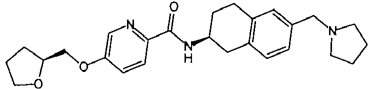 | 435,57 | 436 |
| 3-214 | 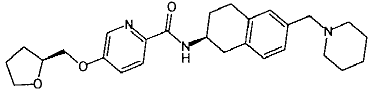 | 449,60 | 450 |

### Herstellung benötigter Startmaterialien (Tabelle 1)

### N-((S)-6-Acetyl-1,2,3,4-tetrahydro-naphthalen-2-yl)-4-[(S)-1-(tetrahydro-furan-2-yl)methoxy]-benzamide

Eine Mischung aus N-((S)-6-Bromo-1,2,3,4-tetrahydro-naphthalen-2-yl)-4-[(S)-1-(tetrahydro-furan-2-yl)methoxy]-benzamide (1,0 g) und THF (10 mL) wurde auf -78 °C (Trockeneisbad) gekühlt und eine Lösung von Methyllithium (2,0 mL; 1,6 M in Diethylether) zugetropft. Eine Minute nach beendeter Zugabe wurde eine Lösung von Butyllithium (1,4 mL; 2,5 M in Toluol) zugetropft. Eine Minute nach beendeter Zugabe wurde N-Methoxy-N-methylacetamide (0,24 g) zugesetzt. Nach dem Erwärmen auf Raumtemperatur wurde die Reaktionsmischung mit Wasser verdünnt und mit Ethylacetat extrahiert. Die organischen Phasen wurden über Magnesiumsulfat getrocknet und eingeengt. Der Rückstand wurde an Kieselgel chromatographiert (Eluent: Heptan/Ethylacetat 1:1). Man erhielt so das Produkt mit dem Molekulargewicht 393,49 (C24H27NO4); MS (ESI): 394 (M+H+).

### N-[(S)-6-(2-Oxo-ethyl)-1,2,3,4-tetrahydro-naphthalen-2-yl]-4-[(S)-1-(tetrahydro-furan-2-yl)methoxy] -benzamide

Eine Mischung aus N-((S)-6-Allyl-1,2,3,4-tetrahydro-naphthalen-2-yl)-4-[(S)-1-(tetrahydro-furan-2-yl)methoxy]-benzamide (0,50 g), 2-Propanol (50 mL) und Wasser (50 mL) wurde mit Natriumperiodat (0,60 g) und Osmiumtetroxid (1,3 mg) versetzt. Nach 14 Stunden heftigem Rühren wurde die Reaktionsmischung mit Ethylacetat extrahiert. Die organische Phase wurde mit Kochsalzlösung gewaschen, über Natriumsulfat getrocknet und eingeengt. Man erhielt so das Produkt mit dem Molekulargewicht 393,49 (C24H27NO4); MS (ESI): 394 (M+H+).

### N-((S)-6-Allyl-1,2,3,4-tetrahydro-naphthalen-2-yl)-4-[(S)-1-(tetrahydro-furan-2-yl)methoxy]-benzamide

Eine Mischung aus N-((S)-6-Bromo-1,2,3,4-tetrahydro-naphthalen-2-yl)-4-[(S)-1-(tetrahydro-furan-2-yl)methoxy]-benzamide (1,00 g), Toluol (10 mL), Pd(PPh3)4 (26,7 mg) und Allyltributylzinn (2,46 g) wurde für 5 Stunden am Rückfluss gekocht. Die abgekühlte Reaktionsmischung wurde mit Ethylacetat verdünnt und mit Wasser gewaschen. Die organische Phase wurde mit Kochsalzlösung gewaschen, über Natriumsulfat getrocknet und eingeengt. Der Rückstand wurde durch Chromatographie an Kieselgel gereinigt. Man erhielt so das Produkt mit dem Molekulargewicht 391,51 (C25H29NO3); MS (ESI): 392 (M+H+).

### 5-[(S)-1-(Tetrahydro-furan-2-yl)methoxy]-pyridine-2-carboxylic acid ((S)-6-formyl-1,2,3,4-tetrahydro-naphthalen-2-yl)-amide

Nach Methode A wurde (S)-6-Bromo-1,2,3,4-tetrahydro-naphthalen-2-ylamine mit 5-Hydroxy-pyridine-2-carboxylic acid umgesetzt. Das erhaltene Amid wurde nach Methode F mit Methanesulfonic acid (S)-1-(tetrahydro-furan-2-yl)methyl ester alkyliert und nach Methode I in den gewünschten Aldehyd überführt. Man erhielt so das Produkt mit dem Molekulargewicht 380,45 (C22H24N2O4); MS (ESI): 381 (M+H+).

### Beispiel 4

### N-((S)-6-Piperidin-3-yl-1,2,3,4-tetrahydro-naphthalen-2-yl)-4-[(S)-1-(tetrahydro-furan-2-yl)methoxy]-benzamide

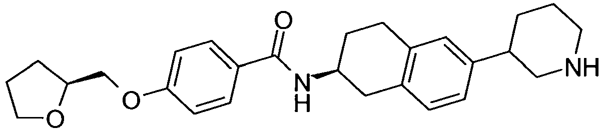

### Methode J-1

Eine Mischung aus N-((S)-6-Pyridin-3-yl-1,2,3,4-tetrahydro-naphthalen-2-yl)-4-[(S)-1-(tetrahydro-furan-2-yl)methoxy]-benzamide (0,60 g), Eisessig (30 mL) und Platin(IV)-oxid (0,10 g) wurde für 12 Stunden unter einer Wasserstoffatmosphäre (Ballon) heftig gerührt. Es wurde vom Katalysator abgesaugt und das Filtrat eingeengt. Man erhielt so das Produkt mit dem Molekulargewicht 434,58 (C27H34N2O3); MS (ESI): 435 (M+H+).
Durch präparative Trennung an einer chiralen Phase (Chiralpak AD-H) wurden die reinen Diastereomere (N-((S)-(R)-6-Piperidin-3-yl-1,2,3,4-tetrahydro-naphthalen-2-yl)-4-[(S)-1-(tetrahydro-furan-2-yl)methoxy]-benzamide und N-((S)-(S)-6-Piperidin-3-yl-1,2,3,4-tetrahydro-naphthalen-2-yl)-4-[(S)-1-(tetrahydro-furan-2-yl)methoxy]-benzamide) erhalten.

### N-((S)-6-Pyridin-3-yl-1,2,3,4-tetrahydro-naphthalen-2-yl)-4-[(S)-1-(tetrahydro-furan-2-yl)methoxy]-benzamide

Eine Mischung aus N-((S)-6-Bromo-1,2,3,4-tetrahydro-naphthalen-2-yl)-4-[(S)-1-(tetrahydro-furan-2-yl)methoxy]-benzamide (1,00 g), Toluol (10 mL) und Pd(PPh3)4 (2,69 g) wurde mit 3-Pyridyl-boronsäure (0,28 g), Ethanol (3 mL) und Caesiumcarbonatlösung (1,2 mL; 2 M in Wasser) versetzt. Es wurde für 7 Stunden am Rückfluss gekocht. Die abgekühlte Reaktionsmischung wurde mit Ethylacetat verdünnt und mit Wasser gewaschen. Die organische Phase wurde mit Kochsalzlösung gewaschen, über Natriumsulfat getrocknet und eingeengt. Der Rückstand wurde durch Chromatographie an Kieselgel gereinigt. Man erhielt so das Produkt mit dem Molekulargewicht 428,54 (C27H28N2O3); MS (ESI): 429 (M+H+).

### Beispiel 5

### N-((S)-6-Piperidin-2-yl-1,2,3,4-tetrahydro-naphthalen-2-yl)-4-[(S)-1-(tetrahydro-furan-2-yl)methoxy]-benzamide

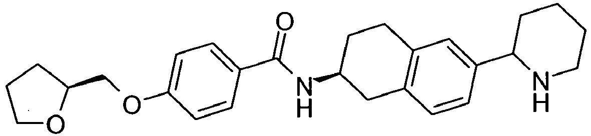

Nach Methode J-1 wurde N-[(S)-6-(1-Oxy-pyridin-2-yl)-1,2,3,4-tetrahydro-naphthalen-2-yl]-4-[(S)-1-(tetrahydro-furan-2-yl)methoxy]-benzamide hydriert. Man erhielt so das Produkt mit dem Molekulargewicht 434,58 (C27H34N2O3); MS (ESI): 435 (M+H+).

### N-[(S)-6-(1-Oxy-pyridin-2-yl)-1,2,3,4-tetrahydro-naphthalen-2-yl]-4-[(S)-1-(tetrahydro-furan-2-yl)methoxy]-benzamide

Eine Mischung aus Pyridin-N-oxid (0,88 g), Kaliumcarbonat (0,64 g), Tri-tert.-butylphosphin (0,101 g; HBF4-Addukt) und Palladium(II)-acetat (26 mg) wurde mit einer Lösung von N-((S)-6-Bromo-1,2, 3,4-tetrahydro-naphthalen-2-yl)-4-[(S)-1-(tetrahydro-furan-2-yl)methoxy] - benzamide (1,00 g) in Toluol (8 mL) versetzt. Es wurde für 5 Stunden am Rückfluss gekocht. Die abgekühlte Reaktionsmischung wurde mit Ethylacetat verdünnt und mit Wasser gewaschen. Die organische Phase wurde mit Kochsalzlösung gewaschen, über Natriumsulfat getrocknet und eingeengt. Der Rückstand wurde durch Chromatographie an Kieselgel gereinigt (Eluent: Dichlormethan/Methanol 15:1). Man erhielt so das Produkt mit dem Molekulargewicht 444,54 (C27H28N2O4); MS (ESI): 445 (M+H+).

### Beispiel 6

### N-[(S)-6-((S)-1-Amino-ethyl)-1,2,3,4-tetrahydro-naphthalen-2-yl]-4-[(S)-1-(tetrahydro-furan-2-yl)methoxy]-benzamide

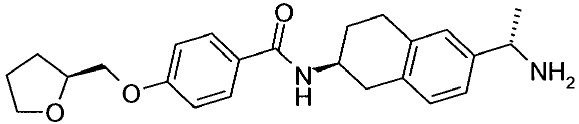

### Methode K

Eine Mischung aus N-{(S)-6-[(S)-1-((R)-2-Methyl-propane-2-sulfinylamino)-ethyl]-1,2,3,4-tetrahydro-naphthalen-2-yl}-4-[(S)-1-(tetrahydro-furan-2-yl)methoxy]-benzamide (0,12 g) und Methanol (2 mL) wurde mit Chlorwasserstoff (2 mL; 5 M in 2-Propanol) versetzt. Nach 30 Minuten wurde die Reaktionsmischung eingeengt. Man erhielt so das Produkt mit dem Molekulargewicht 394,52 (C24H30N2O3); MS (ESI): 395 (M+H+).
Analog kann N-[(S)-6-((R)-1-Amino-ethyl)-1,2,3,4-tetrahydro-naphthalen-2-yl]-4-[(S)-1-(tetrahydro-furan-2-yl)methoxy]-benzamide dargestellt werden.

### N-{(S)-6-[(S)-1-((R)-2-Methyl-propane-2-sulfinylamino)-ethyl]-1,2,3,4-tetrahydro-naphthalen-2-yl}-4-[(S)-1-(tetrahydro-furan-2-yl)methoxy]-benzamide

### Methode L

Eine auf -45°C gekühlte Suspension von N-((S)-6-{[(E)-(R)-2-Methyl-propane-2-sulfinylimino]-methyl}-1,2,3,4-tetrahydro-naphthalen-2-yl)-4-[(S)-1-(tetrahydro-furan-2-yl)methoxy]-benzamide (0,34 g), Diethylether (15 mL) und Dichlormethan (15 mL) wurde mit Methylmagnesiumbromid (1,1 mL; 1,4 M in Toluol) versetzt. Nach dem Aufwärmen auf Raumtemperatur wurde noch 5 Stunden gerührt. Die Reaktionsmischung wurde vorsichtig mit Wasser hydrolysiert und mit Ethylacetat extrahiert. Die organische Phase wurde mit Kochsalzlösung gewaschen, über Natriumsulfat getrocknet und eingeengt. Der Rückstand wurde durch Chromatographie an Kieselgel gereinigt (Eluent: Ethylacetat). Man erhielt so das Produkt mit dem Molekulargewicht 498,69 (C28H38N2O4S); MS (ESI): 499 (M+H+).

### N-((S)-6-{[(E)-(R)-2-Methyl-propane-2-sulfinylimino]-methyl}-1,2,3,4-tetrahydro-naphthalen-2-yl)-4-[(S)-1-(tetrahydro-furan-2-yl)methoxy]-benzamide

### Methode M-1

Eine Mischung aus N-((S)-6-Formyl-1,2,3,4-tetrahydro-naphthalen-2-yl)-4-[(S)-1-(tetrahydro-furan-2-yl)methoxy]-benzamide (1,0 g), (R)-2-Methyl-propane-2-sulfinic acid amide (0,32 g), Pyridinium para-toluolsulfonat (165 mg), Kupfer(II)-sulfat (1,0 g; wasserfrei) und Dichlormethan (10 mL) wurde für 24 Stunden gerührt. Es wurde von festen Anteilen abfiltriert und das Filtrat eingeengt. Der Rückstand wurde durch Chromatographie an Kieselgel gereinigt. Man erhielt so das Produkt mit dem Molekulargewicht 482,65 (C27H34N2O4S); MS (ESI): 483 (M+H+).

### Beispiel 7

### N-[(S)-6-(1-Amino-1-methyl-ethyl)-1,2,3,4-tetrahydro-naphthalen-2-yl]-4-[(S)-1-(tetrahydro-furan-2-yl)methoxy]-benzamide

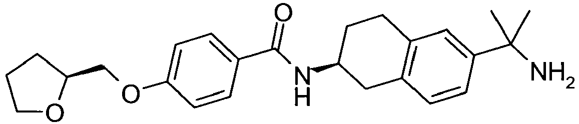

Eine auf 0°C gekühlte Mischung aus N-[(S)-6-(1-Hydroxy-1-methyl-ethyl)-1,2,3,4-tetrahydro-naphthalen-2-yl]-4-[(S)-1-(tetrahydro-furan-2-yl)methoxy]-benzamide (0,30 g), Eisessig (1 mL) und Trimethylsilylcyanid (145 mg) wurde tropfenweise mit Schwefelsäure (1,2 mL; 96%ig) versetzt. Das Kühlbad wurde entfernt und noch 12 Stunden gerührt. Das Reaktionsgemisch wurde mit Wasser verdünnt und mit Ethylacetat extrahiert. Die organische Phase wurde mit Kochsalzlösung gewaschen, über Natriumsulfat getrocknet und eingeengt. Der Rückstand wurde in 1,4-Dioxan (20 mL) aufgenommen und mit verdünnter Salzsäure für 30 Minuten am Rückfluss gekocht. Die Reaktionsmischung wurde mit Ethylacetat gewaschen und mit konzentrierter Natronlauge basisch gestellt. Extraktion mit Dichlormethan ergab eine organische Phase, die über Natriumsulfat getrocknet und eingeengt wurde. (Alternativ kann die Hydrolyse des intermediären Formamids auch durch Kochen mit Natronlauge erreicht werden.) Man erhielt so das Produkt mit dem Molekulargewicht 408,55 (C25H32N2O3); MS (ESI): 409 (M+H+).

### N-[(S)-6-(1-Hydroxy-1-methyl-ethyl)-1,2,3,4-tetrahydro-naphthalen-2-yl]-4-[(S)-1-(tetrahydro-furan-2-yl)methoxy]-benzamide

Eine Mischung aus N-((S)-6-Bromo-1,2,3,4-tetrahydro-naphthalen-2-yl)-4-[(S)-1-(tetrahydro-furan-2-yl)methoxy]-benzamide (1,0 g) und THF (10 mL) wurde auf -78 °C (Trockeneisbad) gekühlt und eine Lösung von Methyllithium (2,0 mL; 1,6 M in Diethylether) zugetropft. Eine Minute nach beendeter Zugabe wurde eine Lösung von Butyllithium (1,4 mL; 2,5 M in Toluol) zugetropft. Eine Minute nach beendeter Zugabe wurde Aceton (0,14 g) zugesetzt. Nach dem Erwärmen auf Raumtemperatur wurde die Reaktionsmischung mit Wasser verdünnt und mit Ethylacetat extrahiert. Die organischen Phasen wurden über Magnesiumsulfat getrocknet und eingeengt. Der Rückstand wurde an Kieselgel chromatographiert (Eluent: Heptan/Ethylacetat 1:2). Man erhielt so das Produkt mit dem Molekulargewicht 409,53 (C25H31NO4); MS (ESI): 410 (M+H+).

### Beispiel 8-1

### N-{(S)-6-[1-(2,2-Dimethyl-propylamino)-1-methyl-ethyl]-1,2,3,4-tetrahydro-naphthalen-2-yl}-4-[(S)-1-(tetrahydro-furan-2-yl)methoxy]-benzamide

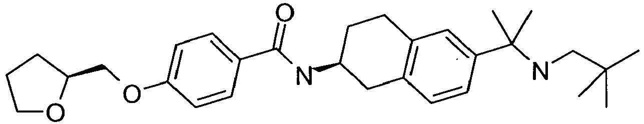

### Methode H-2

Zu einer Mischung aus N-[(S)-6-(1-Amino-1-methyl-ethyl)-1,2,3,4-tetrahydro-naphthalen-2-yl]-4-[(S)-1-(tetrahydro-furan-2-yl)methoxy]-benzamide (30 mg), THF (1 mL), Methanol (0,5 mL), Trimethylacetaldehyd (10 mg) und Essigsäure (9 mg) wurde polymer-gebundenes Natriumcyanoborhydrid (0,15 mmol) gegeben und die Suspension für 12 Stunden bei Raumtemperatur geschüttelt. Es wurde vom Polymer abgesaugt und das Filtrat eingeengt. Der Rückstand wurde durch präparative HPLC gereinigt. Man erhielt so das Produkt mit dem Molekulargewicht 478,68 (C30H42N2O3); MS (ESI): 479 (M+H+).

In der Tabelle 2 sind Beispiele zusammengefasst, die durch reduktive Alkylierung der entsprechenden Amine nach Methode H-2 mit den entsprechenden Carbonylverbindungen (Aldehyd bzw. Keton) erhalten wurden. Sollen nach Methode H-2 N,N-Dialkylierungen an primären Aminen erreicht werden, setzt man 2-3 Äquivalente der Carbonylkomponente und entsprechend mehr Natriumcyanoborhydrid ein.

**Tabelle 2.**

| Bsp. No. | Struktur | Molekular-gewicht | ESI-MS [M+H]+ |
|---|---|---|---|
| 8-2 | 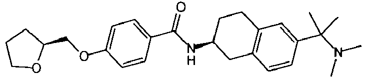 | 436,27 | 437 |
| 8-3 | 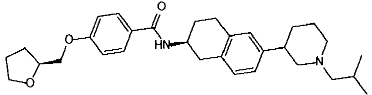 | 490,32 | 491 |
| 8-4 | 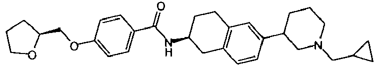 | 488,30 | 489 |
| 8-5 | 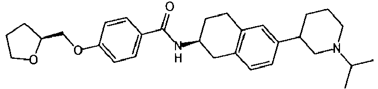 | 476,30 | 477 |
| 8-6 | 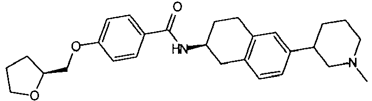 | 448,27 | 449 |
| 8-7 | 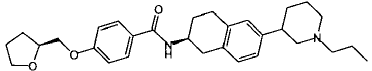 | 476,30 | 477 |
| 8-8 | 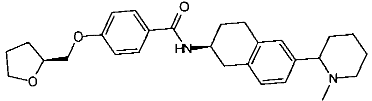 | 448,27 | 449 |
| 8-9 | 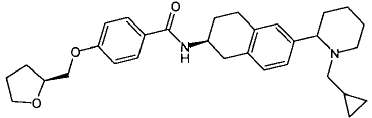 | 488,30 | 489 |
| 8-10 | 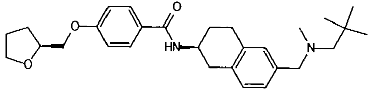 | 464,30 | 465 |
| 8-11 | 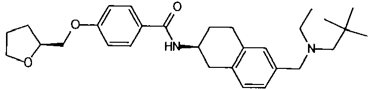 | 478,32 | 479 |
| 8-12 | 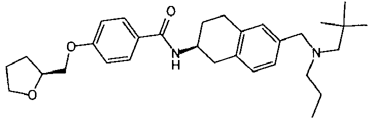 | 492,34 | 493 |
| 8-13 | 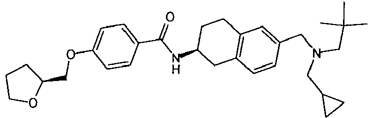 | 504,34 | 505 |
| 8-14 | 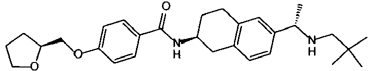 | 464,30 | 465 |
| 8-15 | 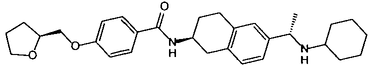 | 476,30 | 477 |
| 8-16 | 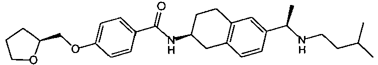 | 464,30 | 465 |
| 8-17 | 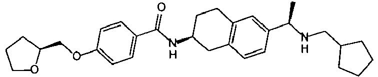 | 476,30 | 477 |
| 8-18 | 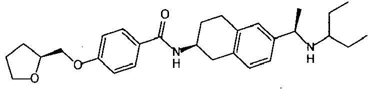 | 464,30 | 465 |
| 8-19 | 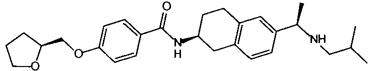 | 450,29 | 451 |
| 8-20 | 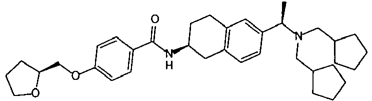 | 558,38 | 559 |
| 8-21 | 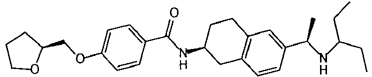 | 464,30 | 465 |
| | | | |
| 8-22 | 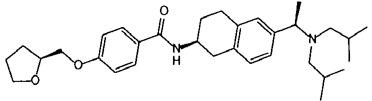 | 506,35 | 507 |
| 8-23 | 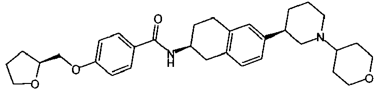 | 518,31 | 519 |
| 8-24 | 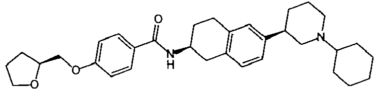 | 516,34 | 517 |
| 8-25 | 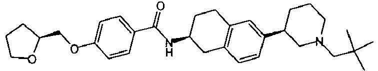 | 504,34 | 505 |
| 8-26 | 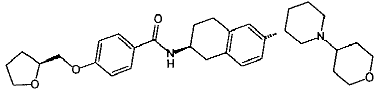 | 518,31 | 519 |
| 8-27 | 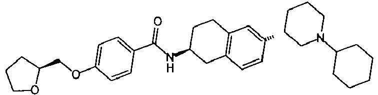 | 516,34 | 517 |
| 8-28 | 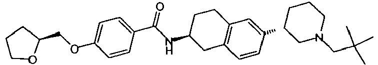 | 504,34 | 505 |

### Beispiel 9-1

### 5-[(S)-1-(Tetrahydro-furan-2-yl)methoxy]-pyridine-2-carboxylic acid (( S)-6-azepan-1-ylmethyl-1,2,3,4-tetrahydro-naphthalen-2-yl)-amide

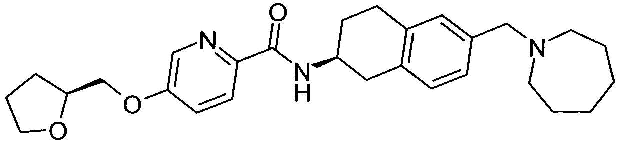

Nach Methode F wurde 5-Hydroxy-pyridine-2-carboxylic acid ((S)-6-azepan-1-ylmethyl-1,2,3,4-tetrahydro-naphthalen-2-yl)-amide mit Methanesulfonic acid (S)-1-(tetrahydro-furan-2-yl)methyl ester alkyliert (DMF, 12 h, 80°C). Man erhielt so das Produkt mit dem Molekulargewicht 463,63 (C28H37N3O3); MS (ESI): 464 (M+H+).

### 5-Hydroxy-pyridine-2-carboxylic acid ((S)-6-azepan-1-ylmethyl-1,2,3,4-tetrahydro-naphthalen-2-yl)-amide

### Methode A-2

Eine Mischung von 5-Hydroxy-pyridine-2-carboxylic acid (0,54 g) und DMF (3 mL) wurde mit (3-Dimethylamino-propyl)-ethyl-carbodiimide (EDC; 0,80 g) und Benzotriazol-1-ol (HOBt; 0,60 g) versetzt und für 5 Minuten gerührt. Dann wurden Ethyl-diisopropyl-amine (0,80 mL) und (S)-6-Azepan-1-ylmethyl-1,2,3,4-tetrahydro-naphthalen-2-ylamine (1,00 g) zugesetzt und die Mischung für 12 Stunden gerührt. Das Reaktionsgemisch wurde mit Wasser verdünnt und mit Ethylacetat extrahiert. Die organische Phase wurde über Natriumsulfat getrocknet, filtriert und eingeengt. Man erhielt so das Produkt mit dem Molekulargewicht 379,51 (C23H29N3O2); MS (ESI): 380 (M+H+).
Analog wurde N-((S)-6-Azepan-1-ylmethyl-1,2,3,4-tetrahydro-naphthalen-2-yl)-4-hydroxy-enzamide durch Umsetzung von 4-Hydroxy-benzoic acid mit (S)-6-Azepan-1-ylmethyl-1,2,3,4-tetrahydro-naphthalen-2-ylamine erhalten.

In der Tabelle 3 sind Beispiele zusammengefasst, die durch Alkylierung von N-((S)-6-Azepan-1-ylmethyl-1,2,3,4-tetrahydro-naphthalen-2-yl)-4-hydroxy-enzamide nach Methode F mit den entsprechenden Alkylierungsmitteln (z. B. Bromide, Iodide oder Sulfonsäureester) hergestellt wurden.

**Tabelle 3.**

| Bsp. No. | Struktur | Molekular-gewicht | ESI-MS [M+H]+ |
|---|---|---|---|
| 9-2 | 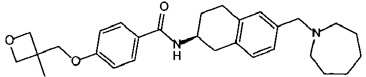 | 462,63 | 463 |
| 9-3 | 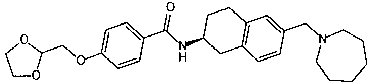 | 464,60 | 465 |
| 9-4 | 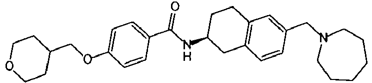 | 476,66 | 477 |
| 9-5 | 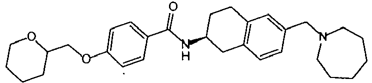 | 476,66 | 477 |
| 9-6 | 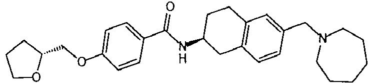 | 462,64 | 463 |

### Beispiel 10

### N-{(S)-6-[(R)-1-(4-Methyl-piperidin-1-yl)-ethyl]-1,2,3,4-tetrahydro-naphthalen-2-yl}-4-[(S)-1-(tetrahydro-furan-2-yl)methoxy]-benzamide

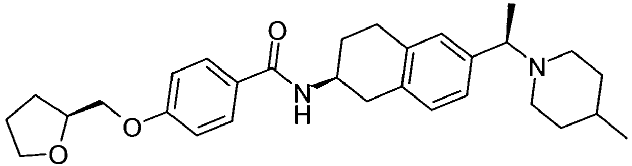

Eine Mischung aus N-[(S)-6-((R)-1-Amino-ethyl)-1,2,3,4-tetrahydro-naphthalen-2-yl]-4-[(S)-1-(tetrahydro-furan-2-yl)methoxy]-benzamide (50 mg), 1,5-Dibromo-3-methyl-pentane (31 mg), Ethyl-diisopropyl-amine (0,10 mL) und Acetonitril (1 mL) wurde für 8 Stunden auf 40 °C erhitzt. Die abgekühlte Reaktionsmischung wurde eingeengt und der Rückstand durch präparative HPLC gereinigt. Man erhielt so das Produkt mit dem Molekulargewicht 476,66 (C30H40N2O3); MS (ESI): 477 (M+H+).

### Beispiel 11

### N-[(S)-6-((R)-1-Azepan-1-yl-ethyl)-1,2,3,4-tetrahydro-naphthalen-2-yl]-4-[(S)-1-(tetrahydro-furan-2-yl)methoxy]-benzamide

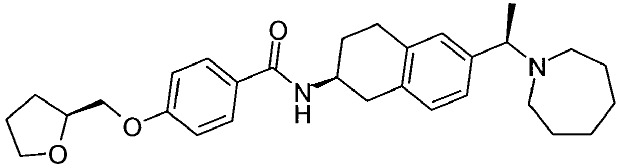

Eine Mischung aus N-[(S)-6-((R)-1-Amino-ethyl)-1,2,3,4-tetrahydro-naphthalen-2-yl]-4-[(S)-1-(tetrahydro-furan-2-yl)methoxy]-benzamide (50 mg), 1,6-Dibromo-hexane (31 mg), Ethyl-diisopropyl-amine (0,10 mL) und Acetonitril (1 mL) wurde für 8 Stunden auf 40 °C erhitzt. Die abgekühlte Reaktionsmischung wurde eingeengt und der Rückstand durch präparative HPLC gereinigt. Man erhielt so das Produkt mit dem Molekulargewicht 476,66 (C30H40N2O3); MS (ESI): 477 (M+H+).

### Beispiel 12

### N-((S)-6-Azepan-1-ylmethyl-1,2,3,4-tetrahydro-naphthalen-2-yl)-3-fluoro-4-[(S)-1-(tetrahydrofuran-2-yl)methoxy]-benzamide

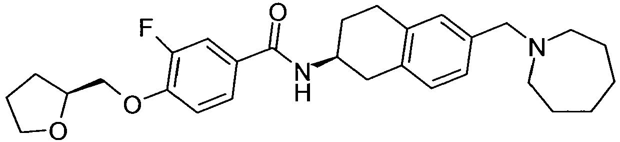

Nach Methode A-2 wurde 3-Fluoro-4-[(S)-1-(tetrahydro-furan-2-yl)methoxy]-benzoic acid mit (S)-6-Azepan-1-ylmethyl-1,2,3,4-tetrahydro-naphthalen-2-ylamine umgesetzt. Man erhielt so das Produkt mit dem Molekulargewicht 480,63 (C29H37FN2O3); MS (ESI): 481 (M+H+).

### 3-Fluoro-4-[(S)-1-(tetrahydro-furan-2-yl)methoxy]-benzoic acid

Nach Methode F wurde 3-Fluoro-4-hydroxy-benzoic acid ethyl ester mit Methanesulfonic acid (S)-1-(tetrahydro-furan-2-yl)methyl ester alkyliert und das Reaktionsprodukt nach Methode E verseift. Man erhielt so das Produkt mit dem Molekulargewicht 240,23 (C12H13FO4); MS (ESI): 241 (M+H+).

### Beispiel 13

### 2-[(S)-6-(4-Methoxy-piperidin-1-ylmethyl)-1,2,3,4-tetrahydro-naphthalen-2-yl]-6-[(S)-1-(tetrahydro-furan-2-yl)methoxy]-2H-isoquinolin-1-one

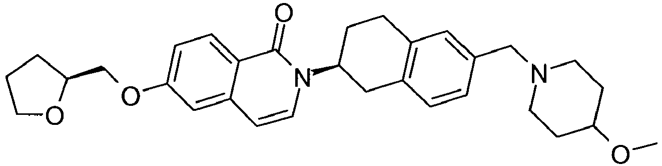

Eine Mischung aus 6-[(S)-1-(Tetrahydro-furan-2-yl)methoxy]-isochromen-1-one (50 mg), NMP (0,2 mL) und (S)-6-(4-Methoxy-piperidin-1-ylmethyl)-1,2,3,4-tetrahydro-naphthalen-2-ylamine (55 mg) wurde im Mikrowellenreaktor für 3 x 30 Minuten auf 220 °C erhitzt. Die abgekühlte Reaktionsmischung wurde durch präparative HPLC gereinigt. Man erhielt so das Produkt mit dem Molekulargewicht 502,66 (C31H38N2O4); MS (ESI): 503 (M+H+).

### 6-[(S)-1-(Tetrahydro-furan-2-yl)methoxy]-isochromen-1-one

Zu einer Lösung von 6-Hydroxy-isochromen-1-one (2 g) in DMF (50 mL) wurden Methanesulfonic acid (S)-1-(tetrahydro-furan-2-yl)methyl ester (2,7 g) und Cäsium carbonate (12 g) gegeben und die Mischung wurde für 7 Stunden bei 80 °C gerührt. Nach der Zugabe von Wasser wurde das Gemisch mit Dichlormethan extrahiert. Die organischen Phasen wurden über Magnesiumsulfat getrocknet und eingeengt. Man erhielt so das Produkt mit dem Molekulargewicht 246,27 (C14H14O4); MS (ESI): 247 (M+H+).

### 6-Hydroxy-isochromen-1-one

### Methode N

Zu einer Lösung von 6-Methoxy-isochromen-1-one (9,3 g) in Dichlormethan (300 mL) wurde bei 0 °C eine Lösung von Bortribromid (1 M in Dichlormethan, 130 mL) zugegeben und die Mischung wurde für 16 Stunden bei Raumtemperatur gerührt. Nach der Zugabe von Natriumcarbonatlösung wurde das Gemisch mit Ethylacetat gewaschen. Die wässrige Phase wurde mit 2 N HCl angesäuert und mit Ethylacetat extrahiert. Die organischen Phasen wurden über Magnesiumsulfat getrocknet und eingeengt. Der Rückstand wurde an Kieselgel chromatographiert. Man erhielt so das Produkt mit dem Molekulargewicht 162,15 (C9H6O3); MS (ESI): 163 (M+H+).

### 6-Methoxy-isochromen-1-one

Eine Lösung von 6-Methoxy-isochroman-1-one (15,1 g), N-Bromsuccinimid (NBS; 27 g) und Benzoylperoxid (500 mg) in Tetrachlormethan (250 mL) wurde unter Bestrahlung mit Licht für 3 Stunden zum Rückfluss erhitzt. Die Mischung wurde filtriert und das Filtrat wurde eingeengt. Der Rückstand wurde in Triethylamin (100 mL) gelöst und für 48 Stunden bei Raumtemperatur gerührt. Die Reaktionsmischung wurde zwischen Wasser und Ethylacetat verteilt und mit konzentrierter Salzsäure auf pH 1 gebracht. Die organische Phase wurde abgetrennt, über Magnesiumsulfat getrocknet und eingeengt. Der Rückstand wurde an Kieselgel chromatographiert. Man erhielt so das Produkt mit dem Molekulargewicht 176,17 (C10H8O3); MS (ESI): 177 (M+H+).

### 6-Methoxy-isochroman-1-one

Zu einer Lösung aus Diisopropylamin (33,5 mL) in trockenem THF (190 mL) wurde bei -78 °C n-Butyllithium (1,6 M Lösung in Hexan, 145,9 mL) zugetropft. Anschließend erwärmte man das Reaktionsgemisch für 5 Minuten auf Raumtemperatur und kühlte es dann erneut auf-78 °C ab und tropfte eine Lösung aus 4-Methoxy-2-methylbenzoesäure (10 g) in trockenem THF (210 mL) zu. Nach 10 Minuten Rühren bei dieser Temperatur wurde Paraformaldehyd (7 g) zugegeben. Dann ließ man die Reaktionsmischung auf Raumtemperatur kommen und rührte 4 Stunden bei dieser Temperatur. Das Reaktionsgemisch wurde mit Wasser versetzt, anschließend wurde das THF im Vakuum entfernt und dann die wässrige Phase mit Diethylether extrahiert. Die wässrige Phase wurde mit konz. HCl sauer gestellt, der erhaltene Niederschlag wurde abfiltriert und mit Wasser mehrfach gewaschen. Man erhielt so das Produkt mit dem Molekulargewicht 178,06 (C10H10O3); MS (ESI): 179 (M+H+).

### Beispiel 14

### 2-[(S)-6-(4-Methoxy-piperidin-1-ylmethyl)-1,2,3,4-tetrahydro-naphthalen-2-yl]-6-[(S)-1-(tetrahydro-furan-2-yl)methoxy]-3,4-dihydro-2H-isoquinolin-1-one

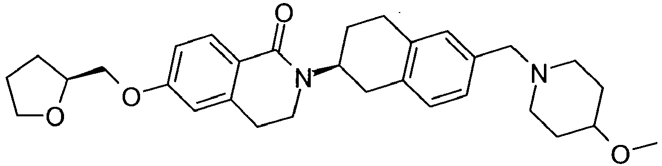

Nach Methode F wurde 6-Hydroxy-2-[(S)-6-(4-methoxy-piperidin-1-ylmethyl)-1,2,3,4-tetrahydro-naphthalen-2-yl]-3,4-dihydro-2H-isoquinolin-1-one mit Methanesulfonic acid (S)-1-(tetrahydro-furan-2-yl)methyl ester alkyliert. Man erhielt so das Produkt mit dem Molekulargewicht 504,68 (C31H40N2O4); MS (ESI): 505 (M+H+).

### 6-Hydroxy-2- [(S)-6-(4-methoxy-piperidin-1-ylmethyl)-1,2,3,4-tetrahydro-naphthalen-2-yl]-3,4-dihydro-2H-isoquinolin-1-one

Eine Mischung aus 6-Methoxy-2-[(S)-6-(4-methoxy-piperidin-1-ylmethyl)-1,2,3,4-tetrahydro-naphthalen-2-yl]-3,4-dihydro-2H-isoquinolin-1-one (0,59 g), NMP (2 mL), Thiophenol (150 mg) und Kaliumcarbonat (235 mg) wurde für 40 Minuten im Mikrowellenreaktor auf 210 °C erwärmt. Das abgekühlte Reaktionsgemisch wurde durch Chromatographie an Kieselgel (Eluent: Dichlormethan/Methanol 9:1) gereinigt. Man erhielt so das Produkt mit dem Molekulargewicht 420,56 (C26H32N2O3); MS (ESI): 421 (M+H+).

### 6-Methoxy-2-[(S)-6-(4-methoxy-piperidin-1-ylmethyl)-1,2,3,4-tetrahydro-naphthalen-2-yl]-3,4-dihydro-2H-isoquinolin-1-one

### Methode O

Eine Mischung aus 6-Methoxy-isochroman-1-one (1,30 g) und Thionylchlorid (0,87 g) wurde mit einem Tropfen DMF versetzt und für 5 Stunden zum Rückfluss erwärmt. Flüchtige Anteile wurden abdestilliert. Der Rückstand wurde in THF (2 mL) aufgenommen und zur einer auf 0 °C gekühlten Mischung aus (S)-6-(4-Methoxy-piperidin-1-ylmethyl)-1,2,3,4-tetrahydro-naphthalen-2-ylamine (2,00 g), THF (20 mL) und Triethylamin (1,0 mL) getropft. Nach 15 Minuten wurde Kalium-tert.-butoxid (0,82 g) zugesetzt und nach 30 Minuten das Kühlbad entfernt. Nach weiteren 12 Stunden bei Raumtemperatur wurde die Reaktionsmischung mit Wasser verdünnt und mit Ethylacetat extrahiert. Die organische Phase wurde über Natriumsulfat getrocknet und eingeengt. Der Rückstand wurde durch präparative HPLC gereinigt. Man erhielt so das Produkt mit dem Molekulargewicht 434,58 (C27H34N2O3); MS (ESI): 435 (M+H+).

### Beispiel 15-1

### 2-((S)-6-Pyrrolidin-1-ylmethyl-1,2,3,4-tetrahydro-naphthalen-2-yl)-6-[(S)-1-(tetrahydro-furan-2-yl)methoxy]-3,4-dihydro-2H-isoquinolin-1-one

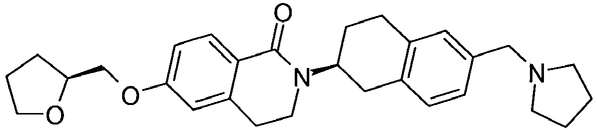

Nach Methode H-1 wurde (S)-6-{1-Oxo-6-[(S)-1-(tetrahydro-furan-2-yl)methoxy]-3,4-dihydro-1H-isoquinolin-2-yl}-5,6,7,8-tetrahydro-naphthalene-2-carbaldehyde mit Pyrrolidine umgesetzt. Man erhielt so das Produkt mit dem Molekulargewicht 460,62 (C29H36N2O3); MS (ESI): 461 (M+H+).

### (S)-6-{1-Oxo-6-[(S)-1-(tetrahydro-furan-2-yl)methoxy]-3,4-dihydro-1H-isoquinolin-2-yl}-5,6,7,8-tetrahydro-naphthalene-2-carbaldehyde

### Methode P

Eine Mischung aus 2-((S)-6-Bromo-1,2,3,4-tetrahydro-naphthalen-2-yl)-6-[(S)-1-(tetrahydro-furan-2-yl)methoxy]-3,4-dihydro-2H-isoquinolin-1-one (1,10 g), Palladium(II)-acetat (16,2 mg), Butyldi-1-adamantylphosphine (77 mg), TMEDA (0,27 mL) und Toluol (22 mL) wurde in einem Autoklaven unter Wasserstoff/Kohlenmonoxid-Atmosphäre für 14 Stunden auf 120°C erwärmt. Die abgekühlte Reaktionsmischung wurde mit Ethylacetat verdünnt und erst mit verdünnter Salzsäure und dann mit Natriumhydrogencarbonatlösung gewaschen. Die organische Phase wurde über Natriumsulfat getrocknet und eingeengt. Der Rückstand wurde durch präparative HPLC gereinigt. Man erhielt so das Produkt mit dem Molekulargewicht 405,50 (C25H27NO4); MS (ESI): 406 (M+H+).

### 2-((S)-6-Bromo-1,2,3,4-tetrahydro-naphthalen-2-yl)-6-[(S)-1-(tetrahydro-furan-2-yl)methoxy]-3,4-dihydro-2H-isoquinolin-1-one

Nach Methode O wurde 6-[(S)-1-(Tetrahydro-furan-2-yl)methoxy]-isochroman-1-one mit (S)-6-Bromo-1,2,3,4-tetrahydro-naphthalen-2-ylamine (J. Org. Chem. 1995, 60, 4324) umgesetzt. Man erhielt so das Produkt mit dem Molekulargewicht 456,38 (C24H26BrNO3); MS (ESI): 456 (M+H+).

### 6-[(S)-1-(Tetrahydro-furan-2-yl)methoxy]-isochroman-1-one

Eine Mischung aus 6-Hydroxy-isochroman-1-one (332 mg), Methanesulfonic acid (S)-1-(tetrahydro-furan-2-yl)methyl ester (284 mg), Cesium Carbonate (1,28 g) und DMF (8 mL) wurde für 7 Stunden auf 70 °C erwärmt. Die abgekühlte Reaktionsmischung wurde zwischen Wasser und Ethylacetat verteilt. Die organische Phase wurde getrocknet und eingeengt. Das Rohprodukt wurde durch Chromatographie an Kieselgel gereinigt. Man erhielt so das Produkt mit dem Molekulargewicht 248,28 (C14H16O4); MS (ESI): 249 (M+H+).

### 6-Hydroxy-isochroman-1-one

Nach Methode N wurde 6-Methoxy-isochroman-1-one mit Bortribromid behandelt.

In Tabelle 4 sind Beispiele zusammengefasst, die durch Umsetzung von (S)-6-{1-Oxo-6-[(S)-1-(tetrahydro-furan-2-yl)methoxy]-3,4-dihydro-1H-isoquinolin-2-yl}-5,6,7,8-tetrahydro-naphthalene-2-carbaldehyde mit Aminen nach Methode H-1 erhalten wurden.

**Tabelle 4.**

| Bsp. No. | Struktur | Molekulargewicht | ESI-MS [M+H]+ |
|---|---|---|---|
| 15-2 | 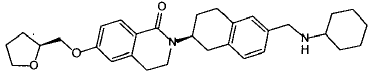 | 488,68 | 489 |
| 15-3 | 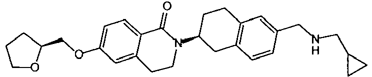 | 460,62 | 461 |
| 15-4 | 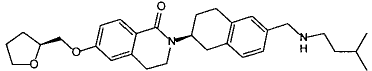 | 476,66 | 477 |
| 15-5 | 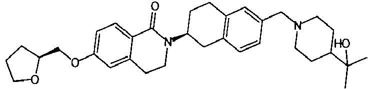 | 532,73 | 533 |
| 15-6 | 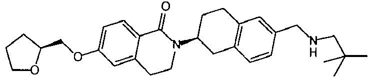 | 476,66 | 477 |
| 15-7 | 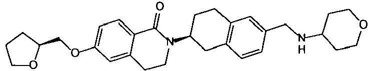 | 490,65 | 491 |
| 15-8 | 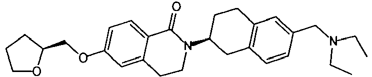 | 462,64 | 463 |
| 15-9 | 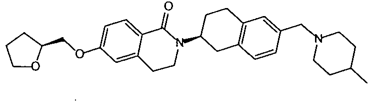 | 488,68 | 489 |
| 15-10 | 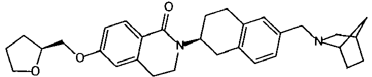 | 486,66 | 487 |
| 15-11 | 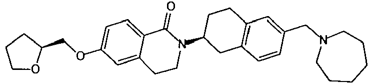 | 488,68 | 489 |
| 15-12 | 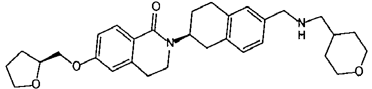 | 504,68 | 505 |
| 15-13 | 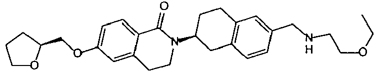 | 478,64 | 479 |
| 15-14 | 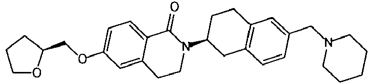 | 474,65 | 475 |
| 15-15 | 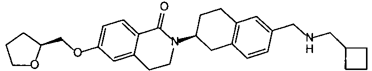 | 474,65 | 475 |
| 15-16 | 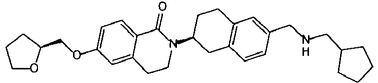 | 488,68 | 489 |
| 15-17 | 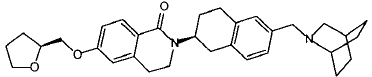 | 500,69 | 501 |

In der Tabelle 5 sind exemplarisch Ergebnisse zusammengefasst, die im oben beschriebenen Calcium-Mobilisierungsassay erhalten wurden.

**Tabelle 5.**

| Bsp. No. | IC50 / µM | Bsp. No. | IC50 / µM | Bsp. No. | IC50 / µM | Bsp. No. | IC50 / µM |
|---|---|---|---|---|---|---|---|
| 1 | 0,09 | 3-44 | 0,31 | 3-100 | 0,33 | 8-16 | 0,12 |
| 2 | 0,67 | 3-48 | 0,21 | 3-103 | 0,20 | 8-26 | 0,23 |
| 3-2 | 3,29 | 3-51 | 0,19 | 3-120 | 2,41 | 9-2 | 1,28 |
| 3-14 | 0,10 | 3-61 | 0,30 | 3-135 | 0,15 | 9-3 | 0,17 |
| 3-26 | 0,36 | 3-64 | 0,18 | 3-166 | 0,11 | 9-4 | 0,44 |
| 3-32 | 0,13 | 3-71 | 0,75 | 3-194 | 0,09 | 9-5 | 0,31 |
| 3-33 | 0,27 | 3-72 | 1,85 | 3-195 | 0,11 | 10 | 0,11 |
| 3-34 | 0,10 | 3-77 | 0,12 | 8-1 | 0,24 | 12 | 0,30 |
| 3-37 | 0,34 | 3-84 | 0,17 | 8-3 | 0,23 | 13 | 1,40 |
| 3-40 | 0,18 | 3-86 | 0,12 | 8-8 | 0,18 | 14 | 0,63 |

In der Tabelle 6 sind exemplarisch Ergebnisse zusammengefasst, die im oben beschriebenen hERG-Inhibitionsassay bzw. im Test auf kinetische Löslichkeit bestimmt wurden. Es zeigt sich, dass erfindungsgemässe Verbindungen vorteilhaft geringe hERG-Inbibtion mit einer hohen Löslichkeit in wässrigen Systemen bei einem physiologisch relevantem pH-Wert von 7,4 kombinieren. Dagegen zeigen Verbindungen des Standes der Technik häufig eine geringere Löslichkeit. So wurde z. B. für das Beispiel No. 110 ((S)-Enantiomer) aus der Schrift US2005/0075324 eine Löslichkeit von <10 µM gefunden. Auch beschreibt die Veröffentlichung in Bioorg. Med. Chem. Lett. 2007, 17, 814-818 (Tabellenunterschrift auf Seite 817) die Schwerlöslichkeit der Verbindungen des Standes der Technik.

**Tabelle 6.**

| Bsp. No. | hERG-Inh. IC50/µM | Neph. Lösl. pH 7,4 /µM |
|---|---|---|
| 1 | >30 | >500 |
| 3-6 | >10 | >500 |
| 3-34 | >30 | 50 |
| 3-83 | >30 | >500 |
| 3-154 | >10 | >500 |
| 3-166 | >10 | 450 |
| 14 | >10 | >500 |

In der Tabelle 7 sind Beispiele mit einem Aminochromangrundgerüst aufgeführt, die nach den oben exemplarisch beschriebenen Verfahren hergestellt werden können. Um z. B. nach dem im Schema 2 oder exemplarisch im Beispiel 3-1 beschriebenen Verfahren Verbindungen zu erhalten, kann man z. B. von 7-Bromo-chroman-3-one ausgehen, das kommerziell angeboten wird (Anichem LLC; 195 Black Horse Lane; North Brunswick, NJ, 08902; USA) oder auf folgendem Weg hergestellt wurde:

### 7-Bromo-chroman-3-one

Eine Mischung aus 4-Bromo-2-hydroxy-benzaldehyde (20 g), Acrylnitril (26,4 g) und DABCO (0,78 g) wurde für 8 Stunden am Rückfluss erhitzt. Nach dem Abkühlen wurde die Reaktionsmischung mit Ethylacetat verdünnt und mit Wasser gewaschen. Die organische Phase wurde eingeengt und der Rückstand über Kieselgel filtriert. Das so erhaltene 7-Bromo-2H-chromene-3-carbonitrile (7,5 g) wurde mit Natronlauge (10,2 g Natriumhydroxid in 100 mL Wasser) für 8 Stunden am Rückfluss gekocht. Die abgekühlte Reaktionsmischung wurde mit Methyl-tert.-butylether gewaschen, mit Salzsäure sauer gestellt und mit Ethylacetat extrahiert. Einengen der organischen Phase ergab 7-Bromo-2H-chromene-3-carboxylic acid. Diese Säure (3,0 g) wurde mit DPPA (3,2 g), Triethylamin (1,6 mL) und Toluol (30 mL) für 12 Stunden auf 85 °C erwärmt. Dann wurde Salzsäure (6 N) zugesetzt und für 2 Stunden zum Rückfluss erhitzt. Die abgekühlte Reaktionsmischung wurde mit Ethylacetat extrahiert und die organische Phase eingeengt. Man erhielt so das Produkt mit dem Molekulargewicht 227,06 (C9H7BrO2); MS (ESI): 227 (M+H+).

Ebenfalls offenbart sind die Beispiele der Tabelle 7, die zur Illustration dienen.

**Tabelle 7.**

| Bsp. No. | Struktur | Molekulargewicht | berech. [M+H]+ |
|---|---|---|---|
| I-1 | 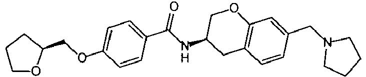 | 436,55 | 437 |
| I-2 | 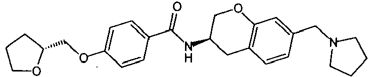 | 436,55 | 437 |
| I-3 | 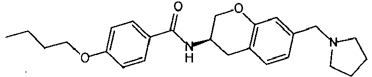 | 408,54 | 409 |
| I-4 | 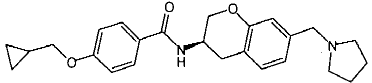 | 406,53 | 407 |
| I-5 | 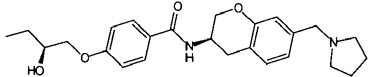 | 424,54 | 425 |
| I-6 | 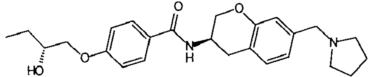 | 424,54 | 425 |
| I-7 | 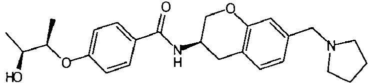 | 424,54 | 425 |
| I-8 | 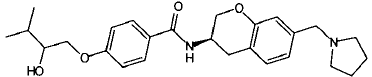 | 438,57 | 439 |
| I-9 | 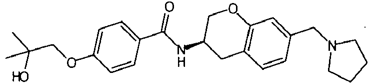 | 424,54 | 425 |
| I-10 | 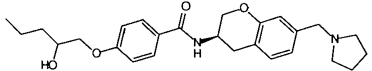 | 438,57 | 439 |
| I-11 | 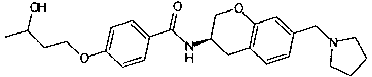 | 424,54 | 425 |
| I-12 | 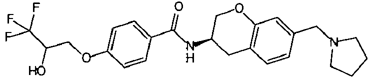 | 464,48 | 464 |
| I-13 | 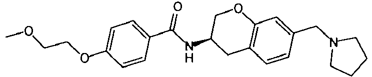 | 410,51 | 411 |
| I-14 | 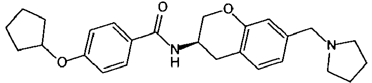 | 420,55 | 421 |
| I-15 | 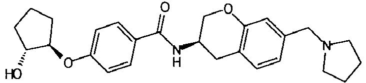 | 436,55 | 437 |
| I-16 | 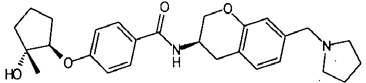 | 450,58 | 451 |
| I-17 | 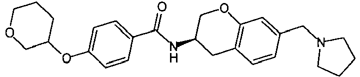 | 436,55 | 437 |
| I-18 | 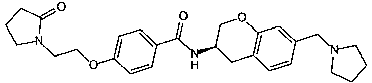 | 463,58 | 464 |
| I-19 | 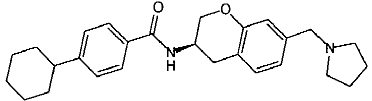 | 418,58 | 419 |
| I-20 | 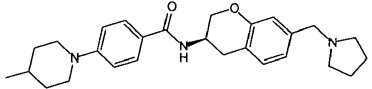 | 433,60 | 434 |
| I-21 | 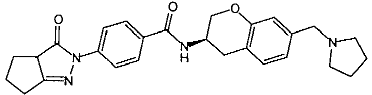 | 458,56 | 459 |
| I-22 | 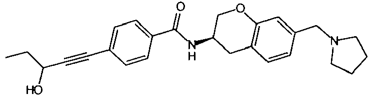 | 418,54 | 419 |
| I-23 | 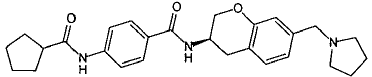 | 447,58 | 448 |
| I-24 | 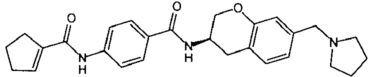 | 445,56 | 446 |
| I-25 | 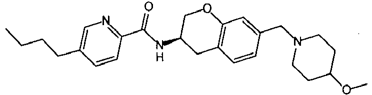 | 437,58 | 438 |
| I-26 | 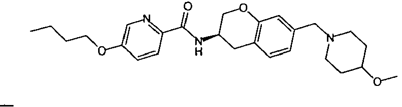 | 453,58 | 454 |
| | | | |
| I-27 | 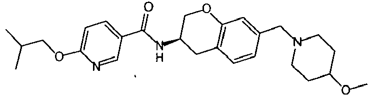 | 453,58 | 454 |
| I-28 | 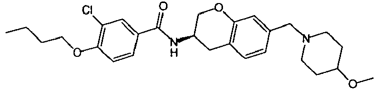 | 487,04 | 487 |
| I-29 | 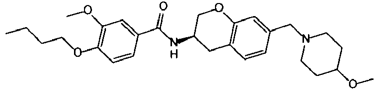 | 482,62 | 483 |
| I-30 | 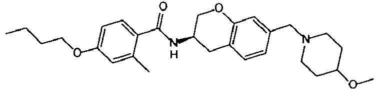 | 466,62 | 467 |
| I-31 | 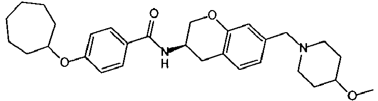 | 492,66 | 493 |
| I-32 | 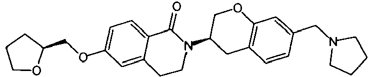 | 462,59 | 463 |

## Patentansprüche

1. Verbindungen der Formel I 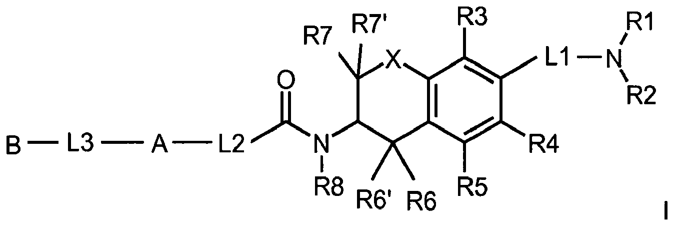 worin bedeuten
R1, R2 unabhängig voneinander H, (C₁-C₈)-Alkyl, (C₁-C₄)-Alkoxy-(C₁-C₄)-alkyl, (C₃-C₈)-Alkenyl, (C₃-C₈)-Alkinyl, CO(R9), (C(R10)(R11))_{q}-R12, CO(C(R13)(R14))ᵣ-R15, CO-O(C₁-C₈)-Alkyl, CO(C(R13)(R14))ᵣ-N(R16)(R17);
oder
R1 und R2 bilden zusammen mit dem Stickstoffatom, an das sie gebunden sind, einen 4 bis 10-gliedrigen mono-, bi- oder spirocyclischen Ring welcher außer dem Stickstoffatom 0 bis 3 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, CN, (C₁-C₆)-Alkyl, (C₃-C₈)-Cycloalkyl, O-(C₁-C₈)-Alkyl, (C₁-C₄)-Alkoxy-(C₁-C₄)-alkyl, Hydroxy-(C₁-C₆)-alkyl, Oxo, CO(R18), CON(R19)(R20), Hydroxy, COO(R21), N(R22)CO(C₁-C₆)-Alkyl, N(R23)(R24) oder SO₂(C₁-C₆)-Alkyl;
R10, R11 unabhängig voneinander H, (C₁-C₆)-Alkyl, Hydroxy-(C₁-C₂)-Alkyl, F, OH;
R9, R13, R14, R16, R17, R18, R19, R20, R21, R22, R23, R24, unabhängig voneinander H, (C₁-C₆)-Alkyl;
oder
R16 und R17, R23 und R24 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R12, R15 unabhängig voneinander H, OH, F, O-(C₁-C₆)-Alkyl, S-(C₁-C₆)-Alkyl, O-phenyl, CN, COO(R25), N(R26)CO(C₁-C₆)-Alkyl, N(R27)(R28), CON(R29) (R30), SO₂(C₁-C₆)-Alkyl, 3-12 gliedriger mono-, bi- oder spirocyclischer Ring, der ein bis vier Heteroatome aus der Gruppe N, O und S enthalten kann und der 3-12 gliedrige Ring weitere Substituenten wie F, Cl, Br, OH, CF₃, NO₂, CN, OCF₃, Oxo, O-(C₁-C₆)-Alkyl, (C₁-C₄)-Alkoxy-(C₁-C₄)-alkyl, S-(C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, N(R31)(R32), COO(R33), SO₂(C₁-C₆)-Alkyl und COOH enthalten kann;
R25, R26, R27, R28, R29, R30, R31, R32, R33 unabhängig voneinander H, (C₁-C₈)-Alkyl;
oder
R27 und R28, R29 und R30, R31 und R32 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
L1 C(R34)(R35), C(R36)(R37)C(R38)(R39), (C₃-C₆)-Cycloalkyl; optional kann R1 mit einem der Reste R34, R35, R36, R37, R38 oder R39 verbunden sein, so dass ein 5-6 gliedriger Ring entsteht;
R34, R35, R36, R37, R38, R39 unabhängig voneinander H, (C₁-C₈)-Alkyl;
R3, R4, R5 unabhängig voneinander H, F, Cl, Br, I, OH, CF₃, NO₂, CN, OCF₃, O-(C₁-C₆)-Alkyl, S-(C₁-C₆)-Alkyl, O-(C₁-C₄)-Alkoxy-(C₁-C₄)-alkyl, (C₁-C₆)-Alkyl, CON(R40)(R41), CO(R42);
R40, R41, R42 unabhängig voneinander H, (C₁-C₈)-Alkyl;
oder
R40 und R41 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
X C(R43)(R43');
R6, R6', R7, R7', R43, R43' unabhängig voneinander H, F, (C₁-C₈)-Alkyl, OH, O-(C₁-C₆)-Alkyl;
oder
R6 und R6', oder R43 und R43' zusammen optional Oxo;
R8 H, (C₁-C₈)-Alkyl;
L2 Bindung, C(R44)(R45);
R44, R45 unabhängig voneinander H, (C₁-C₈)-Alkyl;
A 5-bis 6-gliedriger aromatischer Ring, der bis zu 2 Heteroatome ausgewählt aus der Gruppe Stickstoff, Sauerstoff und Schwefel beinhalten kann, und substituiert sein kann mit einem oder mehreren der Substituenten H, F, Cl, Br, I, OH, CF₃, NO₂, CN, OCF₃, O-(C₁-C₆)-Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60);
im Fall L2 = Bindung kann C(O)NR8 mit einem ortho-ständigen Substituenten von A über eine Brücke enthaltend ein oder zwei Elemente aus der Gruppe Kohlenstoff und Stickstoff verbunden sein, so dass insgesamt ein 9 bis 10-gliedriger bicyclischer Ring entsteht;
R54, R55, R56, R57, R58, R59, R60 unabhängig voneinander H, (C₁-C₈)-Alkyl;
oder
R54 und R55, R56 und R57 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R61, R62, R63, R64 unabhängig voneinander H, (C₁-C₈)-Alkyl;
L3 C(R62)(R63)O, und
B ein 4- bis 10-gliedriger mono-, bi- oder spirocyclischer nicht-aromatischer Ring, welcher 1 bis 3 Heteroatome beinhaltet, ausgewählt aus der Gruppe Sauerstoff, Stickstoff und Schwefel, wobei das Ringsystem zusätzlich substituiert sein kann mit einem oder mehreren der folgenden Substituenten: F, CF₃, (C₁-C₆)-Alkyl, O-(C₁-C₈)-Alkyl, (C₁-C₄)-Alkoxy-(C₁-C₄)-alkyl, Hydroxy-(C₁-C₄)-alkyl, Oxo, CO(R64), Hydroxy;
bedeuten;
sowie deren physiologisch verträgliche Salze.

2. Verbindungen der Formel I gemäß Anspruch 1, worin
L2 Bindung bedeutet.

3. Verbindungen der Formel I gemäß Anspruch 1 oder 2, worin
R1, R2 R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, (C₁-C₆)-Alkyl, O-(C₁-C₈)-Alkyl, (C₃-C₈)-Cycloalkyl, (C₁-C₄)-Alkoxy-(C₁-C₄)-alkyl, Hydroxy-(C₁-C₆)-alkyl, Oxo, CO(R18), Hydroxy, N(R22)CO(C₁-C₆)-Alkyl, oder SO₂(C₁-C₆)-Alkyl;
bedeuten.

4. Verbindungen der Formel I gemäß Ansprüchen 1 bis 3, worin
A ausgewählt wird aus der Gruppe 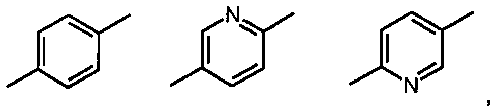 worin A die in Anspruch 1 genannten Substituenten tragen kann.

5. Verbindungen der Formel I gemäß Ansprüchen 1 bis 4, worin
B ein 4- bis 6-gliedriger nicht-aromatischer Ring, welcher 1 bis 2 Sauerstoffatome beinhaltet, wobei das Ringsystem zusätzlich substituiert sein kann mit einem oder mehreren der folgenden Substituenten: (C₁-C₆)-Alkyl oder Hydroxy; bedeutet.

6. Verbindungen der Formel I gemäß Ansprüchen 1 bis 5, worin
das kombinierte Element B-L3 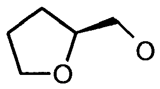 bedeutet.

7. Verbindungen gemäß Anspruch 1, **gekennzeichnet durch** die Formel II 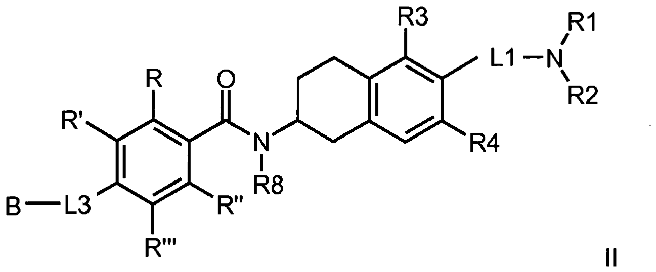 worin die Variablen R1, R2, L1, R3, R4 und R8 die in Anspruch 1 angegebenen Bedeutungen haben und
R, R', R", R"' unabhängig voneinander H, F, Cl, Br, I, OH, CF₃, NO₂, CN, OCF₃, O-(C₁-C₆)-Alkyl, O-(C₁-C₄)-Alkoxy-(C₁-C₄)-alkyl, (C₁-C₆)-Alkyl, N(R54)(R55), SO₂-CH₃, CON(R56)(R57), N(R58)CO(R59), CO(R60);
L3 CH₂O;
B 4- bis 6-gliedriger nicht-aromatischer Ring, welcher 1 bis 2 Sauerstoffatome beinhaltet, wobei das Ringsystem zusätzlich substituiert sein kann mit einem oder mehreren der folgenden Substituenten: F, (C₁-C₆)-Alkyl, O-(C₁-C₈)-Alkyl, (C₁-C₄)-Alkoxy-(C₁-C₄)-alkyl, Oxo, Hydroxy bevorzugt (C₁-C₆)-Alkyl oder Hydroxy;
bedeuten.

8. Verbindungen der Formel II, gemäß Anspruch 7, worin
das kombinierte Element B-L3 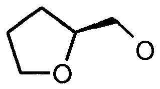 bedeutet.

9. Verbindungen gemäß Anspruch 1, **gekennzeichnet durch** die Formel IV 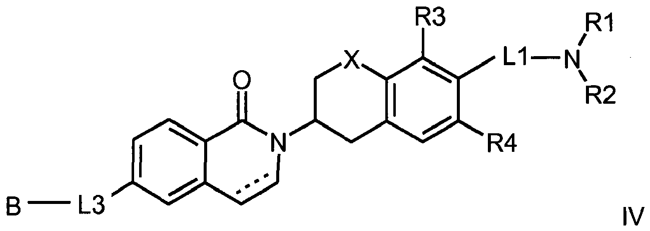 worin R1, R2, R3, R4, X, L1, L3 und B die für Formel I in Anspruch 1 angegebenen Bedeutungen haben, worin die unterbrochene Linie eine optionale Doppelbindung anzeigt, so dass sowohl Dihydroisochinolinone als auch Isochinolinone von der Formel IV umfasst werden.

10. Arzneimittel enthaltend eine oder mehrere der Verbindungen der Formel I gemäß einem oder mehreren der Ansprüche 1 bis 9.

11. Arzneimittel enthaltend eine oder mehrere der Verbindungen der Formel I gemäß einem oder mehreren der Ansprüche 1 bis 9 und einen oder mehrere Wirkstoffe, die günstige Wirkungen auf Stoffwechselstörungen oder damit assoziierte Erkrankungen haben.

12. Arzneimittel enthaltend eine oder mehrere der Verbindungen der Formel I gemäß einem oder mehreren der Ansprüche 1 bis 9 und ein oder mehrere Antidiabetika.

13. Arzneimittel enthaltend eine oder mehrere der Verbindungen der Formel I gemäß einem oder mehreren der Ansprüche 1 bis 9 und einen oder mehrere Lipidmodulatoren.

14. Arzneimittel enthaltend eine oder mehrere der Verbindungen der Formel I gemäß einem oder mehreren der Ansprüche 1 bis 9 und einen oder mehrere Antiadiposita.

15. Verbindungen der Formel I gemäß einem oder mehreren der Ansprüche 1 bis 9 zur Verwendung in der Behandlung und/oder Prävention von Störungen des Fettsäurestoffwechsels und Glucoseverwertungsstörungen.

16. Verbindungen der Formel I gemäß einem oder mehreren der Ansprüche 1 bis 9 zur Verwendung in der Behandlung und/oder Prävention von Störungen, bei denen Insulinresistenz eine Rolle spielt.

17. Verbindungen der Formel I gemäß einem oder mehreren der Ansprüche 1 bis 9 zur Verwendung in der Behandlung und/oder Prävention von Diabetes mellitus und der damit verbundenen Folgeerkrankungen.

18. Verbindungen der Formel I gemäß einem oder mehreren der Ansprüche 1 bis 9 zur Verwendung in der Behandlung und/oder Prävention von Dyslipidämien und deren Folgen.

19. Verbindungen der Formel I gemäß einem oder mehreren der Ansprüche 1 bis 9 zur Verwendung in der Behandlung und/oder Prävention von nichtalkoholischer Fettleber und ihren Verlaufsformen.

20. Verbindungen der Formel I gemäß einem oder mehreren der Ansprüche 1 bis 9 zur Verwendung in der Behandlung und/oder Prävention von Zuständen, die mit dem Metabolischen Syndrom assoziert sind.

21. Verbindungen der Formel I gemäß einem oder mehreren der Ansprüche 1 bis 9 zur Verwendung in der Behandlung und/oder Prävention von Obesitas und damit verbundenen Folgeerkrankungen.

22. Verbindungen gemäß einem oder mehreren der Ansprüche 1 bis 9 in Kombination mit mindestens einem weiteren Wirkstoff zur Verwendung in der Behandlung und/oder Prävention von Störungen des Fettsäurestoffwechsels und Glucoseverwertungsstörungen.

23. Verbindungen gemäß einem oder mehreren der Ansprüche 1 bis 9 in Kombination mit mindestens einem weiteren Wirkstoff zur Verwendung in der Behandlung und/oder Prävention von Störungen, bei denen Insulinresistenz eine Rolle spielt.

24. Verfahren zur Herstellung eines Arzneimittels enthaltend eine oder mehrere der Verbindungen gemäß einem oder mehreren der Ansprüche 1 bis 9, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. Compounds of formula I 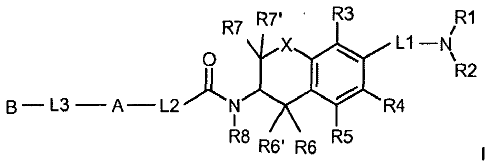 wherein:
R1 and R2 are each independently H, (C₁-C₈)-alkyl, (C₁-C₄)-alkoxy-(C₁-C₄)-alkyl, (C₃-C₈)-alkenyl, (C₃-C₈)-alkynyl, CO(R9), (C(R10)(R11))_{q}-R12, CO(C(R13)(R14))ᵣ-R15, CO-O(C₁-C₈)-alkyl, CO(C(R13)(R14))ᵣ-N(R16)(R17);
or
R1 and R2, taken together with the nitrogen atom to which they are bonded, form a 4- to 10-membered, mono-, bi- or spirocyclic ring which, apart from the nitrogen atom, may contain 0 to 3 additional heteroatoms selected from the group consisting of oxygen, nitrogen and sulphur, wherein the heterocyclic ring system may also be substituted by F, Cl, Br, CF₃, CN, (C₁-C₆)-alkyl, (C₃-C₈)-cycloalkyl, O-(C₁-C₈)-alkyl, (C₁-C₄)-alkoxy-(C₁-C₄)-alkyl, hydroxy-(C₁-C₆)-alkyl, oxo, CO(R18), CON(R19)(R20), hydroxyl, COO(R21), N(R22)CO(C₁-C₆)-alkyl, N(R23)(R24) or SO₂(C₁-C₆)-alkyl;
R10 and R11 are each independently H, (C₁-C₆)-alkyl, hydroxy-(C₁-C₂)-alkyl, F, and OH; R9, R13, R14, R16, R17, R18, R19, R20, R21, R22, R23, R24 are each independently H, (C₁-C₆)-alkyl;
Or
R16 and R17, R23 and R24, optionally taken together with the nitrogen atom to which they are bonded, form a 5-6-membered ring which, apart from the nitrogen atom, may also contain 0-1 further heteroatom from the group consisting of NH, N-(C₁-C₆)-alkyl, oxygen and sulphur;
q and r are each independently 0, 1, 2, 3, 4, 5, 6;
R12 and R15 are each independently H, OH, F, O-(C₁-C₆)-alkyl, S-(C₁-C₆)-alkyl, O-phenyl, CN, COO(R25), N(R26)CO(C₁-C₆)-alkyl, N(R27)(R28), CON(R29)(R30), SO₂(C₁-C₆)-alkyl, 3-12-membered mono-, bi- or spirocyclic ring, which may contain one to four heteroatoms from the group consisting of N, O and S, and the 3-12-membered ring may contain further substituents such as F, Cl, Br, OH, CF₃, NO₂, CN, OCF₃, oxo, O-(C₁-C₆)-alkyl, (C₁-C₄)-alkoxy-(C₁-C₄)-alkyl, S-(C₁-C₆)-alkyl, (C₁-C₆)-alkyl, (C₂-C₆)-alkenyl, (C₃-C₈)-cycloalkyl, O-(C₃-C₈)-cycloalkyl, (C₃-C₈)-cycloalkenyl, O-(C₃-C₈)-cycloalkenyl, (C₂-C₆)-alkynyl, N(R31)(R32), COO(R33), SO₂(C₁-C₆)-alkyl and COOH;
R25, R26, R27, R28, R29, R30, R31, R32 and R33 are each independently H, (C₁-C₈)-alkyl;
or
R27 and R28, R29 and R30, and R31 and R32, optionally taken together with the nitrogen atom to which they are bonded, each independently form a 5-6-membered ring which, apart from the nitrogen atom, may also contain 0-1 further heteroatom from the group consisting of NH, N-(C₁-C₆)-alkyl, oxygen and sulphur;
L1 is C(R34)(R35), C(R36)(R37)C(R38)(R39), or (C₃-C₆)-cycloalkyl; optionally, R1 may be bonded to one of R34, R35, R36, R37, R38 or R39 radicals, so as to form a 5-6-membered ring;
R34, R35, R36, R37, R38 and R39 are each independently H, (C₁-C₈)-alkyl;
R3, R4, and R5 are each independently H, F, Cl, Br, I, OH, CF₃, NO₂, CN, OCF₃, O-(C₁-C₆)-alkyl, S-(C₁-C₆)-alkyl, O-(C₁-C₄)-alkoxy-(C₁-C₄)-alkyl, (C₁-C₆)-alkyl, CON(R40)(R41), CO(R42);
R40, R41 and R42 are each independently H, (C₁-C₈)-alkyl;
or
R40 and R41, optionally taken together with the nitrogen atom to which they are bonded, each independently form a 5-6-membered ring which, apart from the nitrogen atom, may also contain 0-1 further heteroatom from the group consisting of NH, N-(C₁-C₆)-alkyl, oxygen, and sulphur;
X is C(R43)(R43');
R6, R6', R7, R7', R43 and R43' are each independently H, F, (C₁-C₈)-alkyl, OH, O-(C₁-C₆)-alkyl;
or
R6 and R6', or R43 and R43' taken together are optionally oxo;
R8 is H, (C₁-C₈)-alkyl;
L2 is a bond, C(R44)(R45);
R44 and R45 are each independently H, (C₁-C₈)-alkyl;
A is a 5- to 6-membered aromatic ring that may contain up to 2 heteroatoms selected from the group consisting of nitrogen, oxygen and sulphur, and may be substituted by one or more of the substituents selected from H, F, Cl, Br, I, OH, CF₃, NO₂, CN, OCF₃, O-(C₁-C₆)-alkyl, O-(C₁-C₄)-alkoxy-(C₁-C₄)-alkyl, (C₁-C₆)-alkyl, N(R54)(R55), SO₂-CH₃, CON(R56)(R57), N(R58)CO(R59), CO(R60);
when L2 is a bond, then C(O)NR8 may be bonded to an ortho substituent of A via a bridge containing one or two elements from the group consisting of carbon and nitrogen, so as to form a 9- to 10-membered bicyclic ring overall;
R54, R55, R56, R57, R58, R59, and R60 are each independently H, (C₁-C₈)-alkyl;
or
R54 and R55, and R56 and R57, optionally taken together with the nitrogen atom to which they are bonded, each independently form a 5-6-membered ring which, apart from the nitrogen atom, may also contain 0-1 further heteroatom from the group consisting of NH, N-(C₁-C₆)-alkyl, oxygen and sulphur;
R61, R62, R63, and R64 are each independently H, (C₁-C₈)-alkyl;
L3 is C(R62)(R63)O, and
B is a 4- to 10-membered mono-, bi- or spirocyclic nonaromatic ring which contains from 1 to 3 heteroatoms selected from the group consisting of oxygen, nitrogen and sulphur, where the ring system may additionally be substituted by one or more of the following substituents F, CF₃, (C₁-C₆)-alkyl, O-(C₁-C₈)-alkyl, (C₁-C₄)-alkoxy-(C₁-C₄)-alkyl, hydroxy-(C₁-C₄)-alkyl, oxo, CO(R64), and hydroxyl;
or a physiologically compatible salt thereof.

2. Compounds of formula I according to claim 1, wherein L2 is a bond.

3. Compounds of formula I according to either claim 1 or claim 2, wherein
R1 and R2, taken together with the nitrogen atom to which they are bonded, form a 4- to 10-membered mono-, bi- or spirocyclic ring which, apart from the nitrogen atom, may contain 0 to 2 additional heteroatoms, selected from the group consisting of oxygen, nitrogen and sulphur, wherein the heterocyclic ring system may also be substituted by F, Cl, Br, I, CF₃, (C₁-C₆)-alkyl, O-(C₁-C₈)-alkyl, (C₃-C₈)-cycloalkyl, (C₁-C₄)-alkoxy-(C₁-C₄)-alkyl, hydroxy-(C₁-C₆)-alkyl, oxo, CO(R18), hydroxyl, N(R22)CO(C₁-C₆)-alkyl, or SO₂(C₁-C₆)-alkyl.

4. Compounds of formula I according to claims 1 to 3, wherein:
A is selected from the group 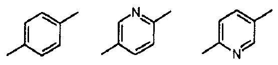
wherein A can carry the substituents referred to in claim 1.

5. Compounds of formula I according to claims 1 to 4, wherein
B is a 4- to 6-membered nonaromatic ring which contains 1 to 2 oxygen atoms, wherein the ring system may be additionally substituted by one or more of the following substituents: (C₁-C₆)-alkyl or hydroxyl.

6. Compounds of formula I according to claims 1 to 5, wherein:
the combined element B-L3 is

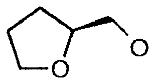

7. Compounds according to claim 1, **characterised by** formula II 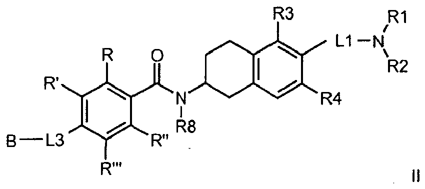 wherein the variables R1, R2, L1, R3, R4 and R8 are defined as in claim 1 and
R, R', R" and R'" are each independently H, F, Cl, Br, I, OH, CF₃, NO₂, CN, OCF₃, O-(C₁-C₆)-alkyl, O-(C₁-C₄)-alkoxy-(C₁-C₄)-alkyl, (C₁-C₆)-alkyl, N(R54)(R55), SO₂-CH₃, CON(R56)(R57), N(R58)CO(R59), and CO(R60);
L3 is CH₂O; and
B is a 4- to 6-membered nonaromatic ring which contains from 1 to 2 oxygen atoms, wherein the ring system may be additionally substituted by one or more of the following substituents: F, (C₁-C₆)-alkyl, O-(C₁-C₈)-alkyl, (C₁-C₄)-alkoxy-(C₁-C₄)-alkyl, oxo, hydroxyl, preferably (C₁-C₆)-alkyl or hydroxyl.

8. Compounds of formula II according to claim 7, wherein
the combined element B-L3 is

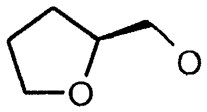

9. Compounds according to claim 1, **characterised by** formula IV 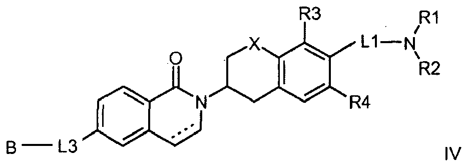 wherein R1, R2, R3, R4, R8, X, L1, L3 and B are each defined as for formula In claim 1,
wherein the broken line indicates an optional double bond so that both dihydroisoquinolinone and isoquinolinone are included by formula IV.

10. Pharmaceutical preparation containing one or more of the compounds of formula I according to one or more of claims 1 to 9.

11. Pharmaceutical preparation containing one or more of the compounds of formula I according to one or more of claims 1 to 9 and one or more active ingredients which have positive effects on metabolic disturbances or diseases associated therewith.

12. Pharmaceutical preparation containing one or more of the compounds of formula I according to one or more of claims 1 to 9 and one or more antidiabetics.

13. Pharmaceutical preparation containing one or more of the compounds of formula I according to one or more of claims 1 to 9 and one or more lipid modulators.

14. Pharmaceutical preparation containing one or more of the compounds of formula I according to one or more of claims 1 to 9 and one or more anti-obesity agents.

15. Compounds of formula I according to one or more of claims 1 to 9 for use in the treatment and/or prevention of disorders relating to fatty acid metabolism and glucose utilisation disorders.

16. Compounds of formula I according to one or more of claims 1 to 9 for use in the treatment and/or prevention of disorders in which insulin resistance plays a role.

17. Compounds of formula I according to one or more of claims 1 to 9 for use in the treatment and/or prevention of diabetes mellitus or secondary diseases associated therewith.

18. Compounds of formula I according to one or more of claims 1 to 9 for use in the treatment and/or prevention of dyslipidemia and the consequences thereof.

19. Compounds of formula I according to one or more of claims 1 to 9 for use in the treatment and/or prevention of nonalcoholic fatty liver and forms thereof.

20. Compounds of formula I according to one or more of claims 1 to 9 for use in the treatment and/or prevention of states associated with metabolic syndrome.

21. Compounds of formula I according to one or more of claims 1 to 9 for use in the treatment and/or prevention of obesity and secondary diseases associated therewith.

22. Compounds according to one or more of claims 1 to 9 in combination with at least one further active ingredient for use in the treatment and/or prevention of disorders relating to fatty acid metabolism and glucose utilisation disorders.

23. Compounds according to one or more of claims 1 to 9 in combination with at least one further active ingredient for use in the treatment and/or prevention of disorders in which insulin resistance plays a role.

24. Method for preparing a pharmaceutical preparation containing one or more of the compounds according to one or more of claims 1 to 9, **characterised in that** the active ingredient is mixed with a pharmaceutically suitable carrier and this mixture is made into a form which is suitable to be administered.

## Revendications

1. Composés de formule I : 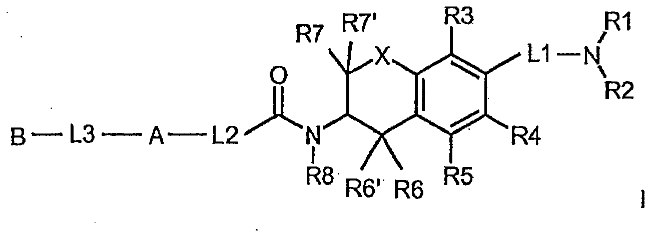 où
R1, R2 représentent indépendamment l'un de l'autre, H, alkyle (C₁-C₈), alcoxy (C₁-C₄)-alkyle (C₁-C₄), alcényle (C₃-C₈), alcinyle (C₃-C₈), CO(R9), (C(R10) (R11))_{q}-R12, CO(C(R13)(R14))ᵣ-R15, CO-O-alkyl (C₁-C₈), CO(C(R13)(R14))ᵣ-N(R16)(R17) ;
ou
R1 et R2 forment avec l'atome d'azote sur lequel ils sont liés, un cycle mono, bi ou spirocyclique ayant 4 à 10 membres, qui peut contenir en plus de l'atome d'azote, 0 à 3 autres hétéroatomes, choisis parmi le groupe oxygène, azote et soufre, où le système hétérocyclique peut en outre, être substitué par F, Cl, Br, CF₃, CN, alkyle (C₁-C₆), cycloalkyle (C₃-C₈), O-alkyle (C₁-C₈), alcoxy (C₁-C₄) -alkyle (C₁-C₄), hydroxyalkyle (C₁-C₆), oxo, CO(R18), CON(R19)(R20), hydroxy, COO(R21), N(R22)CO-alkyle (C₁-C₆), N(R23)(R24) ou SO₂-alkyle (C₁-C₆) ;
R10, R11 représentent indépendamment l'un de l'autre, H, alkyle (C₁-C₆), hydroxyalkyle (C₁-C₂), F, OH ;
R9, R13, R14, R16, R17, R18, R19, R20, R21, R22, R23, R24 représentent chacun indépendamment l'un de l'autre, H, alkyle (C₁-C₆) ;
ou
R16 et R17, R23 et R24 forment le cas échéant, avec l'atome d'azote sur lequel ils sont liés, un cycle ayant 5-6 membres, qui peut contenir en plus de l'atome d'azote, 0 ou 1 autre hétéroatome choisi parmi le groupe NH, N-alkyle (C₁-C₆), oxygène, et soufre ;
q, r représentent indépendamment l'un de l'autre, 0, 1, 2, 3, 4, 5, 6 ;
R12, R15 représentent indépendamment l'un de l'autre, H, OH, F, O-alkyle (C₁-C₆), S-alkyle (C₁-C₆), O-phényl, CN, COO(R25), N(R26)CO-alkyle (C₁-C₆), N(R27)(R28), CON(R29)(R30), SO₂-alkyle (C₁-C₆), système mono-, bi- ou spirocyclique ayant 3 à 12 membres, qui peut contenir un à quatre hétéroatomes choisis parmi N, O et S et le cycle de 3-12 membres peut présenter d'autres substituants comme F, Cl, Br, OH, CF₃, NO₂, CN, OCF₃ oxo, O-alkyle (C₁-C₆), alcoxy (C₁-C₄)-alkyle (C₁-C₄), S-alkyl (C₁-C₆), alkyle (C₁-C₆), alcényle (C₂-C₆), cycloalkyle (C₃-C₈), O-cycloalkyle (C₃-C₈), cycloalcényle (C₃-C₈), O-cycloalcényle (C₃-C₈), alcinyle (C₂-C₆), N(R31)(R32), COO(R33), SO₂-alkyle (C₁-C₆) et COOH ;
R25, R26, R27, R28, R29, R30, R31, R32, R33 représentent indépendamment l'un de l'autre, H, alkyle (C₁-C₈) ;
ou
R27 et R28, R29 et R30, R31 et R32 forment indépendamment l'un de l'autre, le cas échéant avec l'atome d'azote sur lequel ils sont liés, un cycle ayant 5-6 membres, qui peut contenir en plus de l'atome d'azote, 0 ou 1 autre hétéroatome choisi parmi le groupe NH, N-alkyle (C₁-C₆), oxygène et soufre ;
L1 représente C(R34)(R35), C(R36)(R37)C(R38)(R39), cycloalkyle (C₃-C₆) ;
le cas échéant, R1 peut être relié à un des restes R34, R35, R36, R37, R38 ou R39, pour former un cycle ayant 5-6 membres ;
R34, R35, R36, R37, R38, R39 représentent indépendamment l'un de l'autre, H, alkyle (C₁-C₈) ;
R3, R4, R5 représentent indépendamment l'un de l'autre, H, F, Cl, Br, I, OH, CF₃, NO₂, CN, OCF₃, O-alkyle (C₁-C₆), S-alkyle (C₁-C₆), O-alcoxy (C₁-C₄)-alkyle (C₁-C4), alkyle (C₁-C₆), CON(R40)(R41), CO(R42) ;
R40, R41, R42 représentent indépendamment l'un de l'autre, H, alkyle (C₁-C₈) ;
ou
R40 et R41 forment le cas échéant avec l'atome d'azote sur lequel ils sont liés, un cycle ayant 5-6 membres, qui peut contenir en plus de l'atome d'azote, 0 ou 1 autre hétéroatome choisi parmi le groupe NH, N-alkyle (C₁-C₆), oxygène et soufre ;
X représente C(43)(R43') ;
R6, R6', R7, R7', R43, R43' représentent indépendamment l'un de l'autre, H, F, alkyle (C₁-C₈), OH, O-alkyle (C₁-C₆) ;
ou
R6 et R6', ou R43 et R43' représentent ensemble, oxo ;
R8 représente H, alkyle (C₁-C₈) ;
L2 représente une liaison, C(R44)(R45) ;
R44, R45 représentent indépendamment l'un de l'autre, H, alkyle (C₁-C₈) ;
A représente un cycle aromatique ayant 5 à 6 membres, qui peut contenir jusqu'à 2 hétéroatomes choisis parmi le groupe azote, oxygène et soufre, et qui peut être substitué par un ou plusieurs des substituants H, F, Cl, Br, I, OH, CF₃, NO₂, CN, OCF₃, O-alkyle (C₁-C₈), O-alcoxy (C₁-C₄)-alkyle (C₁-C₄), alkyle (C₁-C₆), N(R54)(R55), SO₂-CH₃, CON(R56)(R57), N(R58)CO(R59), CO(R60) ;
au cas où L2 est une liaison =, C(O)NR8 peut être relié à un substituant en position ortho de A par un pont contenant un ou deux éléments du groupe carbone et azote, de sorte que l'on forme un système bicyclique ayant au total, 9 à 10 membres ;
R54, R55, R56, R57, R58, R59, R60 représentent indépendamment l'un de l'autre, H, alkyle (C₁-C₈) ;
ou
R54 et R55, R56 et R57 forment indépendamment l'un de l'autre, le cas échéant avec l'atome d'azote sur lequel ils sont liés, un cycle ayant 5-6 membres, qui peut contenir en plus de l'atome d'azote, 0 ou 1 autre hétéroatome choisi parmi le groupe NH, N-alkyle (C₁-C₆), oxygène et soufre ;
R61, R62, R63, R64 représentent indépendamment l'un de l'autre, H, alkyle (C₁-C₈) ;
L3 représente C(R62)(R63)O, et
B représente un système non aromatique mono-, bi- ou spirocyclique ayant 4 à 10 membres, qui contient 1 à 3 hétéroatomes choisis parmi le groupe oxygène, azote et soufre, où le système cyclique peut en outre, être substitué par un ou plusieurs des substituants suivants : F, CF₃, alkyle (C₁-C₆), O-alkyle (C₁-C₈), alcoxy (C₁-C₄)-alkyle (C₁-C₄), hydroxyalkyle (C₁-C₄), oxo, CO(R64), hydroxy ;
ainsi que leurs sels physiologiquement compatibles.

2. Composés de formule I selon la revendication 1, où L2 représente une liaison.

3. Composés de formule I selon la revendication 1 ou 2, où
R1 et R2 forment avec l'atome d'azote sur lequel ils sont liés, un système mono-, bi- ou spirocyclique ayant 4 à 10 membres, qui peut contenir en plus de l'atome d'azote, 0 à 2 autres hétéroatomes, choisis parmi le groupe oxygène, azote et soufre, où le système hétérocyclique peut en outre, être substitué par F, Cl, Br, CF₃, alkyle (C₁-C₆), O-alkyle (C₁-C₈), cycloalkyle (C₃-C₈), alcoxy (C₁-C₄)-alkyle (C₁-C₄), hydroxyalkyle (C₁-C₆), oxo, CO(R18), hydroxy, N(R22)CO-alkyle (C₁-C₆) ou SO₂-alkyl (C₁-C₆).

4. Composés de formule I selon les revendications 1 à 3, où
A est choisi parmi le groupe 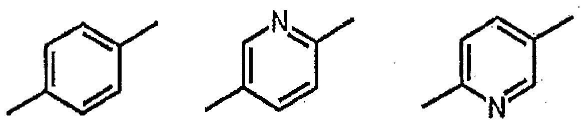 où A peut porter les substituants cités à la revendication 1.

5. Composés de formule I selon les revendications 1 à 4, où
B représente un cycle non aromatique ayant 4 à 6 membres, qui contient 1 à 2 atomes d'oxygène, où le système cyclique peut être substitué par un ou plusieurs des substituants suivants : alkyle (C₁-C₆) ou hydroxy.

6. Composés de formule I selon les revendications 1 à 5, où
l'élément B-L3 combiné représente

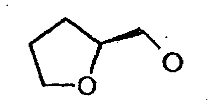

7. Composés de formule I selon la revendication 1, **caractérisés par** la formule II : 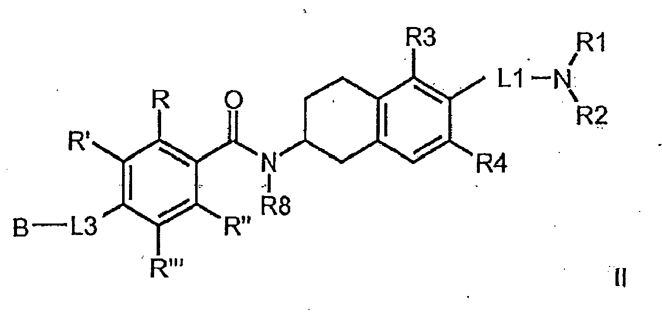 où les variables R1, R2, L1, R3, R4 et R8 ont les significations données à la revendication 1, et
R, R', R", R''' représentent indépendamment les uns des autres, H, F, Cl, Br, I, OH, CF₃, NO₂, CN, OCF₃, O-alkyle (C₁-C₆), O-alcoxy (C₁-C₄)-alkyle (C₁-C₄), alkyle (C₁-C₆), N(R54)(R55), SO₂-CH₃, CON(R56) (R57), CO(R60) ;
L3 représente CH₂O ;
B représente un cycle non aromatique ayant 4 à 6 membres, qui contient 1 à 2 atomes d'oxygène, où le système cyclique peut être substitué par un ou plusieurs des substituants suivants : F, alkyle (C₁-C₆), O-alkyle (C₁-C₆), alcoxy (C₁-C₄)-alkyle (C₁-C₄), oxo, hydroxy, de préférence alkyle (C₁-C₆) ou hydroxy.

8. Composés de formule II selon la revendication 7, où
l'élément B-L3 combiné représente

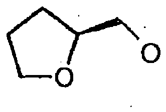

9. Composés de formule I selon la revendication 1, **caractérisés par** la formule IV : 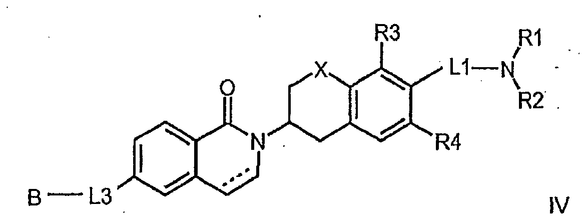 où R1, R2, R3, R4, X, L1, L3 et B ont les significations données pour la formule I à la revendication 1, où la ligne interrompue représente une double liaison optionnelle, de sorte que la dihydroisoquinolinone, mais également l'isoquinolinone de formule IV sont comprises.

10. Agent pharmaceutique contenant un ou plusieurs des composés de formule I selon une ou plusieurs des revendications 1 à 9.

11. Agent pharmaceutique contenant un ou plusieurs des composés de formule I selon une ou plusieurs des revendications 1 à 9, et un ou plusieurs agents actifs, qui ont une action favorable sur les troubles du métabolisme ou les maladies y associées.

12. Agent pharmaceutique contenant un ou plusieurs des composés de formule I selon une ou plusieurs des revendications 1 à 9 et un ou plusieurs antidiabétiques.

13. Agent pharmaceutique contenant un ou plusieurs des composés de formule I selon une ou plusieurs des revendications 1 à 9 et un ou plusieurs modulateurs des lipides.

14. Agent pharmaceutique contenant un ou plusieurs des composés de formule I selon une ou plusieurs des revendications 1 à 9 et un ou plusieurs composés anti-adiposités.

15. Composés de formule I selon l'une ou plusieurs des revendications 1 à 9, à utiliser dans le traitement et/ou la prévention de troubles du métabolisme des acides gras et de troubles du métabolisme du glucose.

16. Composés de formule I selon l'une ou plusieurs des revendications 1 à 9, à utiliser dans le traitement et/ou la prévention de troubles dans lesquels la résistance à l'insuline joue un rôle.

17. Composés de formule I selon l'une ou plusieurs des revendications 1 à 9, à utiliser dans le traitement et/ou la prévention du diabète et des maladies y associées.

18. Composés de formule I selon l'une ou plusieurs des revendications 1 à 9, à utiliser dans le traitement et/ou la prévention des dyslipidémies et de leurs conséquences.

19. Composés de formule I selon l'une ou plusieurs des revendications 1 à 9, à utiliser dans le traitement et/ou la prévention la cirrhose graisseuse non alcoolique et de ses formes d'évolution.

20. Composés de formule I selon l'une ou plusieurs des revendications 1 à 9, à utiliser dans le traitement et/ou la prévention d'états qui sont associés au syndrome métabolique.

21. Composés de formule I selon l'une ou plusieurs des revendications 1 à 9, à utiliser dans le traitement et/ou la prévention de l'obésité et des maladies y associées.

22. Composés selon l'une ou plusieurs des revendications 1 à 9, en combinaison avec au moins un autre agent actif, à utiliser dans le traitement et/ou la prévention de troubles du métabolisme des acides gras et des troubles du métabolisme du glucose.

23. Composés selon l'une ou plusieurs des revendications 1 à 9, en combinaison avec au moins un autre agent actif, à utiliser dans le traitement et/ou la prévention de troubles dans lesquels la résistance à l'insuline joue un rôle.

24. Procédé de préparation d'un agent pharmaceutique contenant un ou plusieurs des composés selon l'une ou plusieurs des revendications 1 à 9, **caractérisé en ce que** l'agent actif est mélangé à un support pharmaceutique approprié et ce mélange est mis sous une forme appropriée pour l'administration.
